# EUROPEAN PATENT SPECIFICATION

(11) **EP 4 017 593 B1**
(45) Date of publication and mention of the grant of the patent: **23.07.2025**
(21) Application number: 20767941.6
(22) Date of filing: 21.08.2020
(51) Int. Cl.: A61P 35/00, C07D 471/04, C07D 487/04, A61K 31/437, A61K 31/4985, A61K 31/5025

(54) **BICYCLIC AGONISTS OF STIMULATOR OF INTERFERON GENES STING**
BICYCLISCHE AGONISTEN DES STIMULATORS VON INTERFERONGENEN (STING)
AGONISTES BICYCLIQUES DU SIMULATEUR DE GÈNES D'INTERFÉRON (STING)

(30) Priority: 21.08.2019 US 201962889679 P
(43) Date of publication of application: 29.06.2022
(73) Proprietor: The Scripps Research Institute, La Jolla, CA 92037 (US)
(72) Inventor: PETRASSI, Hank Michael James, San Diego, California 92130 (US); YU, Chenguang, La Jolla, California 92037 (US); WANG, Jie, Shanghai 201203 (CN); CHATTERJEE, Arnab K., San Diego, California 92129 (US); SCHULTZ, Peter G., La Jolla, California 92037 (US); JOHNSON, Kristen, Santee, CA 92071 (US); CHU, Alan, San Diego, CA 92129 (US); CHIN, Emily, San Diego, California 92101 (US); LAIRSON, Luke L., San Diego, California 92103 (US)
(74) Representative: Plougmann Vingtoft a/s
(86) International application number: PCT/US2020/070444
(87) International publication number: WO 2021/035258

(56) References cited:
- EP-A1- 1 403 269
- EP-A1- 1 477 490
- WO-A1-2012/078855
- WO-A1-2014/089378
- WO-A1-2019/165032
- WO-A1-96/16954
- WO-A2-2009/011617
- GB-A- 2 563 642
- US-A- 4 657 893
- US-A- 5 262 537
- US-A1- 2009 239 982
- US-A1- 2017 146 519
- DEFILIPPIS ET AL: "Characterization of a novel human-specific STING agonist that elicits antiviral activity against emerging alphaviruses", PLOS PATHOGENS, vol. 11, no. 12, January 2015 (2015-01-01), pages 1 - 30, XP055510920

## Description

This application claims the benefit of priority to U.S. Patent Application No. 62/889,679 filed on August 21, 2019.

### BACKGROUND

The cGAS-STING signaling pathway plays a critical role in the innate immune response that mammalian host cells mount to eliminate diverse DNA and RNA viruses. STING (Stimulator of Interferon Genes) is an endoplasmic reticulum (ER) resident signaling protein, partially localized to mitochondria-associated membranes, which is broadly expressed in both immune and non-immune cell types. In response to cyclic dinucleotides (CDNs), including 2'-3' cGAMP produced in response to cytosolic DNA by cyclic GMP-AMP synthase (cGAS), STING translocates to the perinuclear region where it rapidly induces type I interferon (IFN) and pro-inflammatory cytokine production in a TBK1-/IRF3-dependent fashion. STING has also been found to directly bind cytosolic DNA, although the physiological relevance of direct DNA sensing activity remains to be fully characterized.

Recent work has demonstrated that STING plays essential roles in immune responses to tumor cells. Efficient tumor-initiated T cell priming within the tumor microenvironment requires interferon-beta (IFN-b) production by resident dendritic cells and the expression of IFN-b has been demonstrated to be dependent upon activation of the STING pathway (1). Indeed, intratumoral delivery of nucleotide-based STING agonists have been demonstrated to induce the profound regression of established tumors in syngeneic mouse models (1). In addition, activation of the STING pathway has also been demonstrated to significantly contribute to the anti-tumor effect of radiation, via IFN-b mediated immune response within the irradiated tumor microenvironment.

GB 2 563 642 A (CURADEV PHARMA LTD [GB]), US 201 7/1 4651 9 Al (DEFILIPPIS VICTOR [US] ET AL), and DEFILIPPIS ET AL. ("Characterization of a novel human-specific STING agonist that elicits antiviral activity against emerging alphaviruses", PLOS PATHOGENS, vol. 11, no. 12, January 2015 (2015-01), pages 1-30.) disclose various amide-based STING modulators potentially useful in therapy, however, they do not mention the compounds of the present invention. They do not disclose a benzoxazine-based compound directly linked to a bicyclic N-containing heterocycle nor a thiophene / phenyl / pyridine linked via an amide bond to bicyclic N-containing heterocycle.

### SUMMARY

In various embodiments, the present disclosure provides an agonist of the Stimulator of Interferon Genes (STING), which can be used in the treatment of tumors.
One aspect of the invention relates to a compound of formula (I): 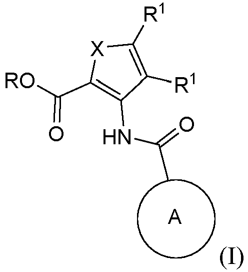 wherein
X is S, -N=C(R¹)-, or -C(R¹)=C(R¹)-;
each R¹ is independently H, F, Cl, C₁-C₆-alkyl, ethenyl or ethynyl either of which can be substituted, cyano, alkoxyl, haloalkyl, or C₃-C₆-cycloalkyl;
   wherein at least one R¹ is F, Cl, C₁-C₆-alkyl, ethenyl or ethynyl either of which can be substituted, cyano, alkoxyl, haloalkyl, or C₃-C₆-cycloalkyl;
or both R¹, together with the carbon atoms to which they are bound, form a fused phenyl;
R is H, alkyl optionally substituted with -((C₁-C₆-alkyl)OC(O)OC₁-C₆-alkyl), or benzyl, wherein the benzyl can be unsubstituted or substituted with methoxyl; and
Ring A is a bicyclic fully aromatic or partially reduced heteroaryl ring system comprising 3, 4, or 5 N atoms, substituted with 0, 1, 2, 3 ,or 4 substituents each independently selected from the set consisting of NH₂, cyano, NHC(=O)O-tBu,OH, carboxamido, C₁-C₆-alkyl, -S(O)₂(C₁-C₆-alkyl), -S(O)(C₁-C₆-alkyl), alkylnitrile, alkoxyl, and haloalkyl; provided that a partially reduced heteroaryl ring system can also be substituted with an oxo group;
or a pharmaceutically acceptable salt thereof,
or formula (II): 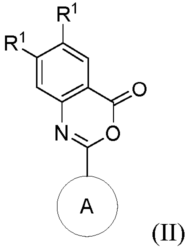 wherein
each R¹ is independently H, F, Cl, ethenyl or ethynyl either of which can be substituted, cyano, alkoxyl, haloalkyl, or C₃-C₆-cycloalkyl;
   wherein at least one R¹ is F, Cl, ethenyl or ethynyl either of which can be substituted, cyano, alkoxyl, haloalkyl, or C₃-C₆-cycloalkyl;
or both R¹, together with the carbon atoms to which they are bound, form a fused phenyl; and
Ring A is a bicyclic fully aromatic or partially reduced heteroaryl ring system comprising 3, 4, or 5 N atoms, substituted with 0, 1, 2, 3 ,or 4 substituents each independently selected from the set consisting of NH₂, cyano, NHC(=O)O-tBu,OH, carboxamido, C₁-C₆-alkyl, -S(O)₂(C₁-C₆-alkyl), -S(O)(C₁-C₆-alkyl), alkylnitrile, alkoxyl, and haloalkyl; provided that a partially reduced heteroaryl ring system can also be substituted with an oxo group;
or a pharmaceutically acceptable salt thereof.
More specifically, per illustrative embodiments, a compound of the present disclosure includes any of the specific compounds shown in Table 1 below.

A second aspect of the invention relates to a compound, or a pharmaceutically acceptable salt thereof, selected from the following table:

| | | | |
|---|---|---|---|
| **1** | 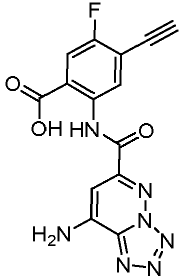 | **2** | 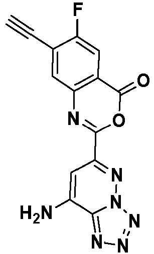 |
| **3** | 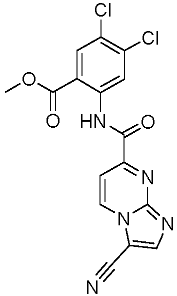 | **4** | 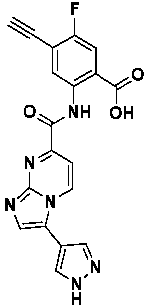 |
| **5** | 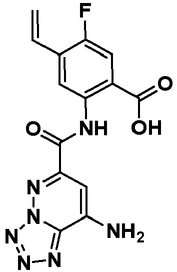 | **48** | 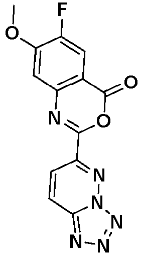 |
| **6** | 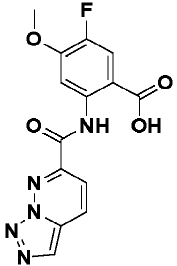 | **49** | 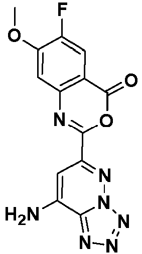 |
| **7** | 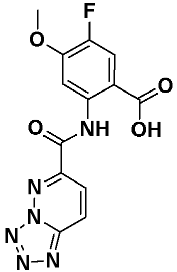 | **50** | 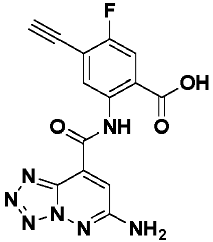 |
| **8** | 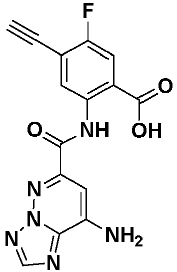 | **51** | 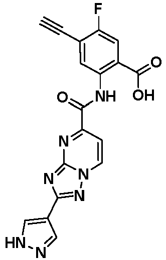 |
| **9** | 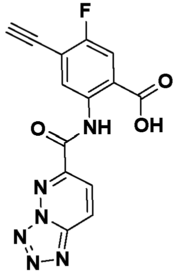 | **52** | 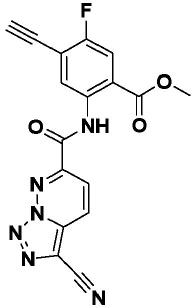 |
| **10** | 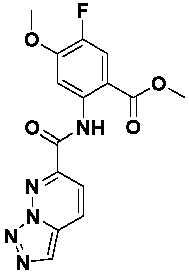 | **53** | 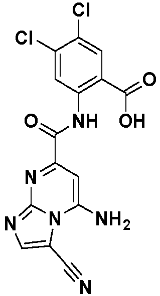 |
| **11** | 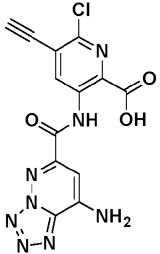 | **54** | 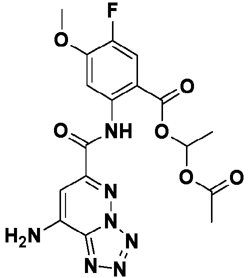 |
| **12** | 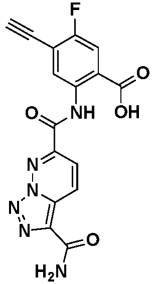 | **55** | 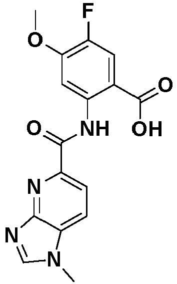 |
| **13** | 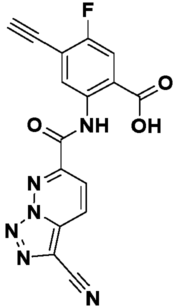 | **56** | 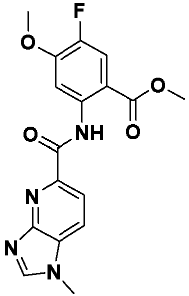 |
| **14** | 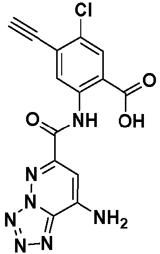 | **57** | 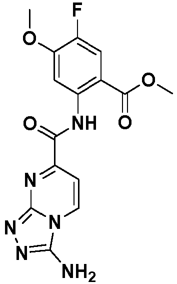 |
| **15** | 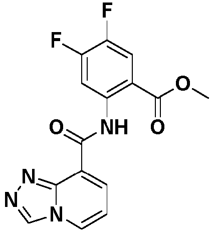 | **58** | 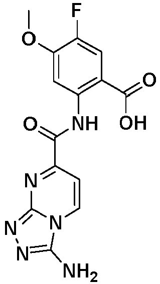 |
| **16** | 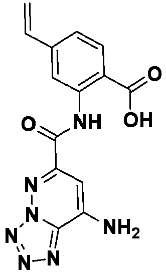 | **59** | 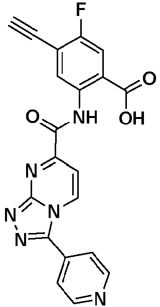 |
| **17** | 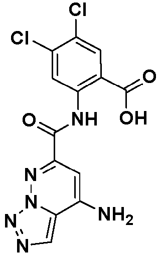 | **60** | 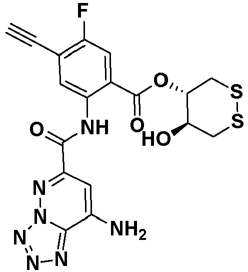 |
| **18** | 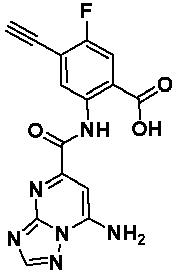 | **61** | 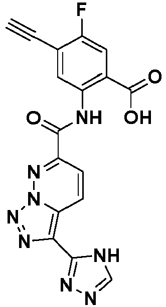 |
| **19** | 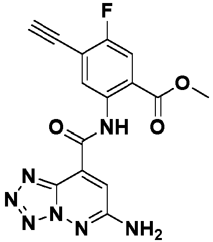 | **62** | 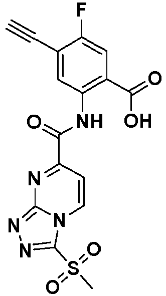 |
| **20** | 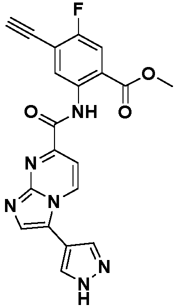 | **63** | 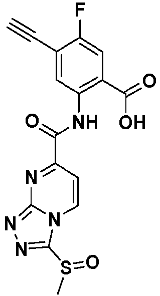 |
| **21** | 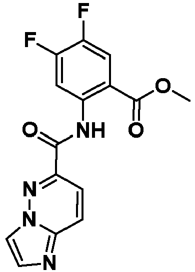 | **64** | 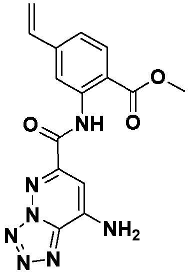 |
| **22** | 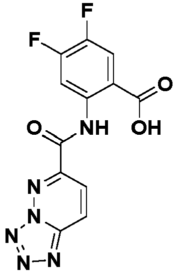 | **65** | 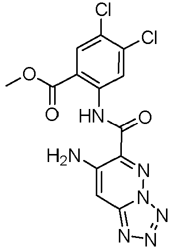 |
| **23** | 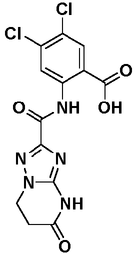 | **66** | 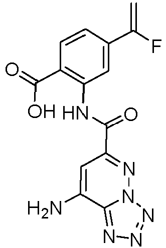 |
| **24** | 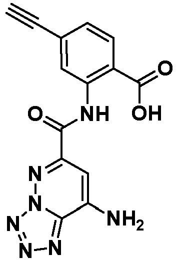 | **67** | 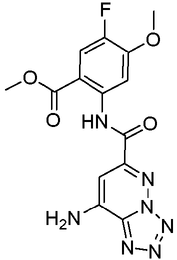 |
| **25** | 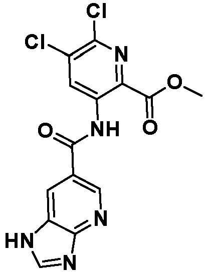 | **68** | 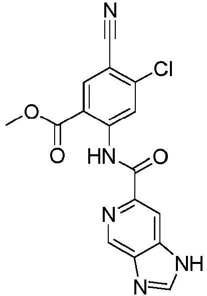 |
| **26** | 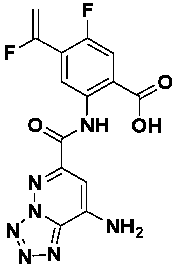 | **69** | 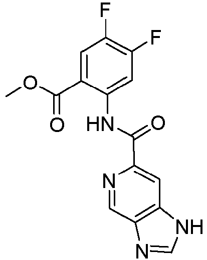 |
| **27** | 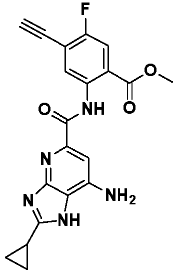 | **70** | 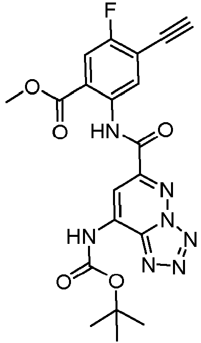 |
| **28** | 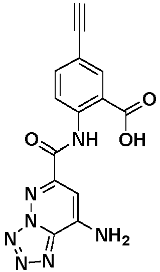 | **71** | 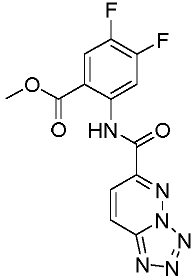 |
| **29** | 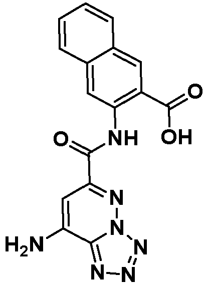 | **72** | 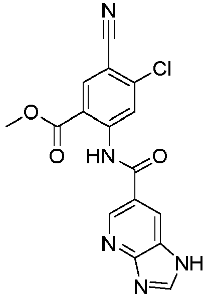 |
| **30** | 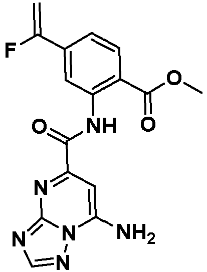 | **73** | 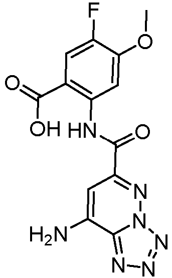 |
| **31** | 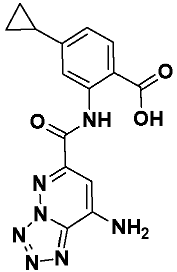 | **74** | 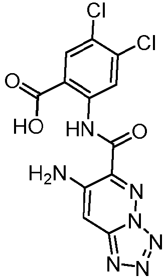 |
| **32** | 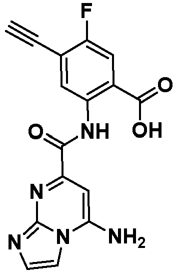 | **75** | 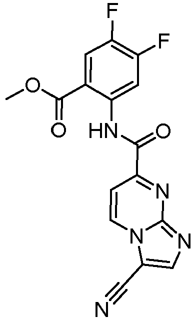 |
| **33** | 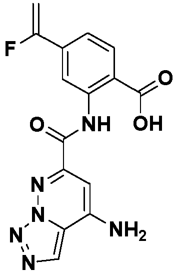 | **76** | 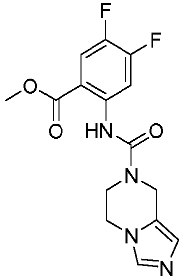 |
| **34** | 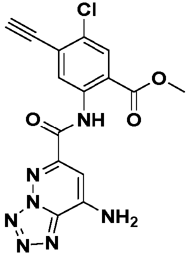 | **77** | 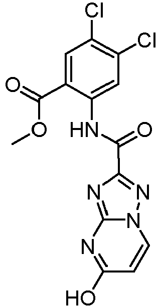 |
| **35** | 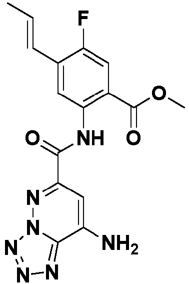 | **78** | 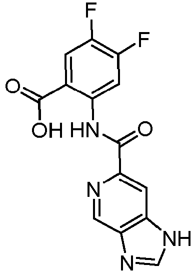 |
| **36** | 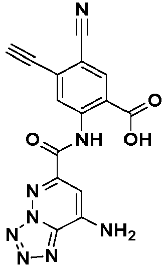 | **79** | 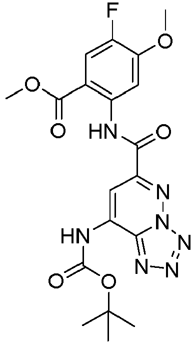 |
| **37** | 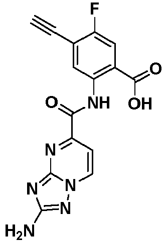 | **80** | 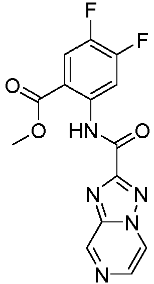 |
| **38** | 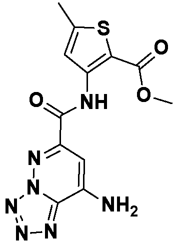 | **81** | 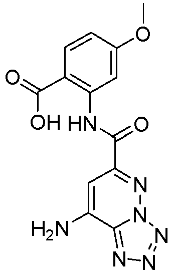 |
| **39** | 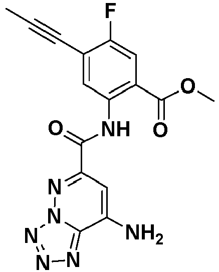 | **82** | 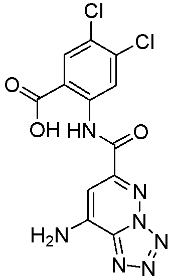 |
| **40** | 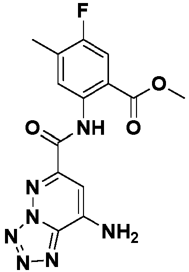 | **83** | 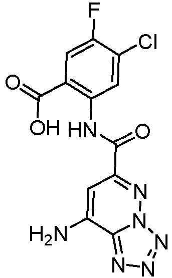 |
| **41** | 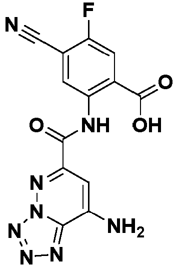 | **84** | 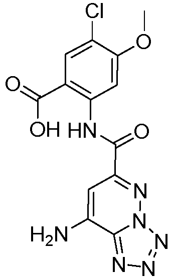 |
| **42** | 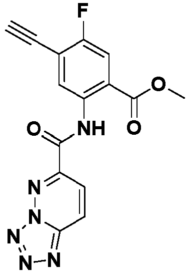 | **85** | 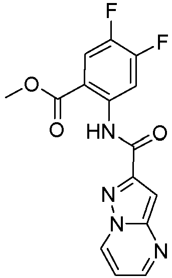 |
| **43** | 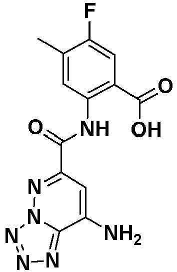 | **86** | 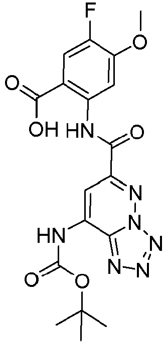 |
| **44** | 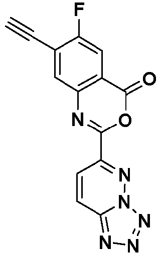 | **87** | 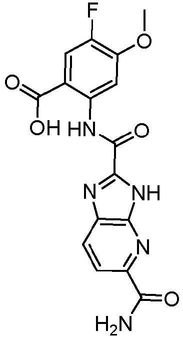 |
| **45** | 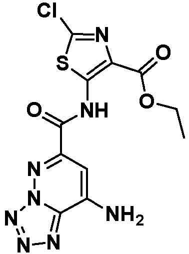 | **88** | 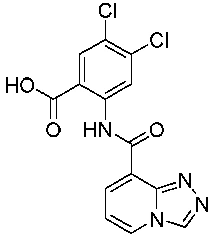 |
| **46** | 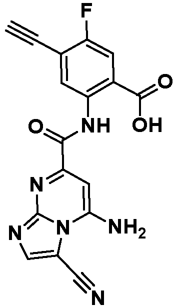 | **47** | 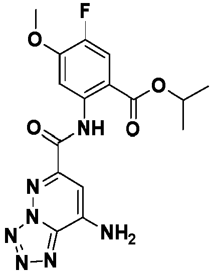 |
| **89** | 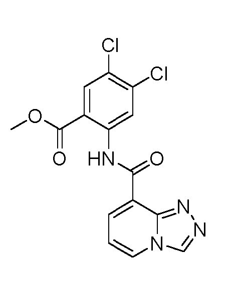 | **90** | 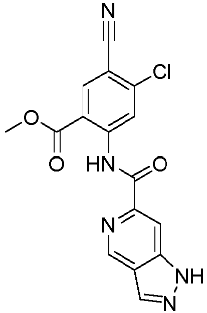 |
| **91** | 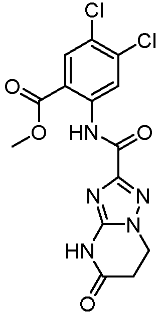 | | |

Additionally, a third aspect of the invention relates to a pharmaceutical composition comprising a compound or pharmaceutically acceptable salt thereof according to any one of the first to the second aspect and a pharmaceutically acceptable carrier.

A fourth aspect of the present invention relates to a compound or pharmaceutically acceptable salt thereof according to any one of the first to the second aspect for use in stimulating expression of interferon genes in a human patient.

A fifth aspect of the present invention relates to a compound or pharmaceutically acceptable salt thereof according to any one of the first to the second aspect for use in the treatment of a tumor in a patient.

### DETAILED DESCRIPTION

There is significant interest in the development of STING pathway agonists for diverse immuno-oncology applications. Most notably, STING pathway agonists have significant potential application as part of combination therapies involving immune-checkpoint targeting drugs, in patients that fail to respond to checkpoint blockade alone.

We have established a robust platform for identifying non-nucleotide small molecule STING agonists. his has been established using a primary assay involving a human THP-1 cell line carrying an IRF-inducible reporter with 5 copies of the IFN signaling response element. Counter screens, involving alternative reporter constructs, rodent cell-based assays, as well as cGAS and STING knock-out cell lines, are used to eliminate luciferase artifacts and ensure human-rodent cross species reactivity, as well as pathway selectivity. Biochemical assays, involving cGAS enzymatic activity and STING protein binding assays, are used to identify the specific target of identified hits.

"Treating" or "treatment" within the meaning herein refers to an alleviation of symptoms associated with a disorder or disease, or inhibition of further progression or worsening of those symptoms, or prevention or prophylaxis of the disease or disorder, or curing the disease or disorder. Similarly, as used herein, an "effective amount" or a "therapeutically effective amount" of a compound of the present disclosure refers to an amount of the compound that alleviates, in whole or in part, symptoms associated with the disorder or condition, or halts or slows further progression or worsening of those symptoms, or prevents, or provides prophylaxis for, the disorder or condition. In particular, a "therapeutically effective amount" refers to an amount that is effective, at dosages and for periods of time necessary, to achieve the desired therapeutic result. A therapeutically effective amount is also one in which any toxic or detrimental effects of compounds of the present disclosure are outweighed by the therapeutically beneficial effects.

The expression "effective amount", when used to describe therapy to an individual suffering from a disorder, refers to the quantity or concentration of a compound of the present disclosure that is effective to inhibit or otherwise act on STING in the individual's tissues wherein STING involved in the disorder, wherein such inhibition or other action occurs to an extent sufficient to produce a beneficial therapeutic effect.

Generally, the initial therapeutically effective amount of a compound described herein or a pharmaceutically acceptable salt thereof that is administered is in the range of about 0.01 to about 200 mg/kg or about 0.1 to about 20 mg/kg of patient body weight per day, with the typical initial range being about 0.3 to about 15 mg/kg/day. Oral unit dosage forms, such as tablets and capsules, may contain from about 0.1 mg to about 1000 mg of the compound or a pharmaceutically acceptable salt thereof. In another embodiment, such dosage forms contain from about 50 mg to about 500 mg of the compound or a pharmaceutically acceptable salt thereof. In yet another embodiment, such dosage forms contain from about 25 mg to about 200 mg of the compound or a pharmaceutically acceptable salt thereof. In still another embodiment, such dosage forms contain from about 10 mg to about 100 mg of the compound or a pharmaceutically acceptable salt thereof. In a further embodiment, such dosage forms contain from about 5 mg to about 50 mg of the compound or a pharmaceutically acceptable salt thereof. In any of the foregoing embodiments the dosage form can be administered once a day or twice per day.

The term "pharmaceutically acceptable salts" refers to nontoxic inorganic or organic acid and/or base addition salts, see, for example, Lit, et al., Salt Selection for Basic Drugs (1986), Int J. Pharm., 33, 201-217, incorporated by reference herein. Representative pharmaceutically acceptable salts include, *e.g.,* alkali metal salts, alkali earth salts, ammonium salts, water-soluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosaliculate, suramate, tannate, tartrate, teoclate, tosylate, triethiodide, and valerate salts. A pharmaceutically acceptable salt can have more than one charged atom in its structure. In this instance the pharmaceutically acceptable salt can have multiple counterions. Thus, a pharmaceutically acceptable salt can have one or more charged atoms and/or one or more counterions.

Standard abbreviations for chemical groups such as are well known in the art are used; e.g., Me = methyl, Et = ethyl, i-Pr = isopropyl, Bu = butyl, t-Bu = tert-butyl, Ph = phenyl, Bn = benzyl, Ac = acetyl, Bz = benzoyl.

"Alkyl" refers to straight or branched chain hydrocarbyl including from 1 to about 20 carbon atoms. For instance, an alkyl can have from 1 to 10 carbon atoms or 1 to 6 carbon atoms. Exemplary alkyl includes straight chain alkyl groups such as methyl, ethyl, propyl, butyl, pentyl, hexyl, heptyl, octyl, nonyl, decyl, undecyl, dodecyl, and also includes branched chain isomers of straight chain alkyl groups, for example without limitation, -CH(CH₃)₂, -CH(CH₃)(CH₂CH₃), -CH(CH₂CH3)₂, -C(CH₃)₃, -C(CH₂ CH₃)₃, -CH₂CH(CH₃)₂, -CH₂CH(CH₃)(CH₂CH₃), -CH₂CH(CH₂CH₃)₂, -CH₂C(C H₃)₃, -CH₂C(CH₂CH₃)₃, -CH(CH₃)CH(CH₃)(CH₂CH₃), -CH₂CH₂CH(CH₃)₂, -CH ₂CH₂CH(CH₃)(CH₂CH₃), -CH₂CH₂CH(CH₂CH₃)₂, -CH₂CH₂C(CH₃)₃, -CH₂CH₂ C(CH₂CH₃)₃, -CH(CH₃)CH_{2C}H(CH₃)₂, -CH(CH₃)CH(CH₃)CH(CH₃)₂-Thus, alkyl groups include primary alkyl groups, secondary alkyl groups, and tertiary alkyl groups. An alkyl group can be unsubstituted or optionally substituted with one or more substituents as described herein.

The term "alkoxy" or "alkoxyl" refers to an -O-alkyl group having the indicated number of carbon atoms. For example, a (C₁-C₆)-alkoxy group includes -O-methyl, -O-ethyl, -O-propyl, -O-isopropyl, -O-butyl, -O-sec-butyl, - O-tert-butyl, -O-pentyl, -O-isopentyl, -O-neopentyl, -O-hexyl, -O-isohexyl, and -O-neohexyl.

The terms "halo" or "halogen" or "halide" by themselves or as part of another substituent mean, unless otherwise stated, a fluorine, chlorine, bromine, or iodine atom, preferably, fluorine, chlorine, or bromine.

A "haloalkyl" group includes mono-halo alkyl groups, poly-halo alkyl groups wherein all halo atoms can be the same or different, and per-halo alkyl groups, wherein all hydrogen atoms are replaced by the same or differing halogen atoms, such as fluorine and/or chlorine atoms. Examples of haloalkyl include trifluoromethyl, 1,1-dichloroethyl, 1,2-dichloroethyl, 1,3-dibromo-3,3-difluoropropyl, perfluorobutyl.

Aryl groups are cyclic aromatic hydrocarbons that do not contain heteroatoms in the ring. An aromatic compound, as is well-known in the art, is a multiply-unsaturated cyclic system that contains 4n+2 π electrons where n is an integer. Thus, aryl groups include, but are not limited to, phenyl, azulenyl, heptalenyl, biphenyl, indacenyl, fluorenyl, phenanthrenyl, triphenylenyl, pyrenyl, naphthacenyl, chrysenyl, biphenylenyl, anthracenyl, and naphthyl groups. In some embodiments, aryl groups contain about 6 to about 14 carbons in the ring portions of the groups. Aryl groups can be unsubstituted or substituted, as defined above. Representative substituted aryl groups can be mono-substituted or substituted more than once, such as, but not limited to, 2-, 3-, 4-, 5-, or 6-substituted phenyl or 2-8 substituted naphthyl groups, which can be substituted with carbon or non-carbon groups such as those listed above.

Heterocyclyl groups or the term "heterocyclyl" includes aromatic and non-aromatic ring compounds containing 3 or more ring members, of which one or more ring atom is a heteroatom such as, but not limited to, N, O, and S. Thus, a heterocyclyl can be a cycloheteroalkyl, or a heteroaryl, or if polycyclic, any combination thereof. In some embodiments, heterocyclyl groups include 3 to about 20 ring members, whereas other such groups have 3 to about 15 ring members. A heterocyclyl group designated as a C2-heterocyclyl can be a 5-ring with two carbon atoms and three heteroatoms, a 6-ring with two carbon atoms and four heteroatoms and so forth. Likewise, a C4-heterocyclyl can be a 5-ring with one heteroatom, a 6-ring with two heteroatoms, and so forth. The number of carbon atoms plus the number of heteroatoms sums up to equal the total number of ring atoms. Ring sizes can also be expressed by the total number of atoms in the ring, e.g., a 3- to 10-membered heterocyclyl group, counting both carbon and non-carbon ring atoms. A heterocyclyl ring can also include one or more double bonds. A heteroaryl ring is an embodiment of a heterocyclyl group. The term "heterocyclyl group" includes fused ring species including those comprising fused aromatic and non-aromatic groups. For example, a dioxolanyl ring and a benzdioxolanyl ring system (methylenedioxyphenyl ring system) are both heterocyclyl groups within the meaning herein. The term also includes polycyclic, e.g., bicyclo- and tricyclo- ring systems containing one or more heteroatom such as, but not limited to, quinuclidyl. Heterocyclyl groups can be unsubstituted or can be substituted.

Heteroaryl groups are heterocyclic aromatic ring compounds containing 5 or more ring members, of which, one or more is a heteroatom such as, but not limited to, N, O, and S; for instance, heteroaryl rings can have 5 to about 8-12 ring members. A heteroaryl group is a variety of a heterocyclyl group that possesses an aromatic electronic structure, which is a multiply-unsaturated cyclic system that contains 4n+2 π electrons wherein n is an integer. A heteroaryl group designated as a C2-heteroaryl can be a 5-ring (i.e., a 5-membered ring) with two carbon atoms and three heteroatoms, a 6-ring (i.e., a 6-membered ring) with two carbon atoms and four heteroatoms and so forth. Likewise, a C4-heteroaryl can be a 5-ring with one heteroatom, a 6-ring with two heteroatoms, and so forth. The number of carbon atoms plus the number of heteroatoms sums up to equal the total number of ring atoms. Heteroaryl is also intended to include oxidized S or N, such as sulfinyl, sulfonyl and N-oxide of a tertiary ring nitrogen. A carbon or heteroatom is the point of attachment of the heteroaryl ring structure such that a stable compound is produced. Examples of heteroaryl groups include, but are not limited to, pyridinyl, pyridazinyl, pyrazinyl, quinaoxalyl, indolizinyl, benzo[b]thienyl, quinazolinyl, purinyl, indolyl, quinolinyl, pyrimidinyl, pyrrolyl, pyrazolyl, oxazolyl, thiazolyl, thienyl, isoxazolyl, oxathiadiazolyl, isothiazolyl, tetrazolyl, imidazolyl, triazolyl, furanyl, benzofuryl, and indolyl. A heteroaryl group can be unsubstituted or optionally substituted with one or more substituents as described herein.

Similarly, other aryl (e.g., phenyl) and heteroaryl (e.g., pyridyl) ring systems described herein can be written either with the explicit double bonds, or with the aryl "circle" nomenclature, but the meanings are the same.

Cycloalkyl groups are groups containing one or more carbocyclic ring including, but not limited to, cyclopropyl, cyclobutyl, cyclopentyl, cyclohexyl, cycloheptyl, and cyclooctyl groups. In some embodiments, the cycloalkyl group can have 3 to about 8-12 ring members, whereas in other embodiments the number of ring carbon atoms range from 3 to 4, 5, 6, or 7. Cycloalkyl groups further include polycyclic cycloalkyl groups such as, but not limited to, norbornyl, adamantyl, bornyl, camphenyl, isocamphenyl, and carenyl groups, and fused rings such as, but not limited to, decalinyl. Cycloalkyl groups also include rings that are substituted with straight or branched chain alkyl groups as defined above.

Cycloalkenyl groups include cycloalkyl groups having at least one double bond between 2 carbons. Thus for example, cycloalkenyl groups include but are not limited to cyclohexenyl, cyclopentenyl, and cyclohexadienyl groups. Cycloalkenyl groups can have from 3 to about 8-12 ring members, whereas in other embodiments the number of ring carbon atoms range from 3 to 5, 6, or 7. Cycloalkyl groups further include polycyclic cycloalkyl groups such as, but not limited to, norbornyl, adamantyl, bornyl, camphenyl, isocamphenyl, and carenyl groups, and fused rings such as, but not limited to, decalinyl, provided they include at least one double bond within a ring. Cycloalkenyl groups also include rings that are substituted with straight or branched chain alkyl groups as defined above.

One or more optional substituents on any group described herein are independently selected from the group consisting of R^{A}, OR^{A}, halo, -N=N-R^{A}, NR^{A}R^{B}, -(C₁-C₆-alkyl)NR^{A}R^{B}, -C(O)OR^{A}, -C(O)NR^{A}R^{B}, -OC(O)R^{A}, and -CN. R^{A} and R^{B} are independently selected from the group consisting of H, -CN, - hydroxy, oxo, C₁-C₆-alkyl, C₁-C₆-alkoxy, C₂-C₆-alkenyl, C₂-C₆-alkynyl, NH₂, - S(O)₀₋₂-(C₁-C₆-alkyl), -S(O)₀₋₂-(C₆-C₁₀-aryl), -C(O)(C₁-C₆-alkyl), -C(O)(C₃-C₁₄-carbocyclyl), -C₃-C₁₄-carbocyclyl, -(C₁-C₆-alkyl)(C₃-C₁₄-carbocyclyl), C₆-C₁₀-aryl, 3- to 14-membered heterocycloalkyl and -(C₁-C₆-alkyl)-(3- to 14-membered heterocycloalkyl) (wherein 1-4 heterocycloalkyl members are independently selected from N, O, and S), and 5- to 10-membered heteroaryl (wherein 1-4 heteroaryl members are independently selected from N, O, and S). Each alkyl, alkoxy, alkenyl, alkynyl, aryl, carbocyclyl, heterocycloalkyl, and heteroaryl moiety of R^{A} and R^{B} is optionally substituted with one or more substituents selected from the group consisting of hydroxy, halo, -NR'₂ (wherein each R' is independently selected from the group consisting of C₁-C₆-alkyl, C₂-C₆-alkenyl, C₂-C₆-alkynyl, C₆-C₁₀-aryl, 3- to 14-membered heterocycloalkyl and -(C₁-C₆-alkyl)-(3- to 14-membered heterocycloalkyl) (wherein 1-4 ring members are independently selected from N, O, and S), and 5- to 10-membered heteroaryl (wherein 1-4 heteroaryl members are independently selected from N, O, and S), -NHC(O)(OC₁-C₆-alkyl), -NO₂, -CN, oxo, -C(O)OH, -C(O)O(C₁-C₆-alkyl), -C₁-C₆-alkyl(C₁-C₆-alkoxy), -C(O)NH₂, C₁-C₆-alkyl, -C(O)C₁-C₆-alkyl, -OC₁-C₆-alkyl, -Si(C₁-C₆-alkyl)₃, -S(O)₀₋₂-(C₁-C₆-alkyl), C₆-C₁₀-aryl, -(C₁-C₆-alkyl)(C₆-C₁₀-aryl), 3- to 14-membered heterocycloalkyl, and -(C₁-C₆-alkyl)-(3- to 14-membered heterocycle) (wherein 1-4 heterocycle members are independently selected from N, O, and S), and -O(C₆-C₁₄-aryl). Each alkyl, alkenyl, aryl, and heterocycloalkyl described above is optionally substituted with one or more substituents selected from the group consisting of hydroxy, -OC₁-C₆-alkyl, halo, -NH₂, -(C₁-C₆-alkyl)NH₂, -C(O)OH, CN, and oxo.

Compounds described herein can exist in various isomeric forms, including configurational, geometric, and conformational isomers, including, for example, *cis-* or *trans-* conformations. The compounds may also exist in one or more tautomeric forms, including both single tautomers and mixtures of tautomers. The term "isomer" is intended to encompass all isomeric forms of a compound of this disclosure, including tautomeric forms of the compound. The compounds of the present disclosure may also exist in open-chain or cyclized forms. In some cases, one or more of the cyclized forms may result from the loss of water. The specific composition of the open-chain and cyclized forms may be dependent on how the compound is isolated, stored or administered. For example, the compound may exist primarily in an open-chained form under acidic conditions but cyclize under neutral conditions. All forms are included in the disclosure.

The substituent -CO₂H may be replaced with bioisosteric replacements such as: 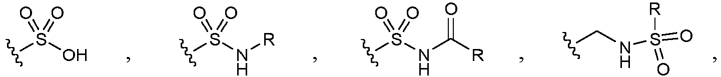 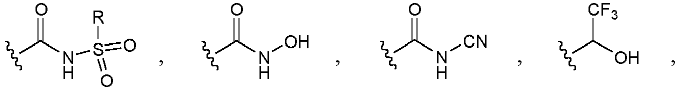 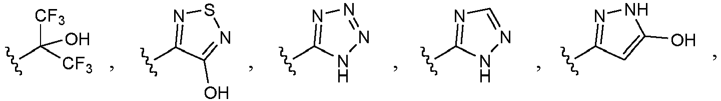 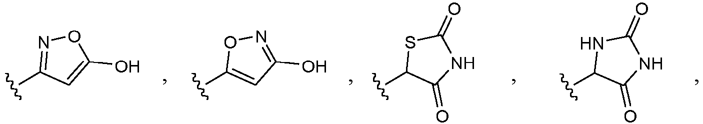 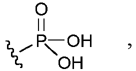 and the like, wherein R has the same definition as R^{A} as defined herein. *See, e.g.,* THE PRACTICE OF MEDICINAL CHEMISTRY (Academic Press: New York, 1996), at page 203.

Some compounds described herein can have asymmetric centers and therefore exist in different enantiomeric and diastereomeric forms. A compound as described herein can be in the form of an optical isomer or a diastereomer. Accordingly, the disclosure encompasses compounds and their uses as described herein in the form of their optical isomers, diastereoisomers and mixtures thereof, including a racemic mixture. Optical isomers of the compounds of the disclosure can be obtained by known techniques such as asymmetric synthesis, chiral chromatography, simulated moving bed technology or via chemical separation of stereoisomers through the employment of optically active resolving agents.

Unless otherwise indicated, the term "stereoisomer" means one stereoisomer of a compound that is substantially free of other stereoisomers of that compound. Thus, a stereomerically pure compound having one chiral center will be substantially free of the opposite enantiomer of the compound. A stereomerically pure compound having two chiral centers will be substantially free of other diastereomers of the compound. A typical stereomerically pure compound comprises greater than about 80% by weight of one stereoisomer of the compound and less than about 20% by weight of other stereoisomers of the compound, for example greater than about 90% by weight of one stereoisomer of the compound and less than about 10% by weight of the other stereoisomers of the compound, or greater than about 95% by weight of one stereoisomer of the compound and less than about 5% by weight of the other stereoisomers of the compound, or greater than about 97% by weight of one stereoisomer of the compound and less than about 3% by weight of the other stereoisomers of the compound, or greater than about 99% by weight of one stereoisomer of the compound and less than about 1% by weight of the other stereoisomers of the compound. The stereoisomer as described above can be viewed as composition comprising two stereoisomers that are present in their respective weight percentages described herein.

If there is a discrepancy between a depicted structure and a name given to that structure, then the depicted structure controls. Additionally, if the stereochemistry of a structure or a portion of a structure is not indicated with, for example, bold or dashed lines, the structure or portion of the structure is to be interpreted as encompassing all stereoisomers of it. In some cases, however, where more than one chiral center exists, the structures and names may be represented as single enantiomers to help describe the relative stereochemistry. Those skilled in the art of organic synthesis will know if the compounds are prepared as single enantiomers from the methods used to prepare them.

As used herein, and unless otherwise specified to the contrary, the term "compound" is inclusive in that it encompasses a compound or a pharmaceutically acceptable salt, stereoisomer, and/or tautomer thereof. Thus, for instance, a compound of formula (I) or formula (II) includes a pharmaceutically acceptable salt of a tautomer of the compound.

### COMPOUNDS

The present disclosure provides in various embodiments a compound of formula (I):

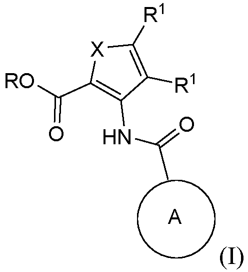

Wherein X is S, -N=C(R¹)-, or -C(R¹)=C(R¹)-_{.}

Each R¹ is independently H, F, Cl, C₁-C₆-alkyl, ethenyl or ethynyl either of which can be substituted, cyano, alkoxyl, haloalkyl, or C₃-C₆-cycloalkyl; wherein at least one R¹ is F, Cl, C₁-C₆-alkyl, ethenyl or ethynyl either of which can be substituted, cyano, alkoxyl, haloalkyl, or C₃-C₆-cycloalkyl.

In an embodiment, both instances of R¹ bound to the ring containing X, together with the carbon atoms to which they are bound, form a fused phenyl.

R is H, alkyl optionally substituted with -((C₁-C₆-alkyl)OC(O)OC₁-C₆-alkyl), or benzyl, wherein the benzyl can be unsubstituted or substituted with methoxyl.
Ring A is a bicyclic fully aromatic or partially reduced heteroaryl ring system comprising 3, 4, or 5 N atoms, substituted with 0, 1, 2, 3 ,or 4 substituents each independently selected from the set consisting of NH₂, cyano, NHC(=O)O-tBu, OH, carboxamido, C₁-C₆-alkyl, -S(O)₂(C₁-C₆-alkyl), -S(O)(C₁-C₆-alkyl), alkylnitrile, alkoxyl, and haloalkyl. In various embodiments, the partially reduced heteroaryl ring system can also be substituted with an oxo group,
or a pharmaceutically acceptable salt thereof,
or formula (II): 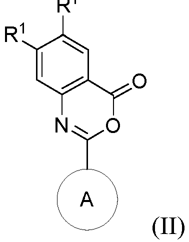
Each R¹ is independently H, F, Cl, ethenyl or ethynyl either of which can be substituted, cyano, alkoxyl, haloalkyl, or C₃-C₆-cycloalkyl;
wherein at least one R¹ is F, Cl, ethenyl or ethynyl either of which can be substituted, cyano, alkoxyl, haloalkyl, or C₃-C₆-cycloalkyl.

In an embodiment, both instances of R¹ bound to the ring containing X, together with the carbon atoms to which they are bound, form a fused phenyl.

Ring A is a bicyclic fully aromatic or partially reduced heteroaryl ring system comprising 3, 4, or 5 N atoms, substituted with 0, 1, 2, 3 ,or 4 substituents each independently selected from the set consisting of NH₂, cyano, NHC(=O)O-tBu, OH, carboxamido, C₁-C₆-alkyl, -S(O)₂(C₁-C₆-alkyl), -S(O)(C₁-C₆-alkyl), alkylnitrile, alkoxyl, and haloalkyl. In various embodiments, the partially reduced heteroaryl ring system can also be substituted with an oxo group;
or a pharmaceutically acceptable salt thereof.

In additional embodiments, the disclosure provides a compound of formula (I), wherein X is S, -N=C(R¹)-, or -C(R¹)=C(R¹)-. Each R¹ is independently H, F, Cl, ethenyl or ethynyl either of which can be substituted, cyano, alkoxyl, or haloalkyl wherein at least one R¹ is F, Cl, ethenyl or ethynyl either of which can be substituted, cyano, alkoxyl, or haloalkyl. R is H, alkyl, or benzyl, wherein the benzyl can be unsubstituted or substituted with methoxyl. Ring A is a bicyclic fully aromatic or partially reduced heteroaryl ring system comprising 3, 4, or 5 N atoms, substituted with 0, 1, 2, 3 ,or 4 substituents each independently selected from the set consisting of NH₂, cyano, NHC(=O)O-tBu,OH, carboxamido, alkylnitrile, alkoxyl, and haloalkyl; provided that a partially reduced heteroaryl ring system can also be substituted with an oxo group.

In other embodiments, the present disclosure provides a compound of formula (II) as described herein.

In various embodiments, optionally in combination with any other embodiment described herein, Ring A is unsubstituted or substituted, as described herein, and is one selected from the group consisting of: 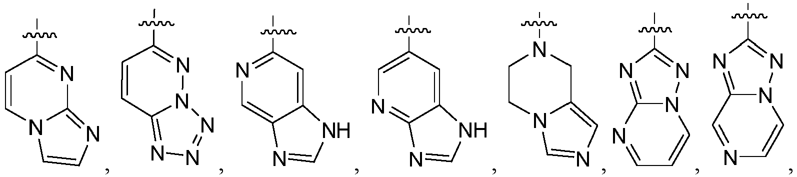 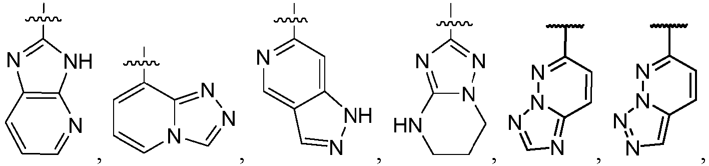 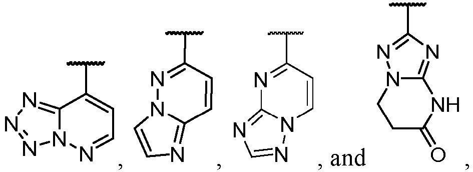 wherein a wavy line indicates a position of bonding.

In further embodiments, the present disclosure provides specific examples of formula (I) and formula (II) compounds, and their pharmaceutically acceptable salts, as set forth in Table 1 below. The compounds are presented with activity scores deriving, in part, from an ISG-LUC activation assay as described herein, and physico-chemical characterizing data.

**Table 1: Specific Compounds and Activity Scores. Activity scores are based upon potency and efficacy data (+ = EC₅₀ > 20,000 nM; ++ = active but less potent and efficacious than reference compound (EC₅₀ > 1000 nM); +++ = activity comparable to reference compound (EC₅₀ < 3000 nM); ++++ = more potent and/or efficacious than reference compound (EC₅₀ < 900 nM)).**

| **Compound No.** | **Structure** | **ISG-LUC activation assay score** | **Analytical Data** |
|---|---|---|---|
| **1** | 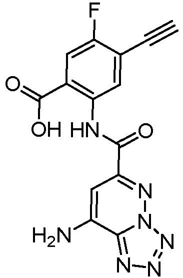 | +++ | ¹H NMR (400 MHz, DMSO-*d*₆) δ 15.73 (s, 1H), 8.82 (d, *J =* 6.6 Hz, 1H), 8.33 (s, 2H), 7.76 (d, *J =* 10.3 Hz, 1H), 7.16 (t, *J =* 1.1 Hz, 1H), 4.54 (s, 1H). |
| | | | MS-ESI: *m*/*z* 342.0 observed [M+H]⁺ |
| **2** | 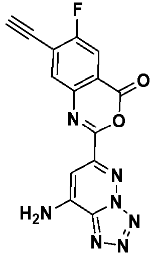 | ++++ | ¹H NMR (400 MHz, DMSO-*d*₆) δ 8.49 (s, 2H), 8.11 (d, *J =* 8.4 Hz, 1H), 8.03 (d, *J =* 6.3 Hz, 1H), 7.35 (s, 1H), 5.04 (s, 1H). |
| | | | MS-ESI: *m*/*z* 324.49 observed [M+H]⁺ |
| **3** | 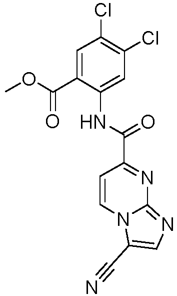 | ++++ | ¹H NMR (400 MHz, DMSO-*d*₆) δ 12.79 (s, 1H), 9.28 (d, *J =* 6.8 Hz, 1H), 9.04 (s, 1H), 8.95 (s, 1H), 8.21 (s,1H), 7.89 (d, *J= 6.8* HZ, 1H),4.00 (s, 3H) |
| | | | MS-ESI: *m*/*z* 390.42 observed [M+H]⁺ |
| **4** | 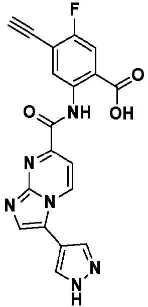 | + | ¹H NMR (400 MHz, DMSO-*d*₆) δ 12.95 (s, 1H), 9.20 (d, *J =* 7.2 Hz, 1H), 9.0 (d, *J =* 6.4 Hz, 1H), 8.41-8.21 (m, 3H), 7.92 (d, *J =* 10.8 Hz, 1H), 7.89 (d, *J =* 10 Hz, 1H), 7.76 (d, *J =* 7.2 Hz, 1H), 4.83 (s, 1H). |
| | | | MS-ESI: *m*/*z* 388.1 observed [M-H]⁻ |
| **5** | 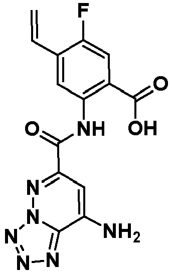 | ++ | ¹H NMR (400 MHz, DMSO) δ 15.73 (s, 1H), 8.90 (d, *J* = 7.1 Hz, 1H), 8.33 (s, 2H), 7.70 (d, *J =* 11.9 Hz, 1H), 7.15 (s, 1H), 6.93 - 6.76 (m, 1H), 5.86 (d, *J =* 17.7 Hz, 1H), 5.48 (d, *J =* 11.2 Hz, 1H). |
| | | | MS-ESI: *m*/*z* 344.52 observed [M+H]⁺ |
| **6** | 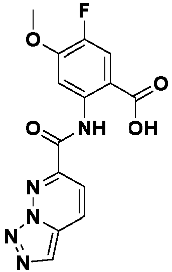 | +++ | ¹H NMR (400 MHz, DMSO-*d*₆) δ 16.32 (s, 1H), 8.70 (d, *J =* 9.2 Hz, 1H), 8.59 (d, *J =* 8.4 Hz, 1H), 8.49 (s, 1H), 7.92 (d, *J =* 9.2 Hz, 1H), 7.75 (d, *J =* 12.4 Hz, 1H), 3.89 (s, 3H). |
| | | | MS-ESI: *m*/*z* 332.1 observed [M+H]⁺ |
| **7** | 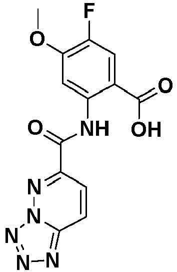 | +++ | ¹H NMR (400 MHz, DMSO-*d*₆) δ 8.97 (d, *J=* 9.4 Hz, 1H), 8.62 (d, *J=* 8.1 Hz, 1H), 8.36 (d, *J=* 9.4 Hz, 1H), 7.77 (d, *J =* 12.4 Hz, 1H), 7.10 (s, 2H), 3.92 (s, 3H). |
| | | | MS-ESI: *m*/*z* 333.52 observed [M+H]⁺ |
| **8** | 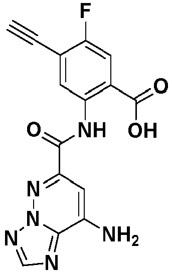 | ++ | ¹H NMR (400 MHz, DMSO-*d*₆) δ 15.22 (s, 1H), 8.83 (d, *J =* 2.4 Hz, 1H), 8.55 (s, 1H), 7.79 (d, *J =* 2.4 Hz, 1H), 7.67 (s, 2H), 7.13 (s, 1H), 4.53 (s, 1H). |
| | | | MS-ESI: *m*/*z* 341.0 observed [M+H]⁺ |
| **9** | 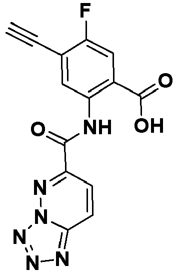 | +++ | ¹H NMR (400 MHz, DMSO-*d*₆) δ 13.02 (s, 1H), 9.02 (d, *J =* 9.5 Hz, 1H), 8.91 (d, *J =* 6.5 Hz, 1H), 8.40 (d, *J =* 9.4 Hz, 1H), 7.93 (d, *J =* 9.8 Hz, 1H), 4.88 (s, 1H). |
| | | | MS-ESI: *m*/*z* 327.46 observed [M+H]⁺ |
| **10** | 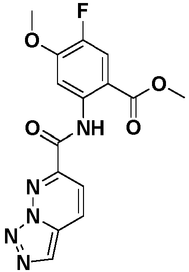 | ++++ | ¹H NMR (400 MHz, DMSO-*d*₆) δ 12.79 (s, 1H), 8.77 (d, *J =* 9.2 Hz, 1H), 8.64 (d, *J =* 8.0 Hz, 1H), 8.54 (s, 1H), 7.92 (d, *J =* 9.2 Hz, 1H), 7.75 (d, *J* = 12.4 Hz, 1H), 3.98 (s, 3H), 3.96 (s, 3H). |
| | | | MS-ESI: *m*/*z* 346.1 observed [M+H]⁺ |
| **11** | 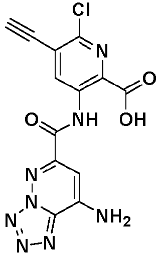 | ++ | ¹H NMR (400 MHz, DMSO-*d*₆) δ 15.37 (s, 1H), 9.18 (s, 1H), 8.39 (s, 2H), 7.17 (s, 1H), 7.01 (s, 1H), 4.82 (s, 1H). |
| | | | MS-ESI: *m*/*z* 359.0 observed [M+H]⁺ |
| **12** | 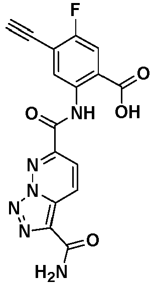 | +++ | ¹H NMR (400 MHz, DMSO-*d*₆) δ 8.86-8.81 (m, 2H), 8.32 (s, 1H),8.12 (d, *J =* 9.2 Hz,1H), 7.80 (d, *J =* 10.4 Hz, 2H), 7.15 (s, 3H), 4.56 (s, 1H). |
| | | | MS-ESI: *m*/*z* 369.1 observed [M+H]⁺ |
| **13** | 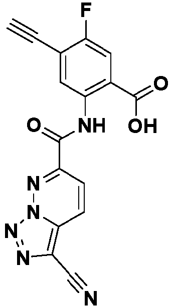 | ++ | ¹H NMR (400 MHz, DMSO-d6) δ 13.00 (s, 1H), 9.04 (d, *J =* 9.2 Hz, 1H), 8.88 (d, *J =* 6.4 Hz, 1H), 8.28 (d, *J =* 9.2 Hz, 1H), 7.91 (d, *J =* 9.6 Hz, 1H), 4.85 (s, 1H). |
| | | | MS-ESI: *m*/*z* 351.0 observed [M+H]⁺ |
| **14** | 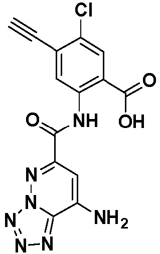 | +++ | ¹H NMR (400 MHz, DMSO) δ 15.84 (s, 1H), 8.88 (s, 1H), 8.34 (s, 2H), 8.04 (s, 1H), 7.15 (s, 1H), 4.61 (s, 1H). |
| | | | MS-ESI: *m*/*z* 358.47 observed [M+H]⁺ |
| **15** | 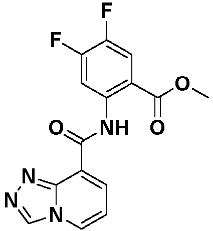 | ++ | ¹H NMR (500 MHz, DMSO) δ 12.48 (s, 1H), 9.50 (s, 1H), 8.86 (dd, *J =* 6.8, 1.1 Hz, 1H), 8.58 (dd, *J =* 13.6, 7.6 Hz, 1H), 8.24 (dd, *J =* 7.0, 1.1 Hz, 1H), 8.00 (dd, *J =* 11.2, 9.0 Hz, 1H), 7.24 (t, *J =* 6.9 Hz, 1H), 3.96 (s, 3H). |
| | | | MS-ESI: *m*/*z* 333.4 observed [M+H]⁺ |
| **16** | 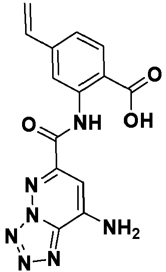 | ++ | ¹H NMR (400 MHz, DMSO-*d*₆) δ 16.00 (s, 1H), 8.75 (d, *J =* 2.0 Hz, 1H), 8.32 (s, 2H), 7.99 (d, *J =* 7.9 Hz, 1H), 7.25 - 7.11 (m, 2H), 6.75 (dd, *J* = 17.6, 10.9 Hz, 1H), 5.82 (d, *J =* 17.6 Hz, 1H), 5.31 (d, *J =* 10.8 Hz, 1H). |
| | | | MS-ESI: *m*/*z* 326.5 observed [M+H]⁺ |
| **17** | 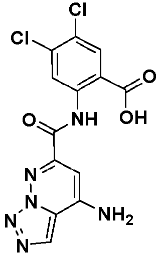 | ++ | 1H NMR (400 MHz, DMSO-d6) δ 14.06 (s, 1H), 8.99 (s, 1H), 8.45 (s, 1H), 8.16 (s, 1H), 8.05 (s, 2H), 6.87 (s, 1H). |
| | | | MS-ESI: *m*/*z* 367.0 observed [M+H]⁺ |
| **18** | 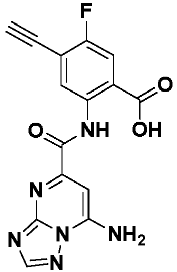 | ++ | ¹H NMR (400 MHz, DMSO-*d*₆) δ 15.07 (s, 1H), 8.88 (d, *J =* 6.4 Hz, 1H), 8.59 (s, 1H), 8.47 (s, 2H), 7.77 (d, *J =* 10.4 Hz, 1H), 7.07 (s, 1H), 4.52 (s, 1H). |
| | | | MS-ESI: *m*/*z* 341.2 observed [M+H]⁺ |
| **19** | 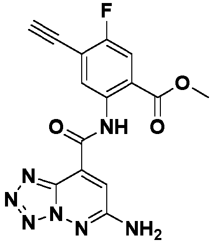 | ++++ | ¹H NMR (400 MHz, DMSO-*d*₆) δ 11.80 (s, 1H), 8.57 (d, *J =* 6.5 Hz, 1H), 7.88 (d, *J =* 9.7 Hz, 1H), 7.79 (s, 1H), 7.69 (s, 2H), 4.89 (s, 1H), 3.94 (s, 3H). |
| | | | MS-ESI: *m*/*z* 356.5 observed [M+H]⁺ |
| **20** | 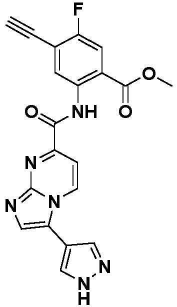 | ++ | ¹H NMR (400 MHz, DMSO-*d*₆) δ 13.44 (s, 1H), 12.66 (s, 1H), 9.22 (d, *J =* 7.2 Hz, 1H), 8.97 (d, *J=* 6.4 Hz, 1H), 8.47-8.26 (m, 4H), 7.92 (d, *J* = 10 Hz, 1H), 7.76 (d, *J =* 7.2 Hz, 1H), 4.87 (s, 1H), 4.02 (s, 3H). |
| | | | MS-ESI: *m*/*z* 405.1 observed [M+H]⁺ |
| **21** | 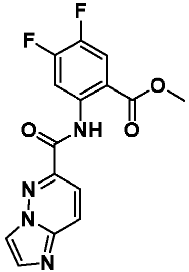 | + | ¹H NMR (500 MHz, DMSO-*d*₆) δ 12.51 (s, 1H), 8.75 (dd, *J =* 13.5, 7.6 Hz, 1H), 8.51 - 8.44 (m, 1H), 8.38 (d, *J =* 9.5 Hz, 1H), 8.08 (dd, *J=* 11.2, 8.9 Hz, 1H), 8.04 (d, *J =* 1.3 Hz, 1H), 7.87 (d, *J* = 9.5 Hz, 1H), 3.98 (s, 3H). |
| | | | MS-ESI: *m*/*z* 333.4 observed [M+H]⁺ |
| **22** | 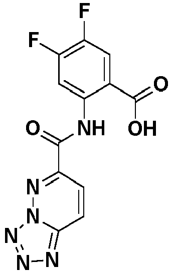 | ++ | ¹H NMR (400 MHz, DMSO-*d*₆) δ 16.79 (s, 1H), 8.94 (d, *J* =9.6 Hz, 1H), 8.68 (dd, *J =* 7.6, 13.6 Hz, 1H), 8.34 (d, *J =* 9.6 Hz, 1H), 7.94 (dd, *J =* 9.6, 11.6 Hz, 1H). |
| **23** | 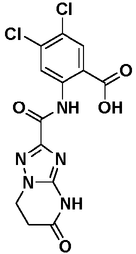 | + | ¹H NMR (400 MHz, DMSO-*d*₆) δ 15.23 (s, 1H), 11.61 (s, 1H), 8.87 (s, 1H), 8.09 (s, 1H), 4.33 (t, *J =* 7.2 Hz, 2H), 2.90 (t, *J =* 7.2 Hz, 2H). |
| | | | MS-ESI: *m*/*z* 370.11 observed [M+H]⁺ |
| **24** | 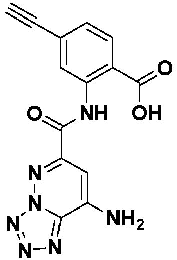 | ++ | ¹H NMR (400 MHz, DMSO-*d*₆) δ 15.77 (s, 1H), 8.78 (s, 1H), 8.20 (s, 1H), 8.00 (d, *J =* 8 Hz, 1H), 7.13 (d, *J =* 8 Hz, 1H), 7.10-7.04 (m, 1H), 4.22 (s, 1H). |
| | | | MS-ESI: *m*/*z* 324.0 observed [M+H]⁺ |
| **25** | 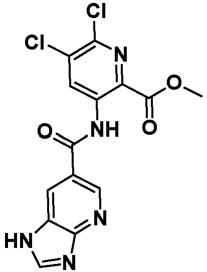 | + | ¹H NMR (400 MHz, DMSO-*d*₆) δ 13.45 (s, 1H), 11.41 (s, 1H), 9.03 (s, 1H), 8.972-8.968 (m, 1H), 8.68 (s, 1H), 8.55 (s, 1H), 3.93 (s, 3H). |
| | | | MS-ESI: *m*/*z* 366.24 observed [M+H]⁺ |
| **26** | 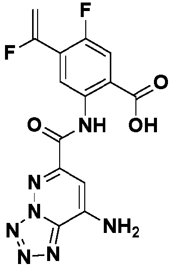 | ++ | ¹H NMR (400 MHz, DMSO-*d*₆) δ 15.72 (s, 1H), 8.94 (d, *J =* 7.3 Hz, 1H), 8.34 (s, 2H), 7.79 (dd, *J =* 12.5, 1.3 Hz, 1H), 7.14 (s, 1H), 5.42-5.18 (m, 2H). |
| | | | MS-ESI: *m*/*z* 362.53 observed [M+H]⁺ |
| **27** | 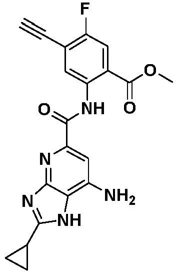 | + | ¹H NMR (400 MHz, DMSO-*d*₆) δ 12.51 (s, 1H), 9.02 (d, *J =* 6.8 Hz, 1Hz), 7.86 (d, *J =* 10 Hz, 1H), 7.27 (s, 1H), 6.40 (s, 2H), 4.82 (s, 1H), 3.98 (s, 3H), 2.17-2.07 (m, 1H), 1.07 (d, *J =* 4 Hz, 4H). |
| | | | MS-ESI: *m*/*z* 394.54 observed [M+H]⁺ |
| **28** | 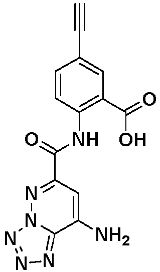 | + | ¹H NMR (400 MHz, DMSO-*d*₆) δ 15.77 (s, 1H), 8.65 (d, *J =* 8.4 Hz, 1H), 8.33 (s, 2H), 8.10 (s, 1H), 7.48-7.46 (m, 1H), 7.22 (s, 3H), 7.17 (s, 1H), 4.07 (s, 1H). |
| | | | MS-ESI: *m*/*z* 324.0 observed [M+H]⁺ |
| **29** | 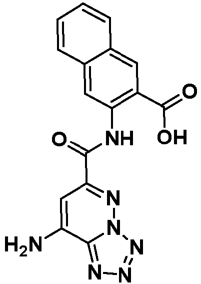 | + | ¹H NMR (400 MHz, DMSO) δ 16.13 (s, 1H), 9.11 (s, 1H), 8.58 (s, 1H), 8.33 (s, 2H), 7.89 (d, J = 8.1 Hz, 1H), 7.83 (d, J = 8.3 Hz, 1H), 7.48 (t, J = 7.5 Hz, 1H), 7.43-7.36 (m, 1H), 7.23-7.17 (m, 1H). |
| | | | MS-ESI: *m*/*z* 350.48 observed [M+H]⁺ |
| **30** | 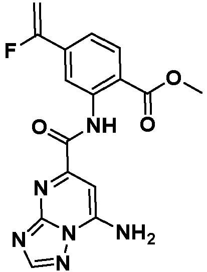 | ++++ | ¹H NMR (400 MHz, DMSO-*d*₆) δ 12.80 (s, 1H), 9.05 (s, 1H), 8.62 (s, 1H), 8.11 (d, *J =* 8.4 Hz, 1H), 7.56 (d, *J =* 8.8 Hz, 1H), 7.010 (s, 1H), 5.57 (dd, *J* = 4, 51.6 Hz, 1H), 5.23-5.17 (m, 1H), 3.99 (s, 3H). |
| | | | MS-ESI: *m*/*z* 357.2 observed [M+H]⁺ |
| **31** | 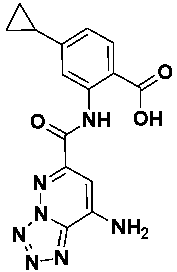 | + | ¹H NMR (400 MHz, DMSO) δ 16.05 (s, 1H), 8.41 (s, 1H), 8.30 (s, 2H), 7.88 (d, J = 8.2 Hz, 1H), 7.13 (d, J = 2.6 Hz, 1H), 6.73 (d, J = 8.3 Hz, 1H), 1.95-1.85 (m, 1H), 1.02-0.93 (m, 2H), 0.73-0.63 (m, 2H). |
| | | | MS-ESI: *m*/*z* 340.50 observed [M+H]⁺ |
| **32** | 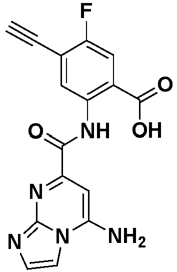 | ++ | ¹H NMR (400 MHz, DMSO) δ 14.88 (s, 1H), 8.87 (d, J = 6.7 Hz, 1H), 7.89 (s, 3H), 7.72 (d, J = 10.6 Hz, 1H), 7.68 (s, 1H), 6.79 (s, 1H), 4.50 (s, 1H). |
| | | | MS-ESI: *m*/*z* 340.49 observed [M+H]⁺ |
| **33** | 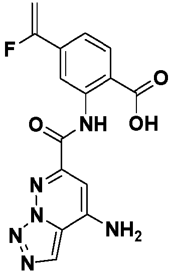 | ++ | ¹H NMR (400 MHz, DMSO-*d*₆) δ 15.79 (s, 1H), 8.95 (s, 1H), 8.48 (s, 1H), 8.09 (d, *J =* 8 Hz, 1H), 7.98 (s, 2H), 7.37-7.34 (m, 1H), 6.89 (s, 1H), 5.43 (d, *J =* 3.6 Hz, 1H), 5.3 (d, *J =* 4 Hz, 1H). |
| | | | MS-ESI: *m*/*z* 343.0 observed [M+H]⁺ |
| **34** | 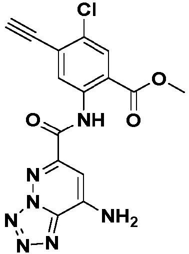 | ++++ | ¹H NMR (400 MHz, DMSO-*d*₆) δ 12.48 (s, 1H), 8.94 (s, 1H), 8.57 (s, 2H), 8.14 (s, 1H), 7.19 (s, 1H), 4.97 (s, 1H), 3.98 (s, 3H). |
| | | | MS-ESI: *m*/*z* 372.38 observed [M+H]⁺ |
| **35** | 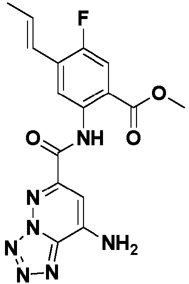 | ++++ | ¹H NMR (400 MHz, DMSO-*d*₆) δ 15.73 (s, 1H), 8.85 (d, *J =* 7.3 Hz, 1H), 8.33 (s, 2H), 7.67 (d, *J=* 11.8 Hz, 1H), 7.15 (s, 1H), 6.54 (dd, *J =* 16.0, 1.9 Hz, 1H), 6.36 (dq, *J* = 15.9, 6.6 Hz, 1H), 1.93 (dd, *J =* 6.6, 1.6 Hz, 3H). |
| | | | MS-ESI: *m*/*z* 372.53 observed [M+H]⁺ |
| **36** | 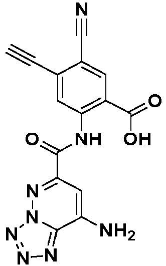 | +++ | ¹H NMR (400 MHz, DMSO-*d*₆) δ 16.12 (s, 1H), 8.96 (s, 1H), 8.39-8.37 (m, 3H), 7.17 (s, 1H), 4.82 (s, 1H). |
| | | | MS-ESI: *m*/*z* 349.1 observed [M+H]⁺ |
| **37** | 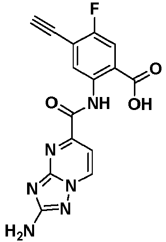 | + | ¹H NMR (400 MHz, DMSO-*d*₆) δ 15.02 (s, 1H), 9.11 (d, *J =* 6.8 Hz, 1H), 8.89 (d, *J =* 6.8 Hz, 1H), 7.78 (d, *J* = 10 Hz, 1H), 7.63 (d, *J* = 6.4 Hz, 1H), 7.29 (s, 3H), 6.68 (s, 2H), 4.56 (s, 1H) |
| | | | MS-ESI: *m*/*z* 341.2 observed [M+H]⁺ |
| **38** | 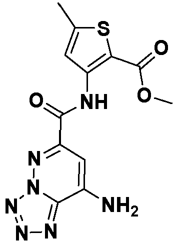 | ++ | ¹H NMR (400 MHz, DMSO-*d*₆) δ 11.87 (s, 1H), 8.55 (s, 2H), 7.96 (s, 1H), 7.18 (s, 1H), 3.89 (s, 3H), 2.56 (s, 3H). |
| | | | MS-ESI: *m*/*z* 334.44 observed [M+H]⁺ |
| **39** | 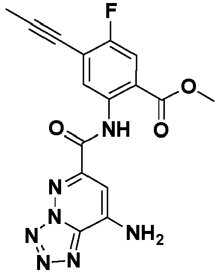 | ++ | ¹H NMR (400 MHz, DMSO-d6) δ 12.41 (s, 1H), 8.66 (d, *J =* 7.1 Hz, 1H), 8.52 (s, 2H), 7.76 (d, *J= 10.2* Hz, 1H), 7.18 (s, 1H), 3.95 (s, 3H), 2.36 (s, 3H). |
| | | | MS-ESI: *m*/*z* 370.44 observed [M+H]⁺ |
| **40** | 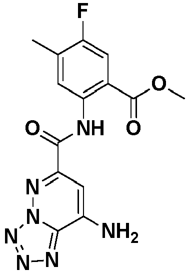 | ++++ | ¹H NMR (400 MHz, DMSO-d6) δ 12.40 (s, 1H), 8.78 (d, *J =* 6.7 Hz, 1H), 8.53 (s, 2H), 7.86 (d, *J= 9.9* Hz, 1H), 7.18 (s, 1H), 3.96 (s, 3H), 2.17 (s, 3H). |
| | | | MS-ESI: *m*/*z* 346.45 observed [M+H]⁺ |
| **41** | 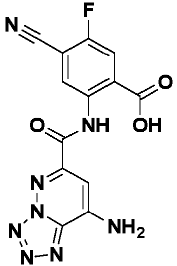 | ++ | ¹H NMR (400 MHz, DMSO-*d*₆) δ 15.71 (s, 1H), 9.04 (d, *J =* 6 Hz, 1H), 8.36 (s, 2H), 7.94 (d, *J =* 10Hz, 1H), 7.17 (s, 1H). |
| | | | MS-ESI: *m*/*z* 343.0 observed [M+H]⁺ |
| **42** | 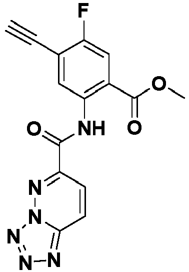 | ++++ | ¹H NMR (400 MHz, DMSO-*d*₆) δ 12.50 (s, 1H), 9.03 (d, *J =* 9.4 Hz, 1H), 8.78 (d, *J =* 6.6 Hz, 1H), 8.40 (d, *J =* 9.5 Hz, 1H), 7.94 (d, *J =* 9.7 Hz, 1H), 4.91 (s, 1H), 4.10(s, 3H). |
| | | | MS-ESI: *m*/*z* 341.49 observed [M+H]⁺ |
| **43** | 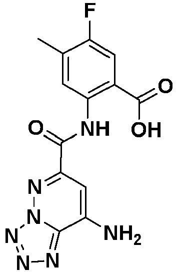 | ++ | ¹H NMR (499 MHz, DMSO-d6) δ 8.60-8.54 (m, 1H), 8.29 (s, 2H), 7.64 (d, *J =* 10.7 Hz, 1H), 7.15 (s, 1H), 2.26 (s, 3H). |
| | | | MS-ESI: *m*/*z* 332.43 observed [M+H]⁺ |
| **44** | 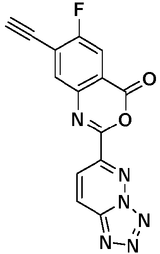 | ++++ | ¹H NMR (499 MHz, DMSO-*d*₆) δ 9.01 (d, *J=* 9.5 Hz, 1H), 8.52 (d, *J=* 9.5 Hz, 1H), 8.29-8.01 (m, 2H), 5.05 (s, 1H). |
| | | | MS-ESI: *m*/*z* 309.05 observed [M+H]⁺ |
| **45** | 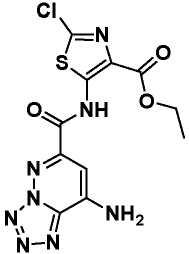 | ++ | ¹H NMR (400 MHz, DMSO-*d*₆) δ 12.2 (s, 1H), 8.71 (s, 2H), 7.15 (s, 1H), 4.47-4.41 (m, 2H), 1.42 (t, *J =* 7.2 Hz, 3H). |
| | | | MS-ESI: *m*/*z* 368.9observed [M+H]⁺ |
| **46** | 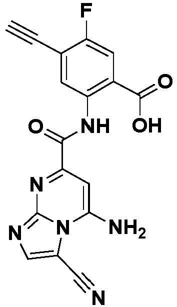 | + | ¹H NMR (400 MHz, DMSO-*d*₆) δ 15.06 (s, 1H), 8.88 (d, *J =* 6.8 Hz, 1H), 7.79 (d, *J* = 10.4 Hz, 1H), 7.76 (s, 2H), 7.05 (s, 1H), 4.53 (s, 1H). |
| | | | MS-ESI: *m*/*z* 365.2 observed [M+H]⁺ |
| **47** | 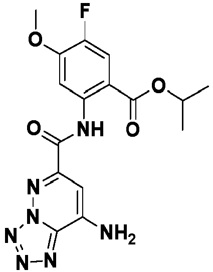 | ++ | ¹H NMR (400 MHz, DMSO-*d*₆) δ 12.59 (s, 1H), 8.23 (d, *J* = 8 Hz, 1H), 8.52 (s, 2H), 7.81 (d, *J =* 12 Hz, 1H), 7.18 (s, 1H), 5.30-5.24 (m, 1H), 3.97 (s, 3H), 1.38 (d, *J* = 6.4 Hz, 6H). |
| **48** | 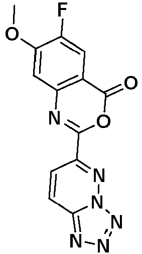 | ++++ | ¹H NMR (400 MHz, DMSO-*d*₆) δ 9.02 (d, *J=* 9.5 Hz, 1H), 8.52 (d, *J=* 9.5 Hz, 1H), 8.06 (d, *J =* 10.6 Hz, 1H), 7.64 (d, *J=* 7.7 Hz, 1H), 4.09 (s, 3H). |
| | | | MS-ESI: *m*/*z* 315.1 observed [M+H]⁺ |
| **49** | 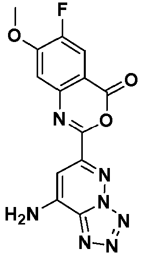 | ++++ | ¹H NMR (400 MHz, DMSO-*d*₆) δ 8.45 (s, 2H), 8.00 (d, *J=* 10.6 Hz, 1H), 7.53 (d, *J =* 7.7 Hz, 1H), 7.36 (s, 1H), 4.08 (s, 3H). |
| | | | MS-ESI: *m*/*z* 330.1 observed [M+H]⁺ |
| **50** | 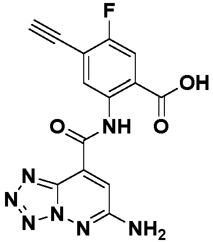 | ++ | 1H NMR (499 MHz, DMSO) δ 8.70 (d, J = 6.6 Hz, 1H), 7.83 (d, J = 9.8 Hz, 1H), 7.68 (s, 1H), 7.62 (s, 2H), 4.75 (s, 1H). |
| | | | MS-ESI: *m*/*z* 342.1 observed [M+H]⁺ |
| **51** | 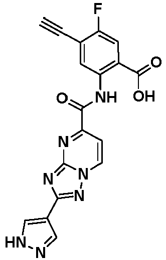 | + | ¹H NMR (400 MHz, DMSO-*d*₆) δ 14.91 (s, 1H), 9.51 (d, *J =* 6.8 Hz, 1H) 8.93 (d, *J =* 6.8 Hz, 1H), 8.32 (s, 2H), 8.15 (s, 1H), 7.89 (d, *J =* 2.8 Hz), 7.84 (d, *J =* 10 Hz), 4.62 (s, 1H). |
| | | | MS-ESI: *m*/*z* 392.0 observed [M+H]⁺ |
| **52** | 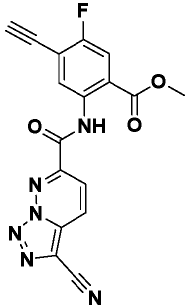 | ++ | ¹H NMR (400 MHz, DMSO-*d*₆) δ 12.47 (s, 1H), 9.06 (d, *J =* 9.2 Hz, 1H), 8.77 (d, *J =* 6.4 Hz, 1H), 8.28 (d, *J =* 9.2 Hz, 1H), 7.93 (d, *J =* 10 Hz, 1H), 4.89 (s,1H), 3.97 (s, 3H). |
| | | | MS-ESI: *m*/*z* 365.1 observed [M+H]⁺ |
| **53** | 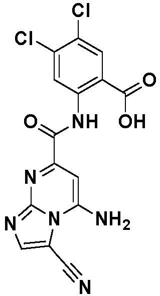 | | ¹H NMR (400 MHz, DMSO-*d*₆) δ 15.28 (s, 1H), 8.97 (s, 1H), 8.71 (s, 1H), 8.46 (s, 2H), 8.13 (s, 1H), 6.94 (s, 1H). |
| | | | MS-ESI: *m*/*z* 391.0 observed [M+H]⁺ |
| **54** | 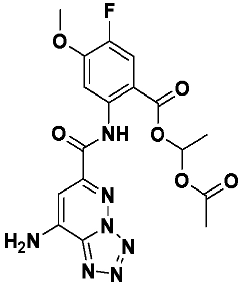 | ++++ | ¹H NMR (400 MHz, DMSO-*d*₆) δ 12.43 (s, 1H), 8.62 (d, *J =* 8.1 Hz, 1H), 8.53 (s, 2H), 7.83 (d, *J =* 12.0 Hz, 1H), 7.17 (s, 1H), 7.04 (d, *J =* 5.5 Hz, 1H), 3.99 (s, 3H), 2.09 (s, 3H), 1.60 (d, *J =* 5.4 Hz, 3H). |
| | | | MS-ESI: *m*/*z* 434.1 observed [M+H]⁺ |
| **55** | 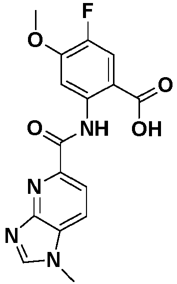 | + | ¹H NMR (400 MHz, DMSO-*d*₆) δ 15.19 (s, 1H), 8.72 (d, *J =* 8.4 Hz, 1H), 8.61 (s, 1H), 8.17 (dd, *J =* 8.4, 33.2 Ha, 2H), 7.72 (d, *J =* 13.2 Hz, 1H), 3.93 (s, 3H), 3.87 (s, 3H). |
| | | | MS-ESI: *m*/*z* 345.0 observed [M+H]⁺ |
| **56** | 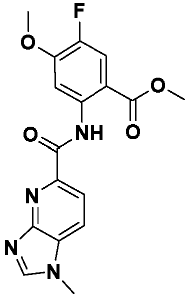 | ++ | ¹H NMR (400 MHz, DMSO-*d*₆) δ 12.97 (s, 1H), 8.82 (d, *J =* 8.4 Hz, 1H), 8.68 (s, 1H), 8.30 (d, *J =* 8.4 Hz, 1H), 8.18 (d, *J* = 8.4Hz, 1H), 7.80 (d, *J =* 12 Hz, 1H), 3.97 (s, 6H), 3.95 (s, 3H). |
| | | | MS-ESI: *m*/*z* 359.2 observed [M+H]⁺ |
| **57** | 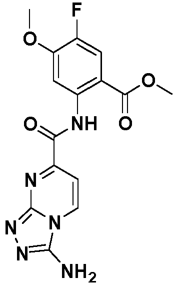 | + | ¹H NMR (400 MHz, DMSO-*d*₆) δ 12.90 (s, 1H), 9.19 (d, *J =* 6.4 Hz, 1H), 8.73 (d, *J =* 8.0 Hz, 1H), 7.83 (d, *J =* 12 Hz, 1H), 7.67 (d, *J =* 6.8 Hz, 1H), 6.78 (s, 2H), 3.97 (s, 6H), 3.95 (s, 3H). |
| | | | MS-ESI: *m*/*z* 360.9 observed [M+H]⁺ |
| **58** | 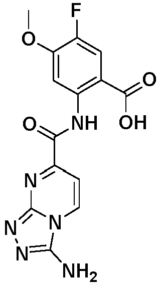 | + | ¹H NMR (400 MHz, DMSO-*d*₆) δ 9.16 (d, *J* = 6.8 Hz, 1H), 8.72 (d, *J* = 8.4 Hz, 1H), 7.78 (d, *J* = 12 Hz, 1H), 7.66 (d, *J =* 6.8 Hz, 1H), 6.71 (s, 2H), 3.94 (s, 3H). |
| | | | MS-ESI: *m*/*z* 346.9 observed [M+H]⁺ |
| **59** | 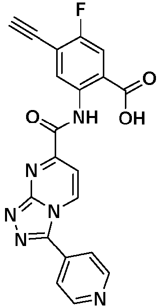 | + | ¹H NMR (400 MHz, DMSO-*d*₆) δ 15.78 (s, 1H), 9.66 (d, *J* = 6.8 Hz, 1H), 8.91 (d, *J* = 6.8 Hz, 1H), 8.85-8.83 (m, 2H), 8.22-8.21 (m, 2H), 8.01 (d, *J* = 6.8 Hz, 1H), 7.81 (d, *J* = 10.4 Hz, 1H), 7.16 (s, 1H), 4.57 (s, 1H). |
| **60** | 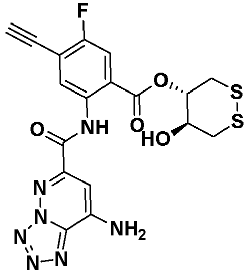 | ++ | ¹H NMR (400 MHz, DMSO-*d*₆) δ 12.26 (s, 1H), 8.83 (d, *J* = 6.6 Hz, 1H), 8.53 (s, 2H), 8.04 (d, *J* = 9.8 Hz, 1H), 7.19 (s, 1H), 5.72 (d, *J* = 5.5 Hz, 1H), 5.06 (ddd, *J* = 10.3, 8.7, 3.8 Hz, 1H), 4.89 (s, 1H), 3.87 (tt, *J =* 9.6, 4.9 Hz, 1H), 3.27-3.20 (m, 1H), 3.16 (dd, *J =* 13.4, 10.4 Hz, 1H), 3.03-2.96 (m, 2H). |
| | 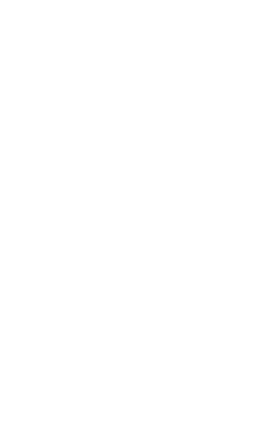 | | MS-ESI: *m*/*z* 476.0 observed [M+H]⁺ |
| **61** | 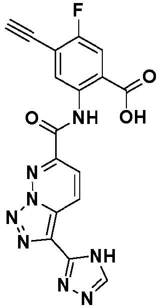 | ++ | ¹H NMR (400 MHz, DMSO-*d*₆) δ 16.19 (s, 1H), 8.92 (d, *J* = 9.2 Hz, 1H), 8.85 (d, *J* = 6.8 Hz, 1H), 8.65 (s, 1H), 8.07 (d, *J* = 9.2 Hz, 1H), 7.79 (d, *J* = 10.4 Hz, 1H), 4.55 (s, 1H). |
| | | | MS-ESI: *m*/*z* 393.3 observed [M+H]⁺ |
| **62** | 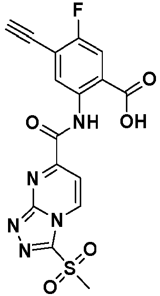 | + | MS-ESI: *m*/*z* 404.1 observed [M+H]⁺ |
| **63** | 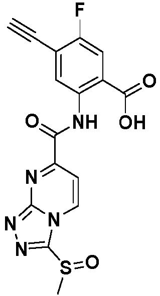 | + | ¹H NMR (400 MHz, DMSO-*d*₆) δ 14.71 (s, 1H), 9.72 (d, *J* = 6.8 Hz, 1H), 8.93 (d, *J* = 6.4, 1H), 8.08 *(d, J* = 6.8, 1H), 7.84 (d, *J* = 10 Hz, 1H), 4.678 (s, 1H), 3.15 (s, 3H). |
| | | | MS-ESI: *m*/*z* 387.9 observed [M+H]⁺ |
| **64** | 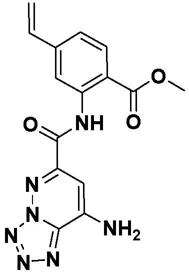 | ++++ | MS-ESI: *m*/*z* 340.54 observed [M+H]⁺ |
| **65** | 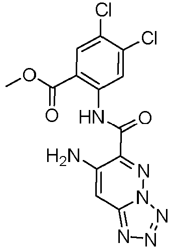 | ++++ | MS-ESI: *m*/*z* 382.38 observed [M+H]⁺ |
| **66** | 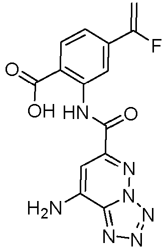 | ++++ | ¹H NMR (400 MHz, DMSO-*d*₆) δ 15.99 (s, 1H), 8.90 (d, *J* = 1.8 Hz, 1H), 8.33 (s, 2H), 8.05 (d, *J* = 8.1 Hz, 1H), 7.32 (d, *J* = 8.3 Hz, 1H), 7.15 (s, 1H), 5.41 - 5.24 (m, 1H), 4.98 (dd, *J* = 18.5, 3.5 Hz, 1H). |
| | | | MS-ESI: *m*/*z* 344.48 observed [M+H]⁺ |
| **67** | 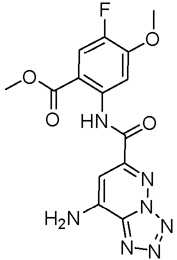 | ++++ | ¹H NMR (400 MHz, DMSO-*d*₆) δ 12.70 (s, 1H), 8.67 - 8.61 (m, 1H), 8.55 (s, 2H), 7.84 (dt, *J* = 12.1, 1.9 Hz, 1H), 7.17 (d, *J* = 1.5 Hz, 1H), 3.97 (s, 3H), 3.94 (s, 3H). |
| **68** | 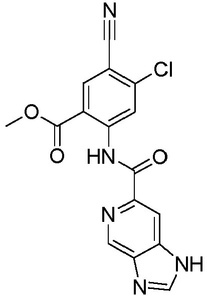 | ++ | ¹H NMR (400 MHz, DMSO-*d*₆) δ 13.26 (s, 1H), 9.23 (s, 1H), 9.12 (d, *J* = 0.8 Hz, 1H), 8.63 (s, 1H), 8.55 (s, 1H), 8.43 (d, *J* = 0.8 Hz,1H), 4.00 (s, 3H) |
| | | | MS-ESI: *m*/*z* 356.19 observed [M+H]⁺ |
| **69** | 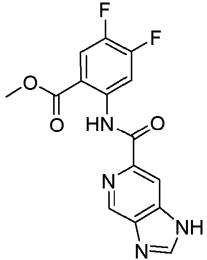 | ++++ | ¹H NMR (400 MHz, DMSO-*d*₆) δ 13.05 (s, 1H), 9.12 (d, *J* = 1.8 Hz, 1H), 8.97 (dd, *J* = 13.9, 7.8 Hz, 1H), 8.63 (d, *J* = 1.6 Hz, 1H), 8.43 (s, 1H), 8.08 (dd, *J* = 11.4, 9.0 Hz, 1H), 3.97 (s, 3H). |
| | | | MS-ESI: *m*/*z* 333.44 observed [M+H]⁺ |
| **70** | 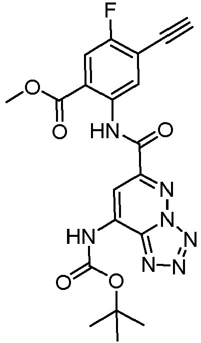 | ++ | ¹H NMR (400 MHz, DMSO-*d*₆) δ 12.51 (s, 1H), 11.56 (s, 1H), 8.86 (d, *J* = 6.4 Hz, 1H), 8.72 (s, 1H), 7.99 (d, *J* = 9.6 Hz, 1H), 4.94 (s, 1H), 4.04 (s, 3H), 1.62 (s, 9H). |
| | | | MS-ESI: *m*/*z* 456.2 observed [M+H]⁺ |
| **71** | 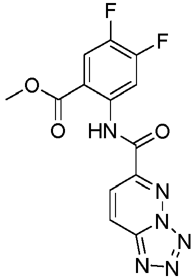 | ++ | ¹H NMR (400 MHz, DMSO-*d*₆) δ 12.71 (s, 1H), 9.04 (d, *J* = 9.6 Hz, 1H), 8.73 (dd, *J* = 7.6, 13.2 Hz, 1H), 8.40 (d, *J* = 9.6 Hz, 1H), 8.13 (dd, *J* = 7.6, 13.2 Hz, 1H), 3.99 (s, 3H) |
| | | | MS-ESI: *m*/*z* 335.18 observed [M+H]⁺ |
| **72** | 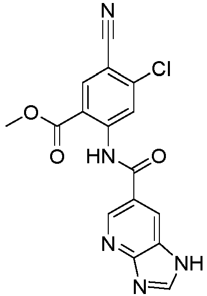 | + | ¹H NMR (400 MHz, DMSO-*d*₆) δ 11.88 (s, 1H), 8.98 (d, *J* = 2.4 Hz, 1H), 8.87 (s, 1H), 8.70 (s, 1H), 8.55 (d, *J* = 2 Hz, 1H), 8.53 (s, 1H), 3.95 (s, 3H) |
| | | | MS-ESI: *m*/*z* 356.21 observed [M+H]⁺ |
| **73** | 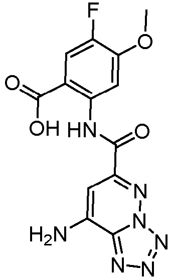 | ++ | ¹H NMR (400 MHz, DMSO-*d*₆) δ 16.06 (s, 1H), 8.56 (d, *J* = 8.2 Hz, 1H), 8.33 (s, 2H), 7.71 (dd, *J* = 13.0, 1.7 Hz, 1H), 7.13 (dd, *J* = 1.8, 0.9 Hz, 1H), 3.86 (s, 3H). |
| | | | MS-ESI: *m*/*z* 348.51 observed [M+H]⁺ |
| **74** | 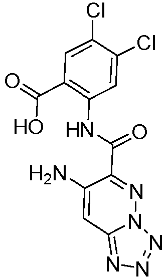 | +++ | ¹H NMR (400 MHz, DMSO-*d*₆) δ 8.91 (s, 1H), 8.17 (s, 1H), 7.58 (s, 2H), 7.34 (s, 1H). |
| | | | MS-ESI: *m*/*z* 368.4 observed [M+H]⁺ |
| **75** | 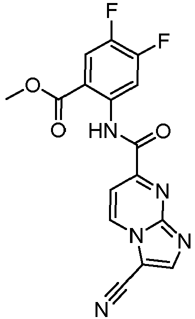 | ++ | ¹H NMR (400 MHz, DMSO-*d*₆) δ 12.85 (s, 1H), 9.28 (d, *J* = 7.2 Hz, 1H)), 8.95 (s, 1H), 8.81 dd, *J* = 7.6, 11.6 Hz, 1H), 8.10 (dd, *J* = 9.2, 11.2 Hz, 1H), 7.89 (d, J = 7.2 Hz, 1H), 3.99 (s, 3H) |
| | | | MS-ESI: *m*/*z* 356.16 observed [M+H]⁺ |
| **76** | 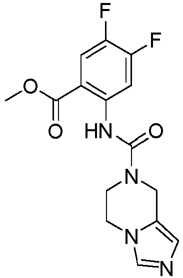 | ++ | ¹H NMR (400 MHz, MeOD) δ 8.89 (s, 1H), 8.44 (dd, *J* = 13.8, 7.6 Hz, 1H), 7.96 (dd, *J* = 11.2, 8.9 Hz, 1H), 7.50 (s, 1H), 4.95 (s, 2H), 4.47 (t, *J* = 5.5 Hz, 2H), 4.08 (t, *J* = 5.5 Hz, 2H), 3.97 (s, 3H). |
| | | | MS-ESI: *m*/*z* 337.5 observed [M+H]⁺ |
| **77** | 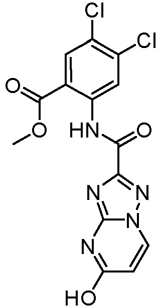 | ++ | ¹H NMR (400 MHz, DMSO-*d*₆) δ 12.2 (s, 1H), 9.03 (s, 1H), 8.54 (d, *J* = 7.6 Hz, 1H), 8.18 (s,1H), 7.18 (s, 1H), 6.16 (d, *J* = 7.6, 1H), 3.95 (s, 3H) |
| | | | MS-ESI: *m*/*z* 382.09 observed [M+H]⁺ |
| **78** | 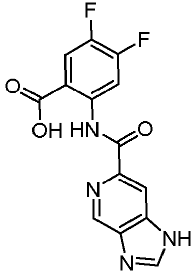 | ++ | ¹H NMR (400 MHz, DMSO-*d*₆) δ 13.25 (s, 1H), 9.06 (s, 1H), 8.96 (dd, *J* = 13.9, 7.5 Hz, 1H), 8.62 (s, 1H), 8.41 (s, 1H), 8.04 (dd, *J* = 11.3, 9.0 Hz, 1H). |
| | | | MS-ESI: *m*/*z* 319.42 observed [M+H]⁺ |
| **79** | 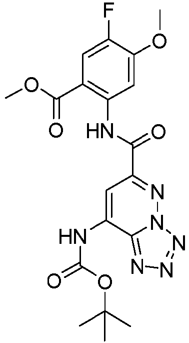 | ++ | ¹H NMR (400 MHz, DMSO) δ 13.46 (s, 1H), 9.28 (d, *J* = 3.3 Hz, 1H), 9.10 (dd, *J* = 8.0, 2.6 Hz, 1H), 9.05 (d, *J* = 2.7 Hz, 1H), 8.19 (dd, *J* = 11.9, 2.7 Hz, 1H), 4.46 (d, *J* = 2.2 Hz, 6H), 2.05 (d, *J* = 2.9 Hz, 9H). |
| **80** | 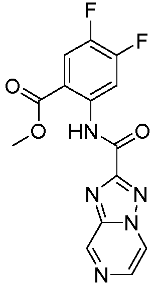 | ++ | ¹H NMR (400 MHz, DMSO) δ 12.60 (s, 1H), 9.63 (d, *J* = 1.6 Hz, 1H), 9.27-9.25 (m, 1H), 8.49 (dd, *J* = 7.6, 13.6 Hz, 1H), 8.44 (d, *J* = 4.4 Hz, 1H), 8.10 (dd, *J* = 8, 19.2 Hz, 1H), 3.97 (s, 3H) |
| | | | MS-ESI: *m*/*z* 334.15 observed [M+H]⁺ |
| **81** | 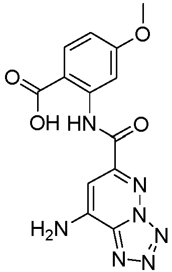 | ++ | ¹H NMR (400 MHz, DMSO) δ 8.439 (s, 2H), 8.41-8.40 (m, 1H), 8.03, (d, *J* = 8.8 Hz, 1H), 7.18 (s, 1H), 6.81-6.79 (m, 1H), 3.861 (s, 3H) |
| | | | MS-ESI: *m*/*z* 330.0 observed [M+H]⁺ |
| **82** | 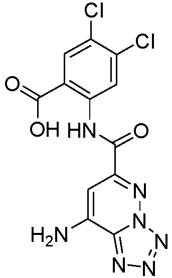 | ++ | ¹H NMR (400 MHz, DMSO-*d*₆) δ 15.94 (s, 1H), 8.91 (d, *J* = 1.9 Hz, 1H), 8.36 (s, 2H), 8.16 - 8.08 (m, 1H), 7.18 - 7.11 (m, 1H). |
| | | | MS-ESI: *m*/*z* 368.0 observed [M+H]⁺ |
| **83** | 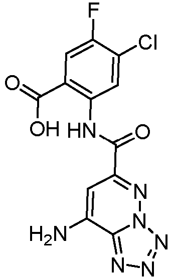 | ++++ | ¹H NMR (400 MHz, DMSO-*d*₆) δ 15.85 (s, 1H), 8.86 (dd, *J* = 7.0, 1.6 Hz, 1H), 8.35 (s, 2H), 7.87 (dd, *J* = 10.4, 1.8 Hz, 1H), 7.15 (d, *J* = 2.3 Hz, 1H). |
| | | | MS-ESI: *m*/*z* 352.4 observed [M+H]⁺ |
| **84** | 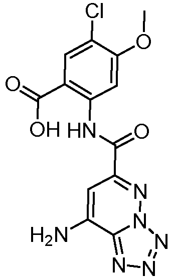 | ++ | ¹H NMR (400 MHz, DMSO-*d*₆) δ 8.59 (s, 1H), 8.41 (s, 2H), 7.99 (s, 1H), 7.15 (s, 1H), 3.92 (s, 3H). |
| | | | MS-ESI: *m*/*z* 364.45 observed [M+H]⁺ |
| **85** | 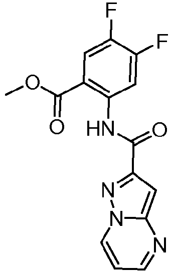 | + | MS-ESI: *m*/*z* 333.47 observed [M+H]⁺ |
| **86** | 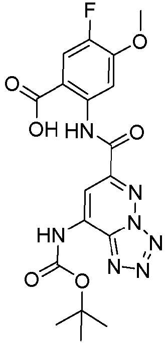 | ++ | ¹H NMR (400 MHz, DMSO-*d*₆) δ 11.52 (s, 2H), 8.72 - 8.69 (m, 2H), 7.83 (d, *J* = 12.2 Hz, 2H), 6.56 (s, 2H), 3.98 (s, 3H), 1.55 (s, 9H). |
| | | | MS-ESI: *m*/*z* 448.46 observed [M+H]⁺ |
| **87** | 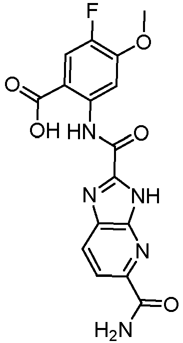 | + | ¹H NMR (400 MHz, DMSO-*d*₆) δ 13.82 (s, 1H), 9.28 (d, *J* = 8.8 Hz, 1H), 7.92 (s, 1H), 7.91-7.77 (m, 1H), 7.75-7.71 (m, 1H), 7.61 (d, J = 8 Hz, 1H), 7.21 (s, 1H), 3.98 (s, 3H) |
| | | | MS-ESI: *m*/*z* 374.0 observed [M+H]⁺ |
| **88** | 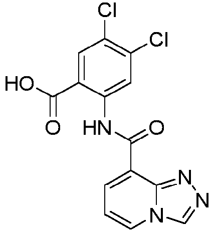 | + | ¹H NMR (400 MHz, DMSO*-d₆*): *δ* 15.34 (s, 1H), 9.39 (s, 1H), 8.84 (s, 1H), 8.74 (dd, *J* = 6.8, 0.8 Hz, 1H), 8.11 (s, 1H), 7.81 (dd, *J* = 6.8, 1.2 Hz, 1H), 7.13 (t, *J* = 7.2 Hz, 1H). |
| | | | MS-ESI: *m*/*z* 351.13 observed [M+H]⁺ |
| **89** | 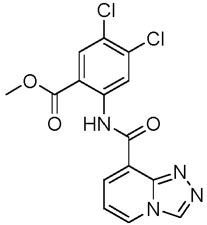 | ++ | ¹H NMR (400 MHz, DMSO-*d*₆) δ 12.48 (s, 1H), 9.50 (s, 1H), 8.87 (d, *J* = 1.2 Hz, 1H), 8.86-8.82 (m, 1H), 8.24 (dd, *J* = 0.8, 6.8 Hz, 1H), 8.12 (s, 1H), 7.24 (t, *J* = 7.2 Hz, 1H), 3.98 (s, 3H) |
| | | | MS-ESI: *m*/*z* 365.11 observed [M+H]⁺ |
| **90** | 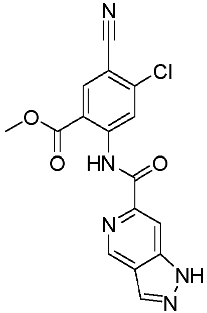 | ++ | ¹H NMR (400 MHz, DMSO-*d*₆) δ 13.96 (s, 1H), 13.29 (s, 1H), 9.30 (d, *J* = 1.2 Hz), 9.21 (s, 1H), 8.54 (s, 1H), 8.50 (s, 1H), 8.35 (s, 1H), 3.99 (s, 3H) |
| | | | MS-ESI: *m*/*z* 356.16 observed [M+H]⁺ |
| **91** | 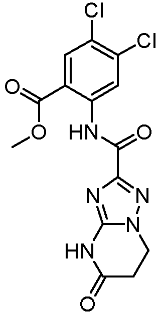 | ++ | ¹H NMR (400 MHz, DMSO-*d*₆) δ 12.04 (s, 1H), 11.71 (s,1H), 9.00 (s, 1H), 8.17 (s, 1H), 4.39 (t, *J* = 7.6 Hz, 2H), 3.93 (s, 3H), 2.94 (t, *J* = 7.6 Hz, 2H) |
| | | | MS-ESI: *m*/*z* 384.14 observed [M+H]⁺ |

### Related documents

[1] Corrales L, Glickman LH, McWhirter SM, Kanne DB, Sivick KE, Katibah GE, Woo SR, Lemmens E, Banda T, Leong JJ, Metchette K, Dubensky TW Jr, Gajewski TF. (2015) Direct Activation of STING in the Tumor Microenvironment Leads to Potent and Systemic Tumor Regression and Immunity. Cell Rep. 11: 1018-30.
[2] Deng, L. et al. (2014) STING-Dependent Cytosolic DNA Sensing Promotes Radiation-Induced Type I Interferon-DependentAntitumor Immunity in Immunogenic Tumors, Immunity. 41: 843.
[3] Corrales L, Matson V, Flood B, Spranger S, Gajewski TF. (2017) Innate immune signaling and regulation in cancer immunotherapy. Cell Res. 27: 96-108.
[4] Corrales L, McWhirter SM, Dubensky TW Jr, Gajewski TF. (2016) The host STING pathway at the interface of cancer and immunity. J Clin Invest. 126: 2404-11.

### A compound or pharmaceutically acceptable salt thereof for use

The present disclosure also provides in an aspect a compound or pharmaceutically acceptable salt thereof for use in stimulating expression of interferon genes in a human patient. The use comprises administering to the patient an effective dose of a compound or pharmaceutically acceptable salt thereof as described herein.

In another aspect, the present disclosure provides a compound or pharmaceutically acceptable salt thereof for use in the treatment of a tumor in a patient. The use comprises administering to the patient an effective dose of a compound or pharmaceutically acceptable salt thereof.

With respect to combination therapies comprising a compound for use of the present disclosure and an immune-checkpoint targeting drug, or as combination therapies for the potentiation of ionizing radiation-based and existing chemotherapies therapeutic approaches, such as DNA-damage-based chemotherapies, the STING agonists of the present disclosure can complement and potentiate the effects of these known therapeutic approaches. This is based on recent papers indicating the critical role of STING-dependent micronuclei-mediated tumor clearance using these approaches, see for example:
[5] Mackenzie, K.F., et all, (2017), cGAS surveillance of micronuclei links genome instability to innate immunity, Nature, 548, 461.
[6] Wang, W. et al., (2016), Effector T Cells Abrogate Stroma-Mediated Chemoresistance in Ovarian Cancer, Cell, 165, 1092-1105.
[7] Charlotte E. Ariyan, et al., January 16, 2018; DOI: 10.1158/2326-6066, Robust antitumor responses result from local chemotherapy and CTLA-4 blockade, cancerimmunolres.aacrjournals.org on January 31, 2018.
[8] Chung Kil Song, et al., www.moleculartherapy.org vol. 15 no. 8 aug. 2007, Chemotherapy Enhances CD8+ T Cell-mediated Antitumor Immunity Induced by Vaccination With Vaccinia Virus.

Compounds of the present disclosure can be used in therapeutic combinations with an effective dose of an immune-checkpoint targeting drug. For example, the immune-checkpoint targeting drug can be an anti-PD-L1 antibody, anti-PD-1 antibody, anti-CTLA-4 antibody, or an anti-4-1BB antibody. See, for example:
[9] Ager, CR, et al., (2017) Cancer Immunol Res; 5(8), 676.
[10] Fu, J. et al. (2015) Sci Transl Med. 2015 April 15; 7(283): 283ra52. doi:10.1126/scitranslmed.aaa4306.
[11] Wang, H., et al. (2017) PNAS, February 14, 2017, vol. 114, no. 7, 1637-1642.

### PHARMACEUTICAL COMPOSITION

The present disclosure provides in another embodiment a pharmaceutical composition comprising a compound or pharmaceutically acceptable salt thereof as described herein in combination with a pharmaceutically acceptable carrier or excipient.

Compositions of the present disclosure can be administered orally, topically, parenterally, by inhalation or spray or rectally in dosage unit formulations. The term parenteral as used herein includes subcutaneous injections, intravenous, intramuscular, intrasternal injection or infusion techniques.

Suitable oral compositions as described herein include without limitation tablets, troches, lozenges, aqueous or oily suspensions, dispersible powders or granules, emulsion, hard or soft capsules, syrups or elixirs.

The compositions of the present disclosure that are suitable for oral use may be prepared according to any method known to the art for the manufacture of pharmaceutical compositions. For instance, liquid formulations of the compounds of the present disclosure contain one or more agents selected from the group consisting of sweetening agents, flavoring agents, coloring agents and preserving agents in order to provide pharmaceutically palatable preparations of the compound or a pharmaceutically acceptable salt thereof.

For tablet compositions, the compound or a pharmaceutically acceptable salt thereof in admixture with non-toxic pharmaceutically acceptable excipients is used for the manufacture of tablets. Examples of such excipients include without limitation inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known coating techniques to delay disintegration and absorption in the gastrointestinal tract and thereby to provide a sustained therapeutic action over a desired time period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

For aqueous suspensions, the compound or a pharmaceutically acceptable salt thereof is admixed with excipients suitable for maintaining a stable suspension. Examples of such excipients include without limitation are sodium carboxymethylcellulose, methylcellulose, hydropropylmethylcellulose, sodium alginate, polyvinylpyrrolidone, gum tragacanth and gum acacia.

Oral suspensions can also contain dispersing or wetting agents, such as naturally-occurring phosphatide, for example, lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the compound or a pharmaceutically acceptable salt thereof in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol.

Sweetening agents such as those set forth above, and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the compound or a pharmaceutically acceptable salt thereof in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Pharmaceutical compositions of the present disclosure may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monoleate, and condensation reaction products of the said partial esters with ethylene oxide, for example polyoxyethylene sorbitan monoleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative, and flavoring and coloring agents. The pharmaceutical compositions may be in the form of a sterile injectable, an aqueous suspension or an oleaginous suspension. This suspension may be formulated according to the known art using those suitable dispersing or wetting agents and suspending agents which have been mentioned above. The sterile injectable preparation may also be sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The compound the compound or a pharmaceutically acceptable salt thereof may also be administered in the form of suppositories for rectal administration. These compositions can be prepared by mixing the compound with a suitable non-irritating excipient which is solid at ordinary temperatures but liquid at the rectal temperature and will therefore melt in the rectum to release the compound. Exemplary excipients include cocoa butter and polyethylene glycols.

Compositions for parenteral administrations are administered in a sterile medium. Depending on the vehicle used and concentration the concentration of the compound or a pharmaceutically acceptable salt thereof in the formulation, the parenteral formulation can either be a suspension or a solution containing dissolved compound. Adjuvants such as local anesthetics, preservatives and buffering agents can also be added to parenteral compositions.

### EXAMPLES

Tissue culture. Wild-type (cat. no. thpl-isg) and STING KO (cat. no. thpd-kostg) THP-1-Lucia ISG cells were purchased from Invivogen and maintained in growth media consisting of RPMI 1640, 2mM L-glutamine, 25 mM HEPES, 10% heat-inactivated fetal bovine serum (FBS), 1,000 units/ml penicillin, 1,000 µg/ml streptomycin, 0.25 µg/ml Amphotericin B, and 100 µg/ml zeocin unless otherwise stated.

Type 1 interferon stimuli. Poly(dA:dT) and 2'3'-cGAMP were purchased from invivogen and resuspended according to manufacturer's instructions.

**ISRE-luciferase assay.** THP-1 Lucia ISG cells were resuspended in low-serum growth media (2% FBS) at a density of 5 x 10⁵ cells/ml and treated with test article or vehicle (DMSO). 50 µL of cells were seeded into each well of a 384-well white greiner plates and incubated for 24 hours. To evaluate expression of the luciferase reporter, 30 µl of Quanti-luc (Invivogen) detection reagent was added to each well and luminescence was read using an Envision plate reader (Perkin Elmer) set with an integration time of 0.1 seconds.

**Viability assay.** Cells were resuspended in low-serum growth media at a density of 5 x 10⁵ cells/ml and treated with test article or vehicle (DMSO). 50 µL of cells were seeded into each well of a 384-well white greiner plates and incubated for 24 hours. To evaluate expression of the luciferase reporter, 30 µl of CellTiter-Glo (Promega) detection reagent was added to each well and luminescence was detected using an Envision Plate Reader set with an integration time of 0.1 seconds.

**Western Blot.** Cells were solubilized in 1X protein lysis buffer (25 mM HEPES, pH 7.4, 300 mM NaCl, 1.5 mM MgCl₂, 1 mM EGTA, 1% P-40, 1% sodium deoxycholate, 2.5 mM sodium pyrophosphate, 1 mM glycerophosphate) with freshly added protease and phosphatase inhibitors (Cell Signaling). Western blotting was performed using Bolt^{™} 4-12% Bis-Tris gels and Bolt^{™} mini transfer system following the manufacturer's instructions (ThermoFisher Scientific). STING and *γ*-tubulin antibodies were purchased from Cell Signaling diluted in 5% BSA, 1X TBS-T buffer (Table 3). Anti-rabbit HRP antibody was diluted in 5% non-fat dried milk, 1X TBS-T buffer and luminescence signal was imaged using a ChemiDoc Imager (BioRad).

**Semi-quantitative real-time PCR (qPCR).** THP-1 cells were resuspended in low-serum growth media at a density of 5 x 10⁵ cells/ml and treated with test article or vehicle (DMSO). 2.5 mL of cells were seeded into each well of a 6-well plate and incubated for 24 hours. RNA was isolated using an RNeasy Plus Mini Kit (Qiagen) and 1 µg of purified RNA was reverse-transcribed into cDNA (VILO, cat. no. 11755050, ThermoFisher Scientific). Gene expression was assessed using Taqman primers and probes listed in Table 4 with the Taqman Universal Mix II (cat. no. 4440038, ThermoFisher) following manufacturer's instructions. Gene expression was normalized using the double delta Ct method and was reported as fold change in expression.

**STING Thermal Shift Assay (TSA).** The c-terminal domains (CTD) of human and mouse STING were expressed and purified as detailed previously ( Ouyang, S., Song, X., Wang, Y., Ru, H., Shaw, N., Jiang, Y., Niu, F., Zhu, Y., Qiu, W., Parvatiyar, K., et al. (2012). Structural analysis of the STING adaptor protein reveals a hydrophobic dimer interface and mode of cyclic di-GMP binding. Immunity 36, 1073-1086.). Test article or vehicle controls were added to diluted STING protein (0.22 mg/ml) in 1X Protein Thermal Shift Buffer provided in the Protein Thermal Shift Dye Kit (cat # 4461146, ThermoFisher Scientific). Thermal Shift dye was added and mixed prior to performing a melt curve following parameters outlined for the Dye kit. Melt temperatures (Tm) were calculated using the Derivative method using Protein Thermal Shift Software v1.3 (cat # 4466038, ThermoFisher Scientific).

**WT STING binding assay (Cisbio, Catalog # 64BDSTGPEH).** An assay format was optimized to demonstrate binding of recombinant 6x His-tagged human STING protein labeled with Terbium Cryptate by the natural ligand, 2'3'cGAMP labeled with d2 (the acceptor). Upon proximity of the two dyes, the excitation of the donor by the flash lamp on the PHERAstar FSX plate reader triggers a Fluorescence Resonance Energy Transfer (FRET) towards the acceptor, which in turn fluoresces at 665 nm. To assess the ability of the synthetic small molecule STING ligands to bind to human STING, a competitive assay format was applied. A 10-point titration of each of the synthetic ligands in 5uL were transferred into a 384 well plate, followed by 20uL of assay buffer containing the 6x His-tagged human STING protein and labeled 2'3'cGAMP ligand and incubated for three hours at room temperature. The raw values obtained from the PHERAstar were used to calculate the reported IC₅₀ values (the signal is inversely proportional to the binding of the synthetic ligand) through curve fitting in Genedata. The percent inhibition was calculated based upon the maximal amount of binding by synthetic compound versus the maximum binding of unlabeled 2'3' cGAMP which was used as a control in each assay.

**Table 2: Cell Signaling Antibodies**

| **Protein target** | **Cat. No.** | **Dilution** |
|---|---|---|
| STING | 13647 | 1:1000 |
| *γ*-tubulin | 5886 | 1:3000 |
| Rabbit IgG | 7074 | 1:3000 |

**Table 3: ThermoFisher Scientific Taqman Primers/Probe**

| **Gene Symbol** | **Species** | **Cat. No.** | **Dye** |
|---|---|---|---|
| IFNB1 | human | Hs01077958_s1 | FAM |
| CXCL10 | human | Hs00171042_m1 | FAM |
| IFIT3 | human | Hs01922752_s1 | FAM |
| B2M | human | Hs00187842_m1 | VIC |

Compounds useful for carrying out a method of the present disclosure can be prepared according to the following procedures in conjunction with ordinary knowledge and skill in organic synthesis, substituting appropriate reagents as apparent to the practitioner.

### Experimental Procedures

**Abbreviations.** The following abbreviations are used: tetrahydrofuran (THF), dichloromethane (DCM), *N,N*-dimethylformamide (DMF), dimethylacetamide (DMA), dimethylsulfoxide (DMSO), trifluoroacetic acid (TFA), triethylamine (TEA), diisopropylethylamine (DIPEA), (1-Cyano-2-ethoxy-2-oxoethylidenaminooxy)dimethylamino-morpholino-carbenium hexafluorophosphate (COMU), 1-[bis(dimethylamino)methylene]-1H-1,2,3-triazolo[4,5-b]pyridinium 3-oxid hexafluorophosphate, N-[(dimethylamino)-1H-1,2,3-triazolo-[4,5-b]pyridin-1-ylmethylene]-N-methylmethanaminium hexafluorophosphate N-oxide (HATU).

**General Examples for the Preparation of Compounds of the Present disclosure.** The starting materials and intermediates for the compounds of this present disclosure may be prepared by the application or adaptation of the methods described below, their obvious chemical equivalents, or, for example, as described in literature such as The Science of Synthesis, Volumes 1-8. Editors E. M. Carreira et al. Thieme publishers (2001-2008). Details of reagent and reaction options are also available by structure and reaction searches using commercial computer search engines such as Scifinder (www.cas.org) or Reaxys (www.reaxys.com).

### PART I: PREPARATION OF INTERMEDIATES

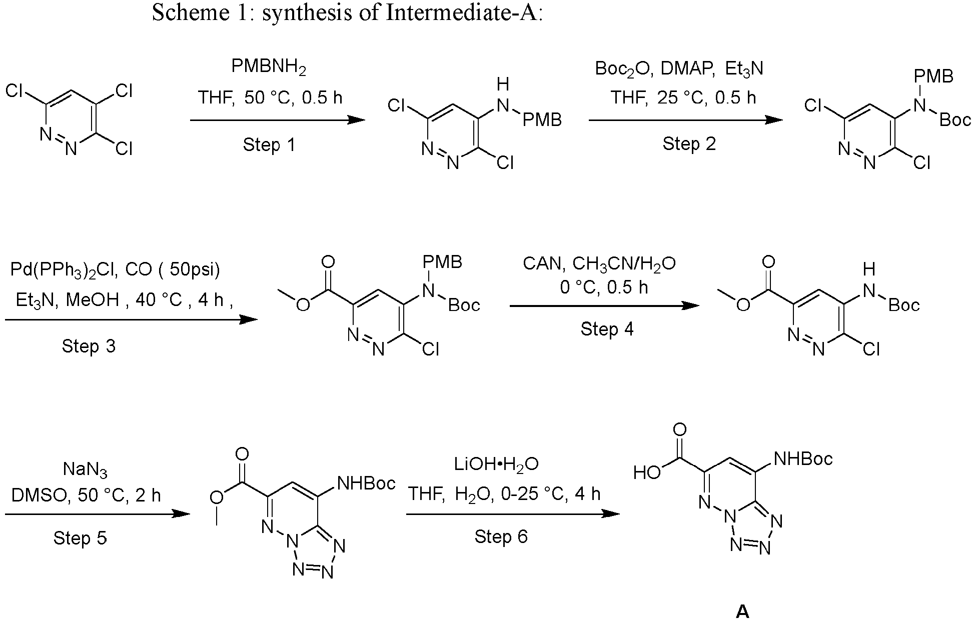

**Step 1: Synthesis of 3,6-dichloro-N-(4-methoxybenzyl)pyridazin-4-amine:** To a solution of 3,4,6-trichloropyridazine (45.0 g, 245 mmol) in THF (450 mL) was added (4-methoxyphenyl)methanamine (101 g, 736 mmol), then the mixture was stirred at 50 °C for 0.5 hour. The mixture was diluted with water (1000 mL) and extracted with EtOAc (1000 mL × 3). The combined organic layers were washed with brine (300 mL × 2), dried over anhydrous Na₂SO₄, filtered and concentrated under vacuum. The residue was purified by column chromatography to give 3,6-dichloro-N-(4-methoxybenzyl)pyridazin-4-amine (70.0 g, 97% purity, 97% yield) as a white solid. LCMS (ESI): *m*/*z* 283.9 [M+H]⁺.

**Step 2: Synthesis of tert-butyl (3,6-dichloropyridazin-4-yl)(4-methoxybenzyl)carbamate:** To a solution of 3,6-dichloro-N-(4-methoxybenzyl)pyridazin-4-amine (70.0 g, 246 mmol) in THF (700 mL) was added Et₃N (49.9 g, 492 mmol), DMAP (15.0 g, 123 mmol) and Boc₂O (79.2 mL, 345 mmol). After stirred at 25 °C for 0.5 hour, the mixture was diluted with water (1000 mL), and then extracted with EtOAc (1000 mL × 3). The combined layers were washed with brine (500 mL × 2), dried over anhydrous Na₂SO₄, filtered and concentrated under reduced pressure. The residue was purified by column chromatography to give tert-butyl (3,6-dichloropyridazin-4-yl)(4-methoxybenzyl)carbamate (77 g, 67% yield) as a white solid. LCMS (ESI): *m*/*z* 384.1 [M+H]⁺.

**Step 3: Synthesis of methyl 5-((tert-butoxycarbonyl)(4-methoxybenzyl)amino)-6-chloropyridazine-3-carboxylate:** To a solution of tert-butyl (3,6-dichloropyridazin-4-yl)(4-methoxybenzyl)carbamate (36.5 g, 95.0 mmol) in methanol (500 mL) was added Et₃N (39.7 mL, 285 mmol) and Pd(PPh₃)₂Cl (6.95 g, 9.50 mmol). The mixture was stirred at 40 °C for 4 hours under carbon monoxide (50 psi). The mixture was diluted with water (500 mL) and extracted with EtOAc (500 mL × 3). The combined organic layers were washed with brine (300 mL × 2), dried over anhydrous Na₂SO₄, filtered and concentrated under reduced pressure. The residue was purified by column chromatography to give methyl 5-((tert-butoxycarbonyl)(4-methoxybenzyl) amino)-6-chloropyridazine-3-carboxylate (22.9 g, 47% yield) as a white solid. LCMS (ESI): *m*/*z* 408.1 [M+H]⁺

**Step 4: Synthesis of methyl 5-((tert-butoxycarbonyl)amino)-6-chloropyridazine-3-carboxylate:** To a solution of 5-((tert-butoxycarbonyl)(4-methoxybenzyl)amino)-6-chloropyridazine-3-carboxylate (15.0 g, 36.7 mmol) in acetonitrile (75.0 mL) and water (75.0 mL) was added CAN (40.3 g, 73.6 mmol) at 0 °C, then the mixture was stirred for 0.5 hour. The mixture was diluted with water (200 mL) and extracted with EtOAc (100 mL × 3). The combined organic layers were dried over anhydrous Na₂SO₄, filtered and concentrated under reduced pressure. The residue was purified by column chromatography to give methyl 5-((tert-butoxycarbonyl)amino)-6-chloropyridazine-3-carboxylate (7.20 g, 65% yield) as a white solid. LCMS (ESI): *m*/*z* 288.0 [M+H]⁺.

**Step 5: Synthesis of methyl 8-((tert-butoxycarbonyl)amino)tetrazolo[1,5-b]pyridazine-6-carboxylate:** To a solution of 5-((tert-butoxycarbonyl)amino)-6-chloropyridazine-3-carboxylate (4.30 g, 13.6 mmol) in DMSO (43.0 mL) was added sodium azide (2.65 g, 40.8 mmol). After stirred at 50 °C for 2 hours, the mixture was diluted with water (200 mL) and extracted with EtOAc (200mL × 3). The combined organic layers were washed with water (200 mL × 3) and brine (200 mL × 2), dried over anhydrous Na₂SO₄, filtered and concentrated under reduced pressure. The residue was purified by column chromatography to give methyl 8-((tert-butoxycarbonyl)amino)tetrazolo[1,5-b]pyridazine-6-carboxylate (2.80 g, 66% yield) as a white solid. ¹H NMR (400 MHz, DMSO-*d₆*) δ 11.44 (s, 1H), 8.51 (s, 1H), 4.00 (s, 3H), 1.53 (s, 9H). LCMS (ESI): *m*/*z* 294.7 [M+H]⁺.

**Step 6: Synthesis of 8-((tert-butoxycarbonyl)amino)tetrazolo[1,5-b]pyridazine-6-carboxylic acid (A):** To a solution of methyl 8-((tert-butoxycarbonyl)amino)tetrazolo[1,5-b]pyridazine-6-carboxylate (250 mg, 0.85 mmol) in THF (5 mL) was added a solution of lithium hydroxide monohydrate (143 mg, 3.4 mmol) in water (5 mL) at 0 °C. The mixture was stirred at 25 °C for 4 hours. After completion of the reaction, 3M HCl was added to neutralize the reaction and the precipitate was filtered and wash with cold water (1 mL x2) to give **A** (191 mg, 80% yield) as a white solid. ¹H NMR (400 MHz, DMSO) δ 8.60 (s, 1H), 8.32 (s, 1H), 8.22 (s, 1H), 1.58 (s, 9H).

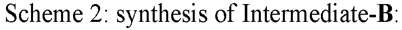

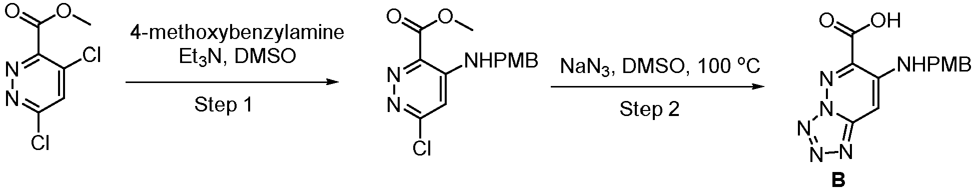

**Step 1: synthesis of methyl 6-chloro-4-((4-methoxybenzyl)amino)pyridazine-3-carboxylate:** To a solution of compound methyl 4,6-dichloropyridazine-3-carboxylate (207 mg, 1 mmol) and Et₃N (279 µL, 2 mmol) in DMSO (2 mL) was added 4-methoxybenzylamine (144 µL, 1.1 mmol) and the reaction mixture stirred at room temperature overnight. After completion of the reaction, water (10 mL) was added and a white precipitation was formed. The mixture was filtered and washed with water to give methyl 6-chloro-4-((4-methoxybenzyl)amino)pyridazine-3-carboxylate (257 mg, 84% yield) as a white solid. LCMS (ESI): *m*/*z* 308.0 [M+H]⁺.

**Step 2: synthesis of 7-((4-methoxybenzyl)amino)tetrazolo[1,5-b]pyridazine-6-carboxylic acid:** To a solution of methyl 6-chloro-4-((4-methoxybenzyl)amino)pyridazine-3-carboxylate (257 mg, 0.84 mmol) in DMSO (3 mL) was added sodium azide (109 mg, 1.68 mmol) and stirred at 100 °C for 16 hours. After completion of the reaction, water (5 mL) was added and a yellow precipitate was formed. The mixture was kept at 0 °C for 10 minutes, filtered and washed with water (2 mL x 2) to give a yellow solid. The solid was suspended in water (10 mL) and neutralized by adding HCl (3M). The suspension was filtered to afford **B** (183 mg, 73% yield) as a yellow solid. ¹H NMR (400 MHz, DMSO-*d₆*): δ 8.43 (s, 1H), 7.42 - 7.32 (m, 2H), 7.22 (s, 1H), 6.96 - 6.88 (m, 2H), 4.48 (d, *J* = 5.0 Hz, 2H), 3.73 (s, 3H). LCMS (ESI): *m*/*z* 300.8 [M+H]⁺.

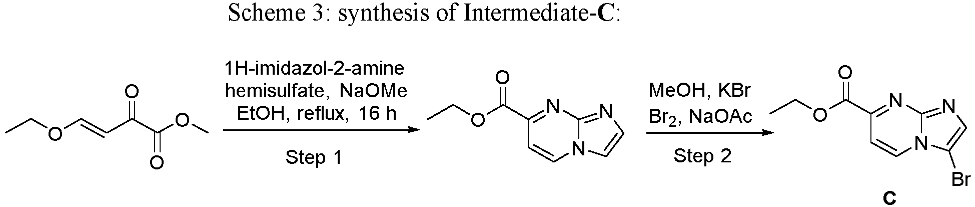

**Step 1: Synthesis of ethyl imidazo[1,2-a]pyrimidine-7-carboxylate:** To a suspension of 1H-imidazol-2-amine hemisulfate (8.5 g, 64.33 mmol) in EtOH (100 mL) was added sodium methoxide (4.4 g, 82.2 mmol) and the resulting mixture was stirred at 90 °C for 30 min. It was then cooled to room temperature and a solution of methyl (E)-4-ethoxy-2-oxobut-3-enoate (10 g, 63.29 mmol) in EtOH (40 mL) was added. The reaction mixture was slowly heated to reflux and stirred for 16 h. After completion of the reaction, it was cooled to room temperature and volatiles were removed under reduced pressure. The residue was purified by column chromatography (0-4% MeOH in DCM) to afford ethyl imidazo[1,2-a]pyrimidine-7-carboxylate (5.2 g, 43% yield) as an off-white solid. ¹H NMR (400 MHz, CDCl₃): *δ* 8.64 - 8.61 (m, 1H), 8.06 - 8.04 (m, 1H), 7.74 - 7.67 (m, 2H), 4.51 (q, *J* = 7.2 Hz, 2H), 1.47 (t, *J* = 7.2 Hz, 3H). LCMS(ESI): *m*/*z* 192.1 [M+H]⁺.

**Step 2: Synthesis of ethyl 3-bromoimidazo[1,2-a]pyrimidine-7-carboxylate (C):** To a suspension of ethyl imidazo[1,2-a]pyrimidine-7-carboxylate (5.2 g, 27.2 mmol) in MeOH (52 mL) was added KBr (3.24 g, 27.2 mmol) and sodium acetate (3.43 g, 40.8 mmol) at 0 °C, under nitrogen atmosphere. Bromine (4.78 g, 29.9 mmol) was added to it over a period of 10-15 min. After completion, the reaction was quenched with 1M aqueous Na₂SO₃ solution (10 mL). Volatiles were then removed under reduced pressure and saturated NaHCO₃ solution (30 mL) was added to the residue. The solid obtained was collected by filtration and purified by column chromatography (0-2% MeOH in DCM) to afford **C** (5.3 g, 72% yield) as an off-white solid. ¹H NMR (400 MHz, DMSO*-d₆*): *δ* 8.97 (m, 1H), 8.18 (s, 1H), 7.71 (d, *J* = 7.2 Hz, 1H), 4.42 (q, *J* = 7.2 Hz, 2H), 3.95 (s, 3H), 1.38 (t, *J* = 7.2 Hz, 3H). LCMS (ESI): *m*/*z* 270.2 [M+H]⁺.

### PART II: PREPARATION OF EXAMPLE COMPOUNDS

All compounds of the present disclosure were prepared using the procedures exemplified below.

### Example 1

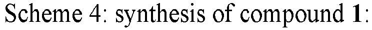

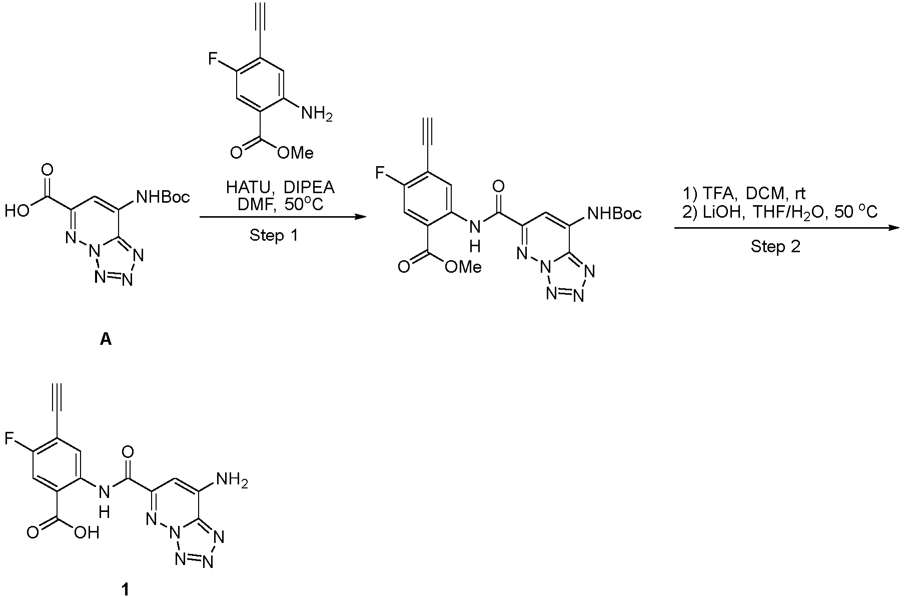

**Step 1:** Synthesis of methyl 2-(8-((tert-butoxycarbonyl)amino)tetrazolo[1,5-b]pyridazine-6-carboxamido)-4-ethynyl-5-fluorobenzoate: To a solution of A (27.8 mg, 0.1 mmol) was added HATU (48 mg, 0.15 mmol), methyl 2-amino-4-ethynyl-5-fluorobenzoate (19.4 mg, 0.11 mmol) and DIPEA (35 µL, 0.2 mmol) and the reaction mixture was stirred at 50 °C overnight. The crude mixture was purified by preparative HPLC to afford methyl 2-(8-((tert-butoxycarbonyl)amino)tetrazolo[1,5-b]pyridazine-6-carboxamido)-4-ethynyl-5-fluorobenzoate as a white solid (28 mg, 51% yield). ¹H NMR (400 MHz, DMSO-*d₆*) δ 12.47 (s, 1H), 11.54 (s, 1H), 8.80 (d, *J* = 6.1 Hz, 1H), 8.67 (s, 1H), 7.93 (d, *J* = 9.2 Hz, 1H), 4.90 (s, 1H), 3.97 (s, 3H), 1.55 (s, 9H). LCMS (ESI): m/z 456.6 [M+H]⁺.

**Step 2: Synthesis of lithium 2-(8-aminotetrazolo[1,5-b]pyridazine-6-carboxamido)-4-ethynyl-5-fluorobenzoate (1)** : To a solution of methyl 2-(8-((tert-butoxycarbonyl)amino) tetrazolo[1,5-b]pyridazine-6-carboxamido)-4-ethynyl-5-fluorobenzoate (28 mg, 0.061 mmol) in CH₂Cl₂ (0.8 mL) was added TFA (0.4 mL) and the reaction mixture was stirred at room temperature for 1h. After completion of the reaction, the mixture was concentrated under reduced pressure. Then the crude mixture was dissolved in THF (2 mL) and lithium hydroxide aqueous solution (310 µL, 0.31 mmol, 1M) was added and stirred at 50 °C for 6 hours. After completion of the reaction, THF was removed under reduced pressure and the residue was suspended with water (1 mL). It was filtered and washed with cold water (0.5 mL x 2) and acetonitrile (0.5 mL), affording compound **1** (lithium salt, 14.5 mg, 68% yield) as a white solid. ¹H NMR (400 MHz, DMSO-*d₆*) δ 15.71 (s, 1H), 8.82 (dd, *J* = 7.0, 1.8 Hz, 1H), 8.33 (s, 2H), 7.76 (dd, *J* = 10.4, 2.0 Hz, 1H), 7.15 (dd, *J* = 2.2, 1.0 Hz, 1H), 4.54 (d, *J* = 2.0 Hz, 1H). LCMS (ESI): m/z 342.46 [M+H]⁺.

The following compounds were synthesized in procedures analogous to those described above for compound 1: 5, 6, 7, 8, 9, 10, 11, 14, 15, 16, 17, 18, 19, 21, 22, 24, 25, 26, 28, 29, 30, 31, 32, 33, 34, 35, 36, 38, 39, 40, 41, 42, 43, 45, 47, 50, 55, 56, 58, 64, 65, 66, 67, 68, 69, 70, 71, 72, 73, 74, 79, 80, 77, 78, 81, 82, 83, 84, 85 and 86 *et al.*

### Example 2

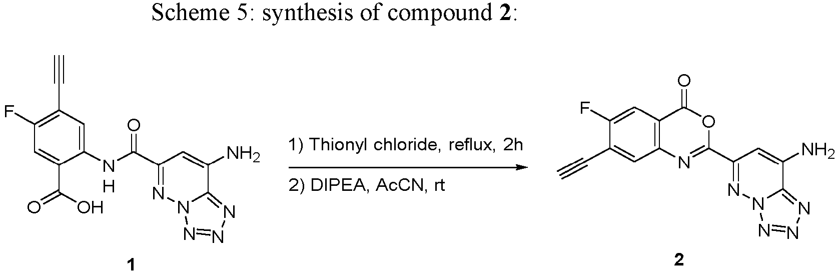

**Synthesis of 2-(8-aminotetrazolo[1,5-b]pyridazin-6-yl)-7-ethynyl-6-fluoro-4H-benzo[d][1,3]oxazin-4-one (2):** A suspension of compound **1** (20 mg, 0.06 mmol) in 0.6 mL of thionyl chloride and the mixture was heated under reflux for 2h. Then, the excess thionyl was removed under vacuum. The solid was dissolved in 1 mL of anhydrous acetonitrile and a solution of DIPEA (20 µL, 0.12 mmol) in 1 mL of anhydrous acetonitrile was added at room temperature. After stirring for 30 minutes, the obtained precipitate was isolated and washed with acetonitrile to give the product (15 mg, 77 % yield). ¹H NMR (400 MHz, DMSO-*d*₆) δ 8.49 (s, 2H), 8.11 (d, *J* = 8.4 Hz, 1H), 8.03 (d, *J* = 6.3 Hz, 1H), 7.35 (s, 1H), 5.04 (s, 1H). MS-ESI: *m*/*z* 324.49 observed (M+H)⁺

Compounds 44, 48 and 49 were prepared by using a procedure analogous to that described for compound **2.**

### Example 3

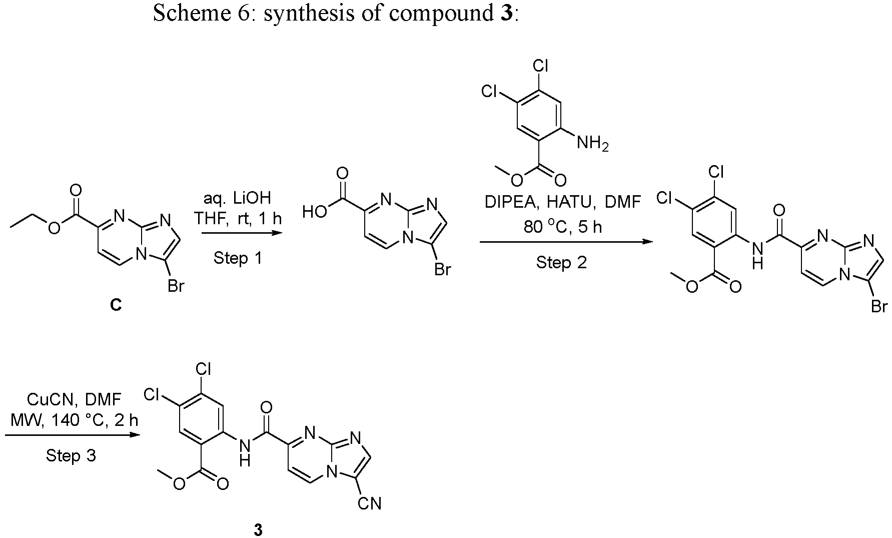

**Step 1: Synthesis of 3-bromoimidazo[1,2-a]pyrimidine-7-carboxylic acid:** An aqueous solution of lithium hydroxide (0.327 g, 7.8 mmol, 2 mL water) was added to a solution of **C** (1.4 g, 5.2 mmol) in THF (8 mL) at room temperature. The reaction was stirred at the same temperature for 1 h. After completion of the reaction, solvent was removed under reduced pressure and the aqueous layers were acidified using 2N HCl at 0 °C. The solid obtained was collected by filtration, washed with cold water (3 mL x 2) and dried to afford 3-bromoimidazo[1,2-a]pyrimidine-7-carboxylic acid (1.0 g, 79% yield) as an off-white solid. ¹H NMR (400 MHz, DMSO*-d₆*): *δ* 9.07 (d, *J* = 6.8 Hz, 1H), 8.29 (s, 1H), 7.65 (d, *J* = 6.8 Hz, 1H). LCMS (ESI): *m*/*z* 243.06 [M+H]⁺.

**Step 2: Synthesis of methyl 2-(3-bromoimidazo[1,2-a]pyrimidine-7-carboxamido)-4,5-dichlorobenzoate:** To a solution of 3-bromoimidazo[1,2-a]pyrimidine-7-carboxylic acid (1.4 g, 5.8 mmol) in DMF (14 mL) was added DIPEA (3.8 g, 29.2 mmol) and HATU (3.3 g, 8.7 mmol) at 0 °C under nitrogen atmosphere. It was stirred for 10 min before addition of methyl 2-amino-4,5-dichlorobenzoate (1.9 g, 8.7 mmol). The reaction mixture was stirred at 80 °C for 5 h. After completion of the reaction, it was cooled to room temperature and saturated NaHCO₃ solution (30 mL) was added. The solid obtained was collected by filtration and purified by column chromatography to afford methyl 2-(3-bromoimidazo[1,2-a]pyrimidine-7-carboxamido)-4,5-dichlorobenzoate (1.0 g, 39% yield) as an off-white solid. ¹H NMR (400 MHz, DMSO*-d₆*): *δ* 13.09 (s, 1H), 9.17 (s, 1H), 8.59 (d, *J* = 6.8, 1H), 8.22 (s, 1H), 7.92 (d, *J* = 6.8, 1H), 7.76 (s, 1H), 3.89 (s, 3H). LCMS (ESI): *m*/*z* 441.0 [M-H]⁻.

**Step 3: Synthesis of methyl 4,5-dichloro-2-(3-cyanoimidazo[1,2-a]pyrimidine-7-carboxamido)benzoate 3:** To a solution of methyl 2-(3-bromoimidazo[1,2-a]pyrimidine-7-carboxamido)-4,5-dichlorobenzoate (0.220 g, 0.5 mmol) in DMF (4 mL) was added CuCN (0.157 g, 1.75 mmol) and it was irradiated in a microwave reactor at 140 °C for 2 h (1 h + 1 h intervals). The reaction mixture was diluted with EtOAc (25 mL) was washed with saturated NaHCO₃ (10 mL) solution, water (10 mL x 2), brine (10 mL), dried over anhydrous Na₂SO₄, filtered and concentrated under reduced pressure. The residue was purified by column chromatography (0-30% EtOAc in PE) to afford 0.11 g of crude product, which was further purified by preparative HPLC to afford compound **3** (11 mg, 6% yield) as an off-white solid. ¹H NMR (400 MHz, DMSO*-d₆*): *δ* 12.79 (s, 1H), 9.28 (d, *J* = 6.8 Hz, 1H), 9.04 (s, 1H), 8.95 (s, 1H), 8.21 (s, 1H), 7.89 (d, *J* = 6.8 Hz, 1H), 4.00 (s, 3H). LCMS (ESI): *m*/*z* 388.1 [M-H]⁻.

Compounds 13, 46, 52 and 75 were prepared by using a procedure analogous to that described for Compound **3.**

### Example 4:

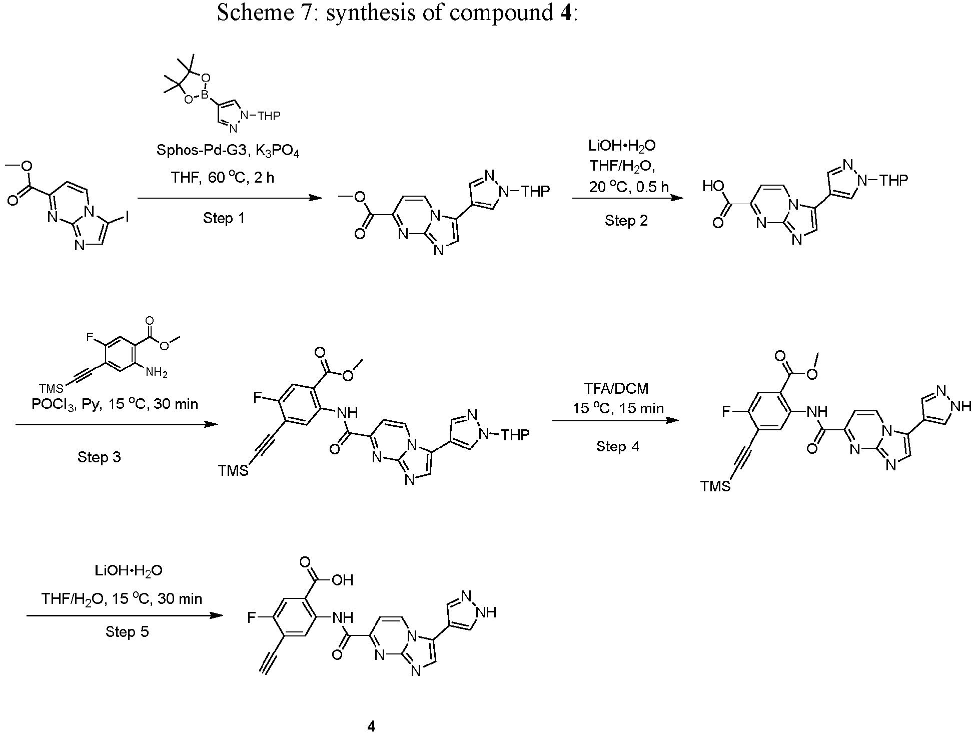

**Step 1: Synthesis of methyl 3-(1-(tetrahydro-2H-pyran-2-yl)-1H-pyrazol-4-yl)imidazo[1,2-a]pyrimidine-7-carboxylate:** A mixture of 1-(tetrahydro-2H-pyran-2-yl)-4-(4,4,5,5-tetramethyl-1,3,2-dioxaborolan-2-yl)-1H-pyrazole (413 mg, 1.48 mmol, 1.5 *eq*), methyl 3-iodoimidazo[1,2-a]pyrimidine-7-carboxylate (0.3 g, 989 umol, 1 *eq*), K₃PO₄ (315 mg, 1.48 mmol, 1.5 *eq)* and SPhos Pd G3 (154 mg, 197 umol, 0.2 *eq*) in THF (3 mL) was degassed and purged with N₂ for 3 times. The mixture was stirred at 60 °C for 2 hours under N₂ atmosphere. The crude reaction mixture was purified by reversed-phase Combi-flash (0.1% FA condition) to give compound methyl 3-(1-(tetrahydro-2H-pyran-2-yl)-1H-pyrazol-4-yl)imidazo[1,2-a]pyrimidine-7-carboxylate (120 mg, 366 umol, 37 % yield) as a yellow solid. LCMS (ESI): *m*/*z* 328.1 [M+H]⁺.

**Step 2: Synthesis of 3-(1-(tetrahydro-2H-pyran-2-yl)-1H-pyrazol-4-yl)imidazo[1,2-a]pyrimidine-7-carboxylic acid:** To a mixture of methyl 3-(1-(tetrahydro-2H-pyran-2-yl)-1H-pyrazol-4-yl)imidazo[1,2-a]pyrimidine-7-carboxylate (100 mg, 305 umol, 1 *eq*) in THF (0.5 mL) and H₂O (0.5 mL) was added LiOH•H₂O (12.8 mg, 305 umol, 1 *eq*) at 20°C. The mixture was stirred at 20 °C for 0.5 hour, and then concentrated to give compound 3-(1-(tetrahydro-2H-pyran-2-yl)-1H-pyrazol-4-yl)imidazo[1,2-a]pyrimidine-7-carboxylic acid (85 mg, crude) as a yellow solid. LCMS (ESI): *m*/*z* 314.1 [M+H]⁺.

**Step 3: Synthesis of methyl 5-fluoro-2-(3-(1-(tetrahydro-2H-pyran-2-yl)-1H-pyrazol-4-yl)imidazo[1,2-a]pyrimidine-7-carboxamido)-4-((trimethylsilyl)ethynyl)benzoate:** To a mixture of 3-(1-(tetrahydro-2H-pyran-2-yl)-1H-pyrazol-4-yl)imidazo[1,2-a]pyrimidine-7-carboxylic acid (50 mg, 159 umol, 1 *eq*) and methyl 2-amino-5-fluoro-4-((trimethylsilyl)ethynyl)benzoate (42.3 mg, 159 umol, 1 *eq*) in Py (0.5 mL) was added POCl₃ (73.4 mg, 478 umol, 44.4 uL, 3 *eq*) at 15 °C. The mixture was stirred at 15 °C for 30 min, and then diluted with H₂O (20 mL) and extracted with EA (10 mL × 3). The combined organic layers were dried over Na₂SO₄, filtered and concentrated under reduced pressure to give a residue. The residue was purified by prep-HPLC to give methyl 5-fluoro-2-(3-(1-(tetrahydro-2H-pyran-2-yl)-1H-pyrazol-4-yl)imidazo[1,2-a]pyrimidine-7-carboxamido)-4-((trimethylsilyl)ethynyl)benzoate (75 mg, 133 umol, two steps 74% yield) was obtained as a yellow solid. ¹H NMR (400 MHz, DMSO-d₆) δ = 12.63 (s, 1H), 9.21 (d, *J* = 7.2 Hz, 1H), 8.91 (d, *J* = 6.8 Hz, 1H), 8.61 (s, 1H), 8.28 (s, 1H), 8.13 (s, 1H), 7.88 (d, *J* = 10.0 Hz, 1H), 7.75 (d, *J* = 7.2 Hz, 1H), 5.51 (dd, *J* = 2.0, 10.0 Hz, 1H), 4.05-3.95 (m, 4H), 3.70 - 3.65 (m, 1H), 2.24 - 2.12 (m, 1H), 2.03 - 1.93 (m, 2H), 1.78 - 1.66 (m, 1H), 1.62 - 1.53 (m, 2H), 0.31 - 0.27 (m, 9H).

**Step 4: Synthesis of methyl 2-(3-(1H-pyrazol-4-yl)imidazo[1,2-a]pyrimidine-7-carboxamido)-5-fluoro-4-((trimethylsilyl)ethynyl)benzoate:** To a mixture of methyl 5-fluoro-2-(3-(1-(tetrahydro-2H-pyran-2-yl)-1H-pyrazol-4-yl)imidazo[1,2-a]pyrimidine-7-carboxamido)-4-((trimethylsilyl)ethynyl)benzoate (15 mg, 26.7 umol, 1 *eq*) in DCM (1 mL) was added TFA (462 mg, 4.05 mmol, 0.3 mL, 151 *eq*) in one portion at 15 °C. The mixture was stirred at 15 °C for 15 min., and then concentrated to give a residue. The residue was purified by prep-HPLC (column: Shim-pack C18 150*25* 10um;mobile phase: [water(0.225%FA)-ACN]; B%: 62%-82%,10min) to give compound methyl 2-(3-(1H-pyrazol-4-yl)imidazo[1,2-a]pyrimidine-7-carboxamido)-5-fluoro-4-((trimethylsilyl)ethynyl)benzoate (8 mg, 16.7 umol, 62% yield) as a yellow solid. LCMS (ESI): *m*/*z* 477.0 [M+H]⁺.

**Step 5: Synthesis of 2-(3-(1H-pyrazol-4-yl)imidazo[1,2-a]pyrimidine-7-carboxamido)-4-ethynyl-5-fluorobenzoic acid (4):** To a mixture of methyl 2-(3-(1H-pyrazol-4-yl)imidazo[1,2-a]pyrimidine-7-carboxamido)-5-fluoro-4-((trimethylsilyl)ethynyl)benzoate (15 mg, 31.4 umol, 1 *eq*) in THF (0.1 mL) and H₂O (0.1 mL) was added LiOH•H₂O (1.45 mg, 34.62 umol, 1.1 *eq*) in one portion at 15 °C. The mixture was stirred at 15 °C for 30 min. The mixture was quenched with 1N HCl to pH = 6, and then concentrated under vacuum to remove. The precipitate was filtered and the filter cake was dried under vacuum to give compound 2-(3-(1H-pyrazol-4-yl)imidazo[1,2-a]pyrimidine-7-carboxamido)-4-ethynyl-5-fluorobenzoic acid (4.28 mg, 10.9 umol, 34% yield, 100% purity) as a yellow solid. ¹H NMR (400 MHz, DMSO-*d*₆) δ = 12.95 (s, 1H), 9.21-9.19 (d, *J* = 7.2 Hz, 1H), 9.01-8.99 (d, *J* = 6.4 Hz, 1H), 8.36-8.33 (m, 3H), 7.90-7.87 (d, *J* = 10.0 Hz, 1H), 7.77-7.75 (d, *J* = 7.2 Hz, 1H), 4.83 (s, 1H). LCMS (ESI): *m*/*z* 391.3 [M+H]⁺.

Compounds 51, 59 and 61 were prepared by using a procedure analogous to that described for compound **4.**

## Claims

1. A compound of formula (I): 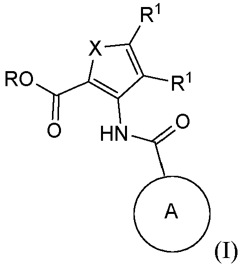 wherein
X is S, -N=C(R¹)-, or -C(R¹)=C(R¹)-;
each R¹ is independently H, F, Cl, C₁-C₆-alkyl, ethenyl or ethynyl either of which can be substituted, cyano, alkoxyl, haloalkyl, or C₃-C₆-cycloalkyl;
wherein at least one R¹ is F, Cl, C₁-C₆-alkyl, ethenyl or ethynyl either of which can be substituted, cyano, alkoxyl, haloalkyl, or C₃-C₆-cycloalkyl;
or both R¹, together with the carbon atoms to which they are bound, form a fused phenyl;
R is H, alkyl optionally substituted with -((C₁-C₆-alkyl)OC(O)OC₁-C₆-alkyl), or benzyl,
wherein the benzyl can be unsubstituted or substituted with methoxyl; and
Ring A is a bicyclic fully aromatic or partially reduced heteroaryl ring system comprising 3, 4, or 5 N atoms, substituted with 0, 1, 2, 3 ,or 4 substituents each independently selected from the set consisting of NH₂, cyano, NHC(=O)O-tBu,OH, carboxamido, C₁-C₆-alkyl, - S(O)₂(C₁-C₆-alkyl), -S(O)(C₁-C₆-alkyl), alkylnitrile, alkoxyl, and haloalkyl; provided that a partially reduced heteroaryl ring system can also be substituted with an oxo group;
or a pharmaceutically acceptable salt thereof,
or formula (II): wherein 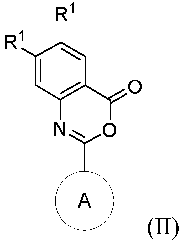
each R¹ is independently H, F, Cl, ethenyl or ethynyl either of which can be substituted, cyano, alkoxyl, haloalkyl, or C₃-C₆-cycloalkyl;
wherein at least one R¹ is F, Cl, ethenyl or ethynyl either of which can be substituted, cyano, alkoxyl, haloalkyl, or C₃-C₆-cycloalkyl;
or both R¹, together with the carbon atoms to which they are bound, form a fused phenyl; and
Ring A is a bicyclic fully aromatic or partially reduced heteroaryl ring system comprising 3, 4, or 5 N atoms, substituted with 0, 1, 2, 3 ,or 4 substituents each independently selected from the set consisting of NH₂, cyano, NHC(=O)O-tBu,OH, carboxamido, C₁-C₆-alkyl, - S(O)₂(C₁-C₆-alkyl), -S(O)(C₁-C₆-alkyl), alkylnitrile, alkoxyl, and haloalkyl; provided that a partially reduced heteroaryl ring system can also be substituted with an oxo group;
or a pharmaceutically acceptable salt thereof.

2. The compound according to claim 1, wherein the compound is of formula (I), wherein X is S, -N=C(R¹)-, or -C(R¹)=C(R¹)-;
each R¹ is independently H, F, Cl, ethenyl or ethynyl either of which can be substituted, cyano, alkoxyl, or haloalkyl;
wherein at least one R¹ is F, Cl, ethenyl or ethynyl either of which can be substituted, cyano, alkoxyl, or haloalkyl
R is H, alkyl, or benzyl, wherein the benzyl can be unsubstituted or substituted with methoxyl; and
Ring A is a bicyclic fully aromatic or partially reduced heteroaryl ring system comprising 3, 4, or 5 N atoms, substituted with 0, 1, 2, 3 ,or 4 substituents each independently selected from the set consisting of NH₂, cyano, NHC(=O)O-tBu,OH, carboxamido, alkylnitrile, alkoxyl, and haloalkyl; provided that a partially reduced heteroaryl ring system can also be substituted with an oxo group.

3. The compound according to claim 1, wherein the compound is of formula (II).

4. The compound according to any one of claims 1 to 3, wherein Ring A is unsubstituted or substituted, and is one selected from the group consisting of: 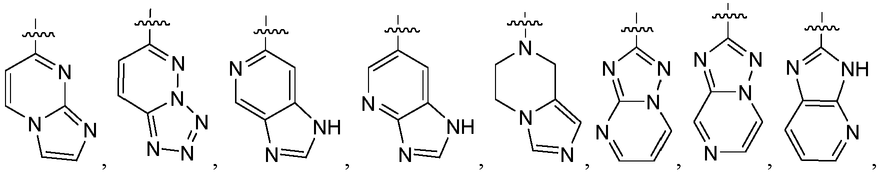 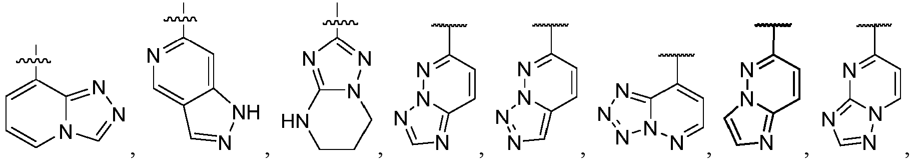 and 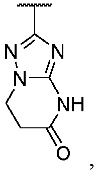 wherein a wavy line indicates a position of bonding.

5. A compound, or a pharmaceutically acceptable salt thereof, selected from the following table:
| | | | |
|---|---|---|---|
| **1** | 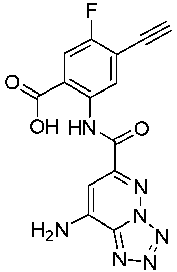 | **2** | 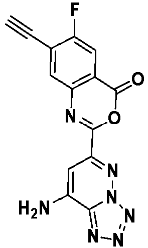 |
| **3** | 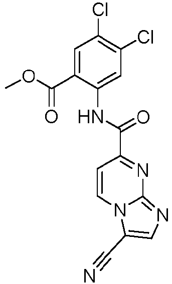 | **4** | 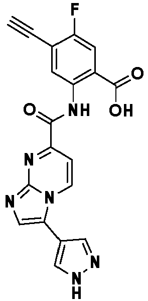 |
| **5** | 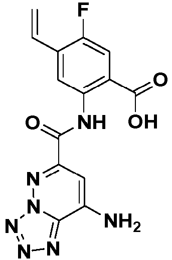 | **48** | 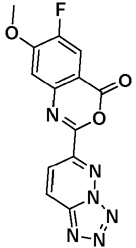 |
| **6** | 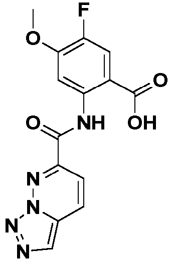 | **49** | 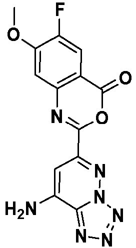 |
| **7** | 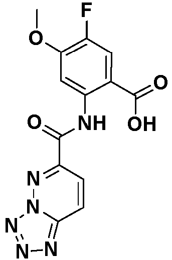 | **50** | 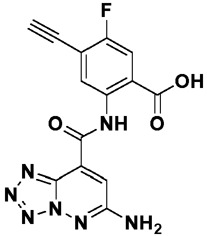 |
| **8** | 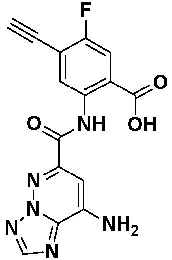 | **51** | 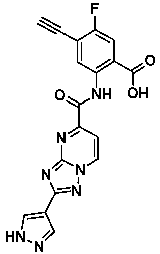 |
| **9** | 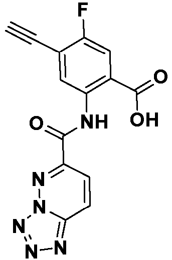 | **52** | 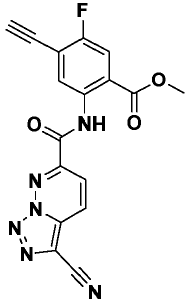 |
| **10** | 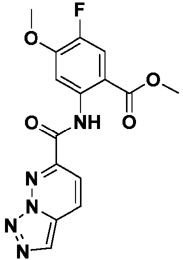 | **53** | 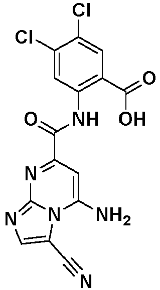 |
| **11** | 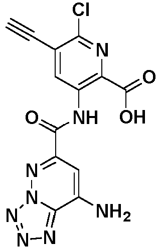 | **54** | 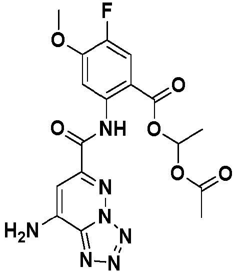 |
| **12** | 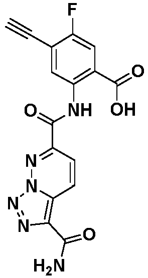 | **55** | 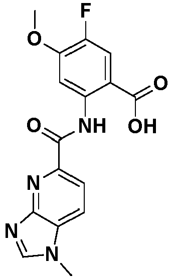 |
| **13** | 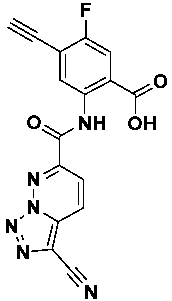 | **56** | 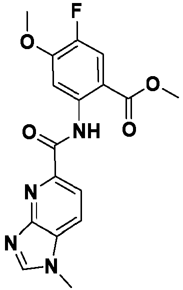 |
| **14** | 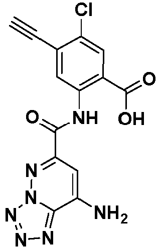 | **57** | 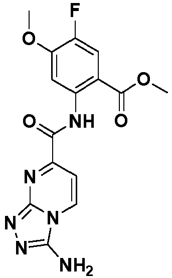 |
| **15** | 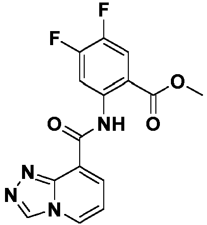 | **58** | 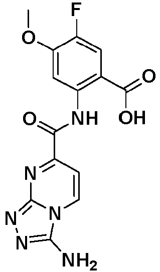 |
| **16** | 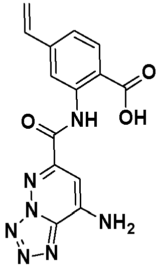 | **59** | 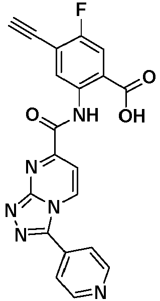 |
| **17** | 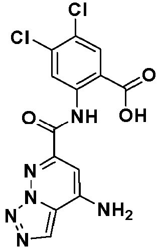 | **60** | 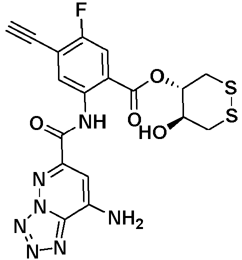 |
| **18** | 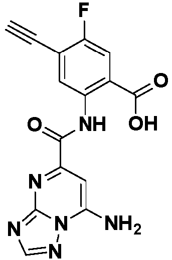 | **61** | 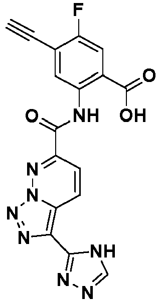 |
| **19** | 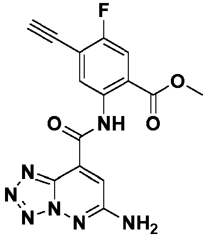 | **62** | 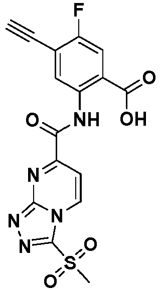 |
| **20** | 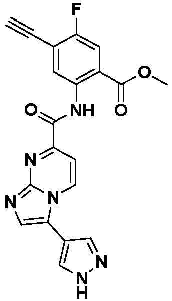 | **63** | 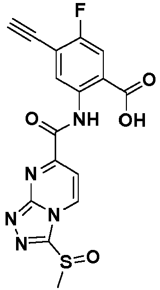 |
| **21** | 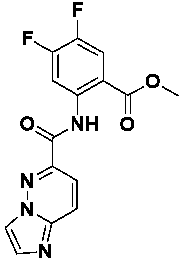 | **64** | 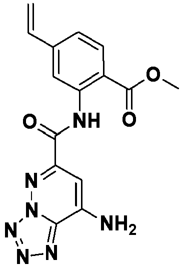 |
| **22** | 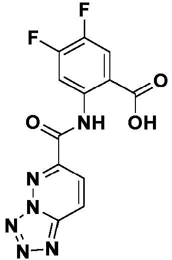 | **65** | 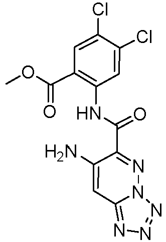 |
| **23** | 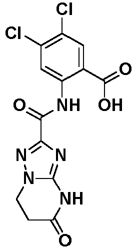 | **66** | 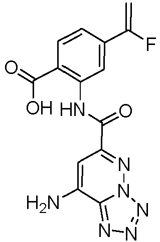 |
| **24** | 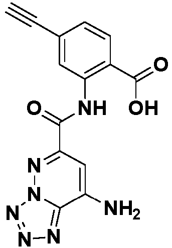 | **67** | 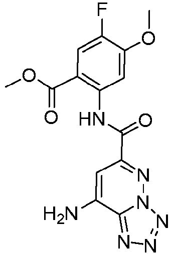 |
| **25** | 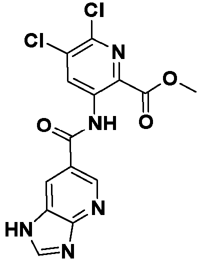 | **68** | 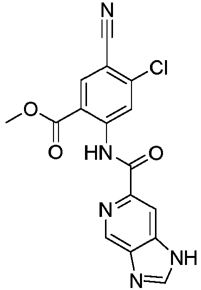 |
| **26** | 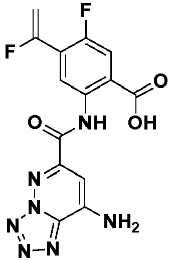 | **69** | 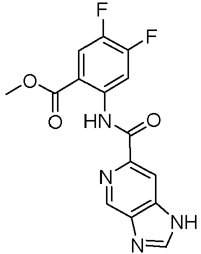 |
| **27** | 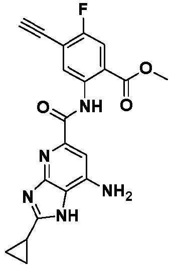 | **70** | 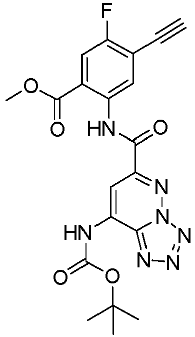 |
| **28** | 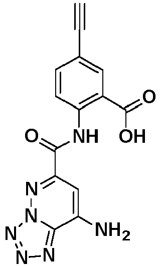 | **71** | 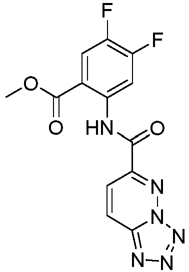 |
| **29** | 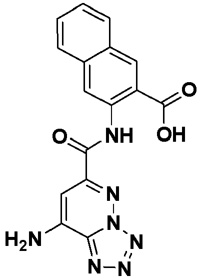 | **72** | 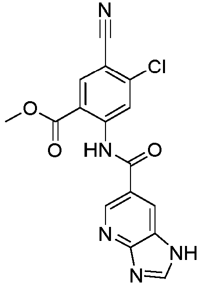 |
| **30** | 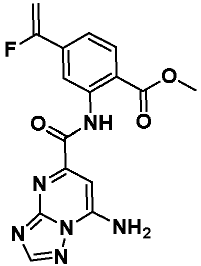 | **73** | 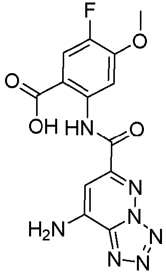 |
| **31** | 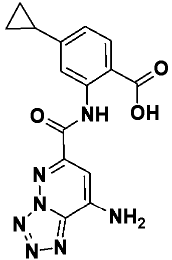 | **74** | 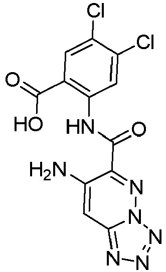 |
| **32** | 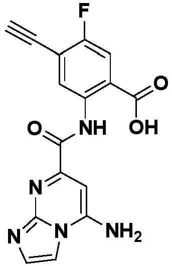 | **75** | 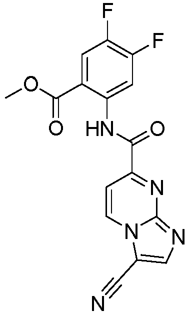 |
| **33** | 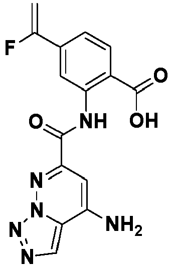 | **76** | 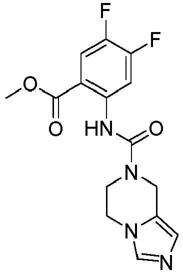 |
| **34** | 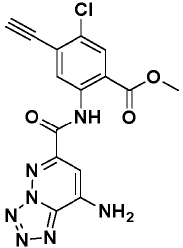 | **77** | 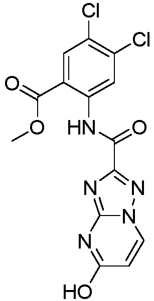 |
| **35** | 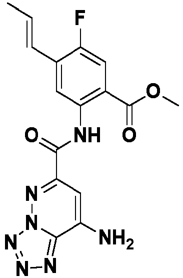 | **78** | 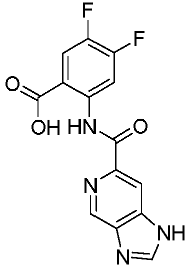 |
| **36** | 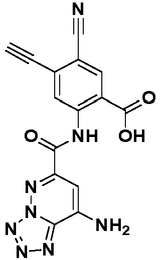 | **79** | 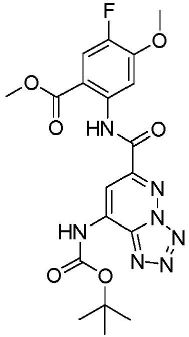 |
| **37** | 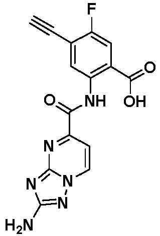 | **80** | 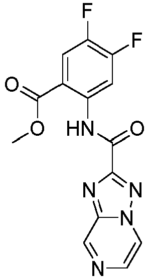 |
| **38** | 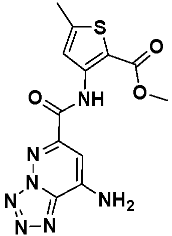 | **81** | 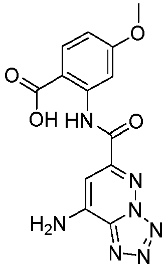 |
| **39** | 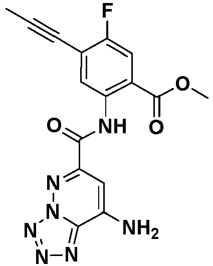 | **82** | 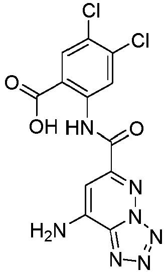 |
| **40** | 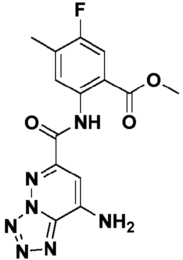 | **83** | 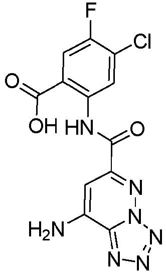 |
| **41** | 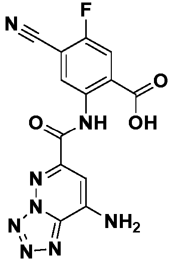 | **84** | 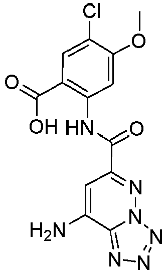 |
| **42** | 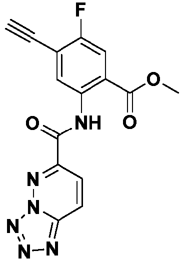 | **85** | 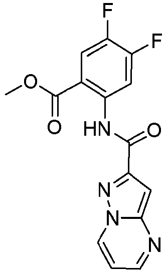 |
| **43** | 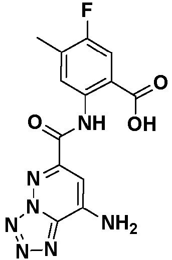 | **86** | 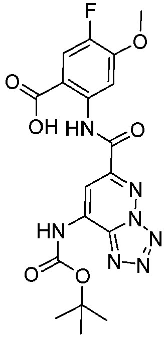 |
| **44** | 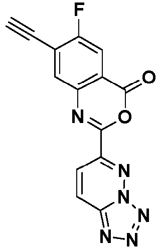 | **87** | 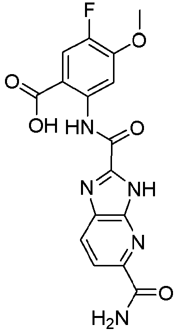 |
| **45** | 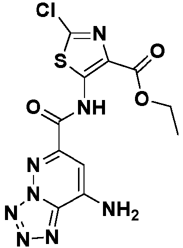 | **88** | 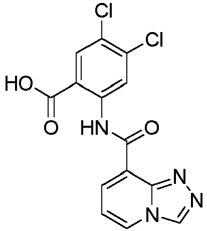 |
| **46** | 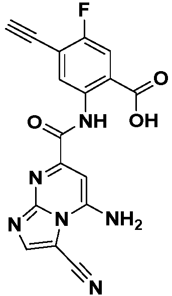 | **47** | 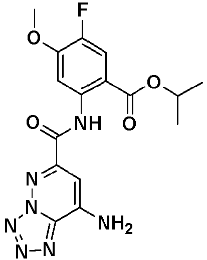 |
| **89** | 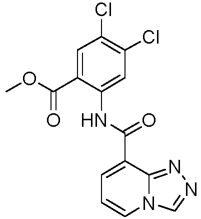 | **90** | 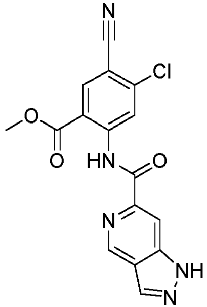 |
| **91** | 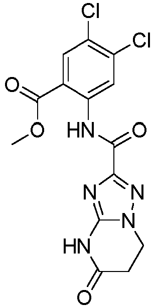 | | |

6. A pharmaceutical composition comprising a compound or pharmaceutically acceptable salt thereof according to any one of claims 1 to 5 and a pharmaceutically acceptable carrier.

7. A compound or pharmaceutically acceptable salt thereof according to any one of claims 1 to 5 for use in stimulating expression of interferon genes in a human patient.

8. A compound or pharmaceutically acceptable salt thereof according to any one of claims 1 to 5 for use in the treatment of a tumor in a patient.

9. The compound or pharmaceutically acceptable salt thereof for use according to claim 7 or 8, wherein the compound is an antibody-drug conjugate or in a liposomal formulation.

10. The compound or pharmaceutically acceptable salt thereof for use according to claim 7 to 9, wherein the use comprises administering an effective dose of an immune-checkpoint targeting drug.

11. The compound or pharmaceutically acceptable salt thereof for use according to claim 10, wherein the immune-checkpoint targeting drug comprises an anti-PD-L1 antibody, anti-PD-1 antibody, anti-CTLA-4 antibody, or an anti-4-1BB antibody.

12. The compound or pharmaceutically acceptable salt thereof for use according to claim 8 or 9, wherein the treatment comprises ionizing radiation or anticancer drugs.

## Patentansprüche

1. Verbindung der Formel (I): 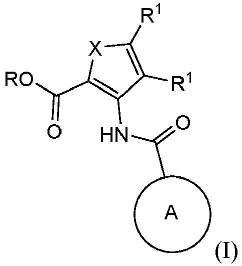 wobei
X gleich S, -N=C(R¹)-, oder -C(R¹)=C(R¹)- ist;
jedes R¹ ist unabhängig voneinander H, F, Cl, C₁-C₆-Alkyl, Ethenyl oder Ethinyl, die jeweils substituiert sein können, Cyano, Alkoxyl, Halogenalkyl, oder C₃-C₆-Cycloalkyl;
wobei mindestens ein R¹ F, Cl, C₁-C₆-Alkyl, Ethenyl oder Ethinyl, die jeweils substituiert sein können, Cyano, Alkoxyl, Halogenalkyl, oder C₃-C₆-Cycloalkyl ist;
oder beide R¹ bilden zusammen mit den Kohlenstoffatomen, an die sie gebunden sind, ein kondensiertes Phenyl;
R ist H, Alkyl, optional substituiert mit -((C₁-C₆-Alkyl)OC(O)OC₁-C₆-Alkyl), oder Benzyl, wobei das Benzyl unsubstituiert sein kann oder mit Methoxyl substituiert sein kann; und
Ring A ist ein bicyclisches, vollständig aromatisches oder teilweise reduziertes Heteroarylringsystem umfassend 3, 4 oder 5 N-Atome, substituiert mit 0, 1, 2, 3 oder 4 Substituenten, die jeweils unabhängig voneinander aus der Gruppe ausgewählt sind, die aus NH₂, Cyano, NHC(=O)O-tBu, OH, Carboxamido, C₁-C₆-Alkyl, -S(O)₂(C₁-C₆-Alkyl), -S(O)(C₁-C₆-Alkyl), Alkylnitril, Alkoxyl, und Halogenalkyl besteht; mit der Maßgabe, dass ein teilweise reduziertes Heteroarylringsystem auch mit einer Oxogruppe substituiert sein kann;
oder ein pharmazeutisch annehmbares Salz davon,
oder der Formel (II): wobei 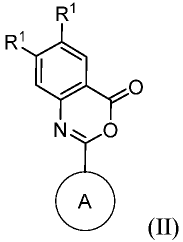
jedes R¹ unabhängig voneinander H, F, Cl, Ethenyl oder Ethinyl, die jeweils substituiert sein können, Cyano, Alkoxyl, Halogenalkyl, oder C₃-C₆-Cycloalkyl ist;
wobei mindestens ein R¹ F, Cl, Ethenyl oder Ethinyl, die jeweils substituiert sein können, Cyano, Alkoxyl, Halogenalkyl, oder C₃-C₆-Cycloalkyl ist;
oder beide R¹ bilden zusammen mit den Kohlenstoffatomen, an die sie gebunden sind, ein kondensiertes Phenyl; und
Ring A ist ein bicyclisches, vollständig aromatisches oder teilweise reduziertes Heteroarylringsystem umfassend 3, 4 oder 5 N-Atome, substituiert mit 0, 1, 2, 3 oder 4 Substituenten, die jeweils unabhängig voneinander aus der Gruppe ausgewählt sind, die aus NH₂, Cyano, NHC(=O)O-tBu, OH, Carboxamido, C₁-C₆-Alkyl, -S(O)₂(C₁-C₆-Alkyl), -S(O)(C₁-C₆-Alkyl), Alkylnitril, Alkoxyl, und Halogenalkyl besteht; mit der Maßgabe, dass ein teilweise reduziertes Heteroarylringsystem auch mit einer Oxogruppe substituiert sein kann;
oder ein pharmazeutisch annehmbares Salz davon.

2. Verbindung nach Anspruch 1, wobei die Verbindung eine Verbindung der Formel (I) ist, wobei
X gleich S, -N=C(R¹)-, oder -C(R¹)=C(R¹)- ist;
jedes R¹ ist unabhängig voneinander H, F, Cl, Ethenyl oder Ethinyl, die jeweils substituiert sein können, Cyano, Alkoxyl, oder Halogenalkyl;
wobei mindestens ein R¹ F, Cl, Ethenyl oder Ethinyl, die jeweils substituiert sein können, Cyano, Alkoxyl oder Halogenalkyl ist,
R ist H, Alkyl oder Benzyl, wobei das Benzyl unsubstituiert oder mit Methoxyl substituiert sein kann; und
Ring A ist ein bicyclisches, vollständig aromatisches oder teilweise reduziertes Heteroarylringsystem umfassend 3, 4 oder 5 N-Atome, substituiert mit 0, 1, 2, 3 oder 4 Substituenten, die jeweils unabhängig voneinander aus der Gruppe ausgewählt sind, die aus NH₂, Cyano, NHC(=O)O-tBu, OH, Carboxamido, Alkylnitril, Alkoxyl, und Halogenalkyl besteht; mit der Maßgabe, dass ein teilweise reduziertes Heteroarylringsystem auch mit einer Oxogruppe substituiert sein kann.

3. Verbindung nach Anspruch 1, wobei die Verbindung eine Verbindung der Formel (II) ist.

4. Verbindung nach einem der Ansprüche 1 bis 3, wobei Ring A unsubstituiert oder substituiert ist, und einer ist, ausgewählt aus der Gruppe bestehend aus: 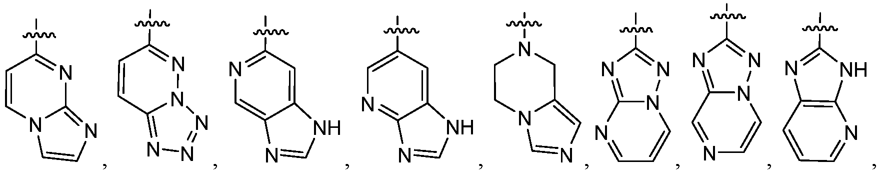 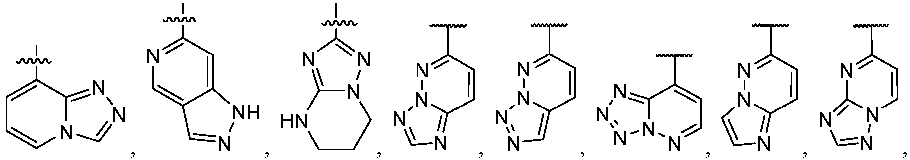 und 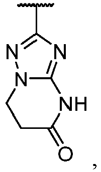 wobei eine gewellte Linie eine Bindungsposition anzeigt.

5. Verbindung, oder pharmazeutisch annehmbares Salz davon, ausgewählt aus der folgenden Tabelle:
| | | | |
|---|---|---|---|
| **1** | 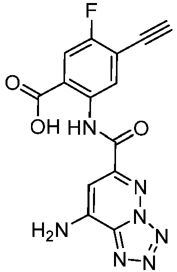 | **2** | 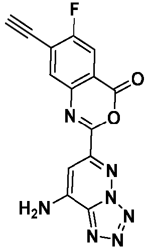 |
| **3** | 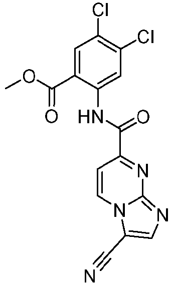 | **4** | 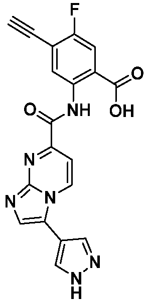 |
| **5** | 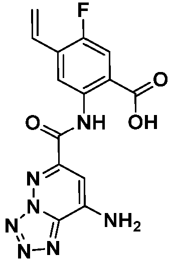 | **48** | 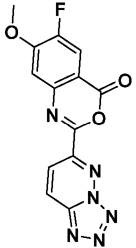 |
| **6** | 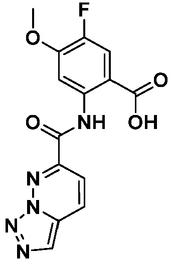 | **49** | 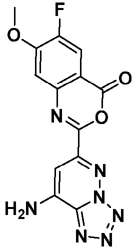 |
| **7** | 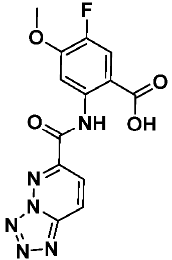 | **50** | 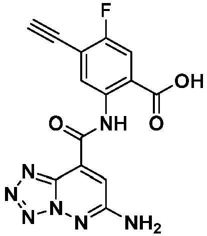 |
| **8** | 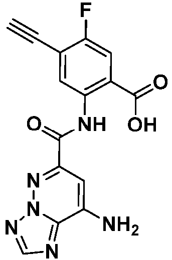 | **51** | 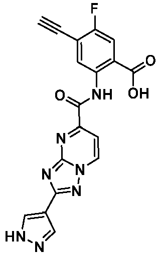 |
| **9** | 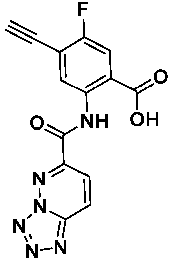 | **52** | 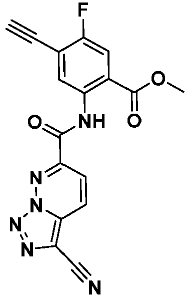 |
| **10** | 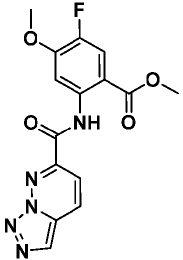 | **53** | 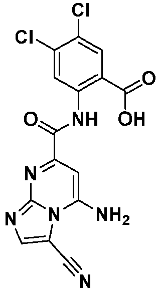 |
| **11** | 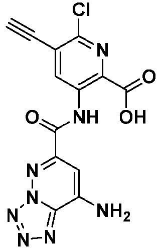 | **54** | 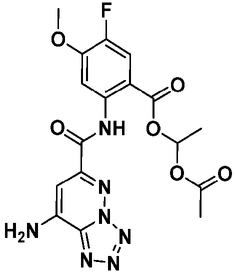 |
| **12** | 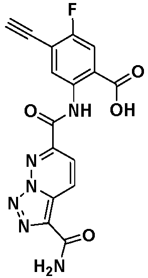 | **55** | 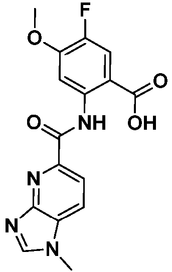 |
| **13** | 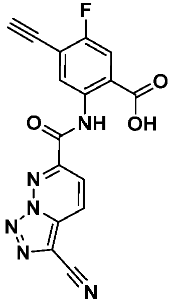 | **56** | 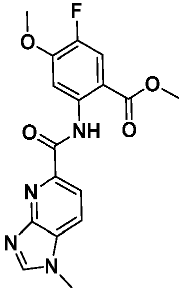 |
| **14** | 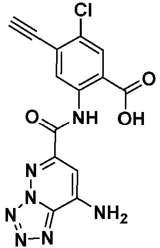 | **57** | 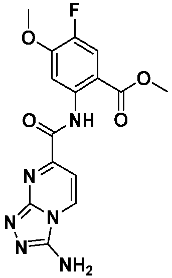 |
| **15** | 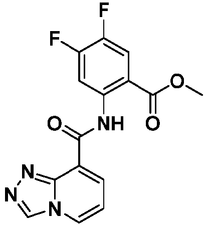 | ***58*** | 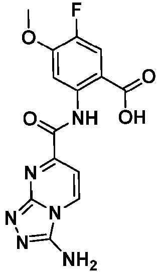 |
| **16** | 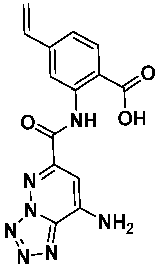 | **59** | 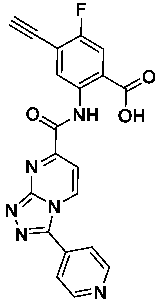 |
| **17** | 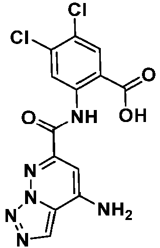 | **60** | 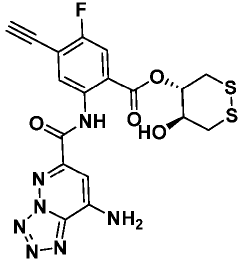 |
| **18** | 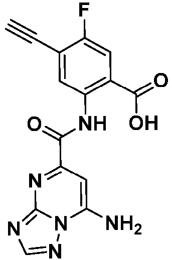 | **61** | 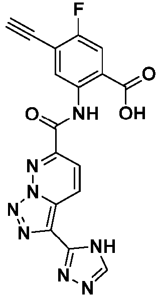 |
| **19** | 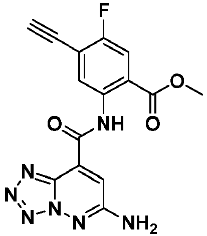 | **62** | 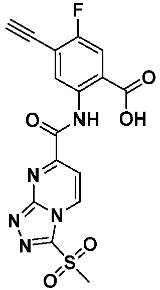 |
| **20** | 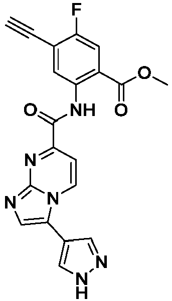 | **63** | 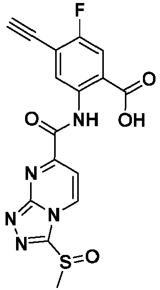 |
| **21** | 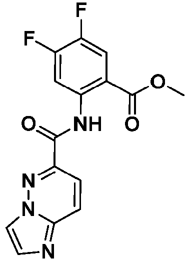 | **64** | 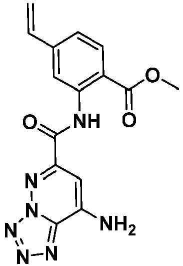 |
| **22** | 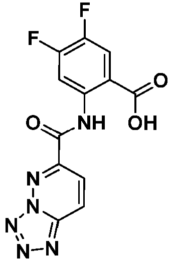 | **65** | 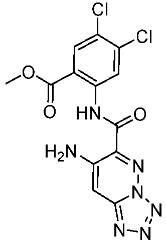 |
| **23** | 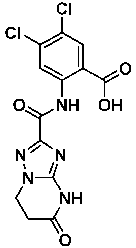 | **66** | 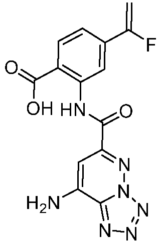 |
| **24** | 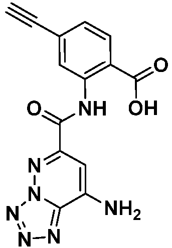 | **67** | 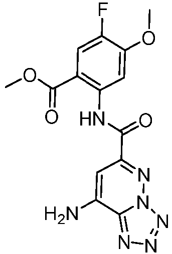 |
| **25** | 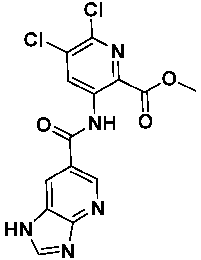 | **68** | 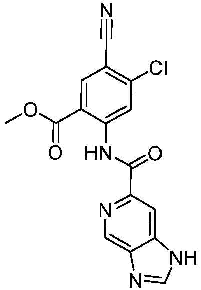 |
| **26** | 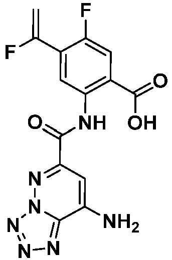 | **69** | 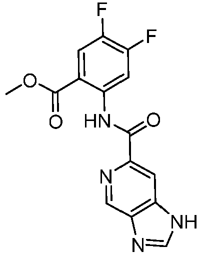 |
| **27** | 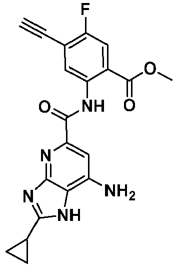 | **70** | 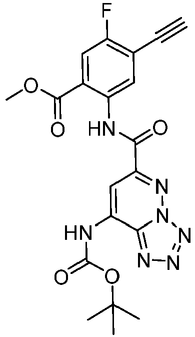 |
| **28** | 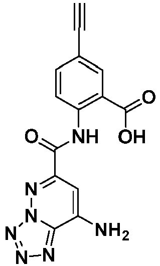 | **71** | 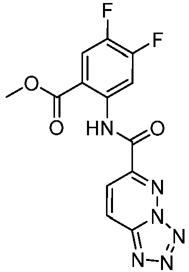 |
| **29** | 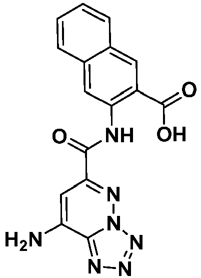 | **72** | 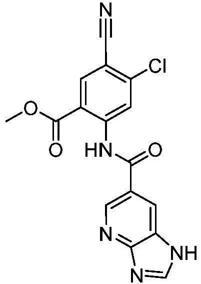 |
| **30** | 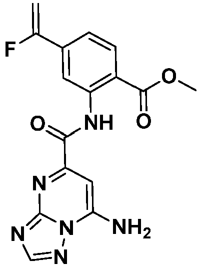 | **73** | 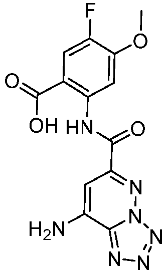 |
| **31** | 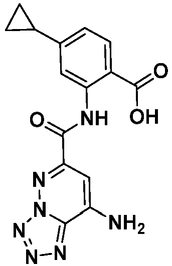 | **74** | 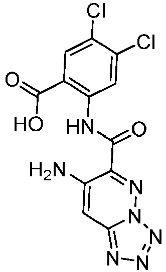 |
| **32** | 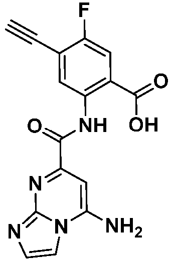 | **75** | 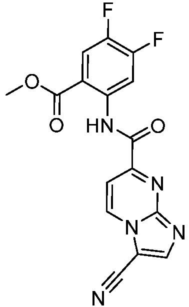 |
| **33** | 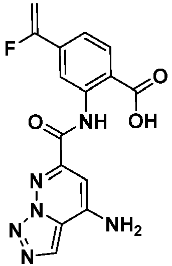 | **76** | 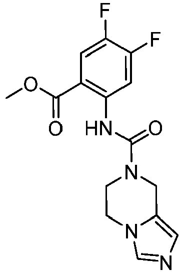 |
| **34** | 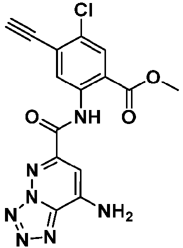 | **77** | 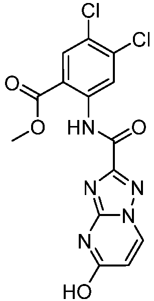 |
| **35** | 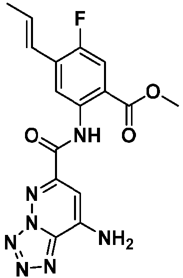 | **78** | 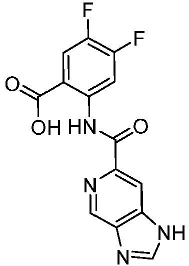 |
| **36** | 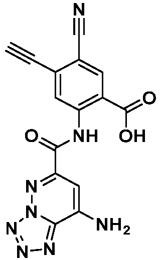 | **79** | 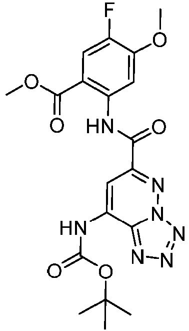 |
| **37** | 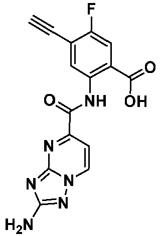 | **80** | 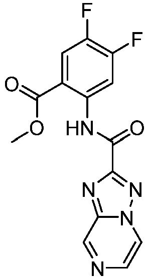 |
| **38** | 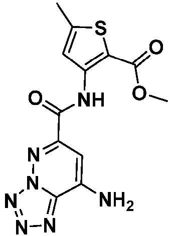 | **81** | 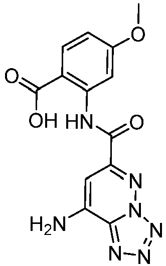 |
| **39** | 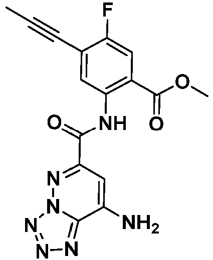 | **82** | 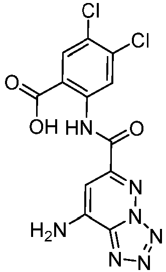 |
| **40** | 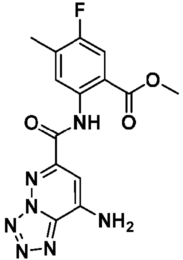 | **83** | 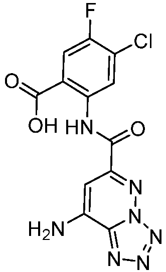 |
| **41** | 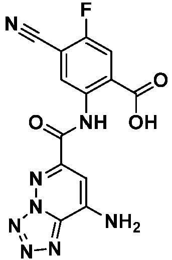 | **84** | 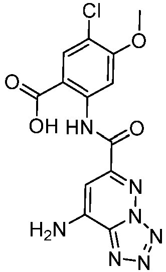 |
| **42** | 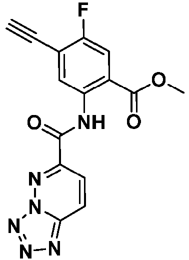 | ***85*** | 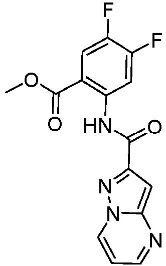 |
| **43** | 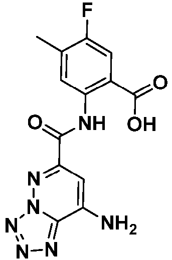 | **86** | 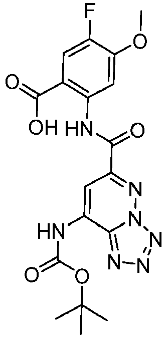 |
| **44** | 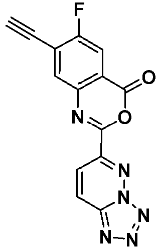 | **87** | 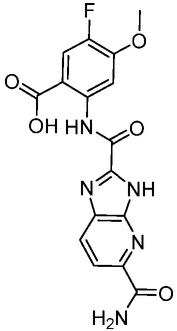 |
| **45** | 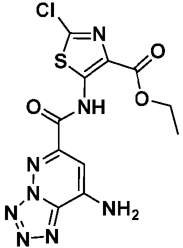 | **88** | 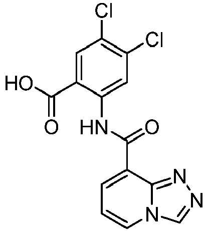 |
| **46** | 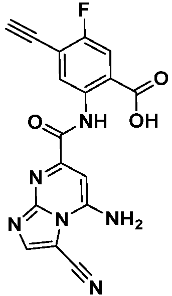 | **47** | 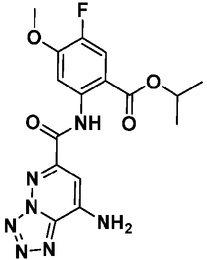 |
| **89** | 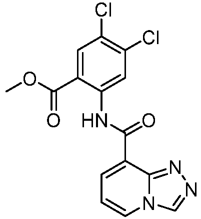 | **90** | 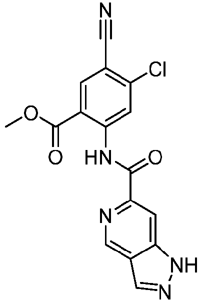 |
| **91** | 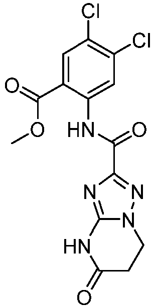 | | |

6. Pharmazeutische Zusammensetzung, umfassend eine Verbindung oder ein pharmazeutisch annehmbares Salz davon nach einem der Ansprüche 1 bis 5 und einen pharmazeutisch annehmbaren Träger.

7. Verbindung oder pharmazeutisch annehmbares Salz davon nach einem der Ansprüche 1 bis 5 zur Verwendung bei der Stimulierung der Expression von Interferon-Genen in einem menschlichen Patienten.

8. Verbindung oder pharmazeutisch annehmbares Salz davon nach einem der Ansprüche 1 bis 5 zur Verwendung bei der Behandlung eines Tumors bei einem Patienten.

9. Verbindung oder pharmazeutisch annehmbares Salz davon zur Verwendung nach Anspruch 7 oder 8, wobei die Verbindung ein Antikörper-Wirkstoff-Konjugat oder in einer liposomalen Formulierung ist.

10. Verbindung oder pharmazeutisch annehmbares Salz davon zur Verwendung nach Anspruch 7 bis 9, wobei die Verwendung die Verabreichung einer wirksamen Dosis eines Immun-Checkpoint-Targeting-Medikaments umfasst.

11. Verbindung oder pharmazeutisch annehmbares Salz davon zur Verwendung nach Anspruch 10, wobei das Immun-Checkpoint-Targeting-Medikament einen Anti-PD-L1-Antikörper, einen Anti-PD-1-Antikörper, einen Anti-CTLA-4-Antikörper oder einen Anti-4-1BB-Antikörper umfasst.

12. Verbindung oder pharmazeutisch annehmbares Salz davon zur Verwendung nach Anspruch 8 oder 9, wobei die Behandlung ionisierende Strahlung oder Anti-Krebs-Medikamente umfasst.

## Revendications

1. Composé de formule (I) : 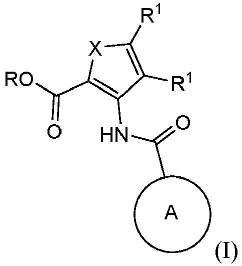 dans laquelle
X est S, -N=C(R¹)-, ou -C(R¹)=C(R¹)- ; chaque R¹ est indépendamment H, F, Cl, un groupe C₁-C₆-alkyle, éthényle or éthynyle pouvant l'un ou l'autre être substitué, cyano, alcoxy, halogénoalkyle, ou C₃-C₆-cycloalkyle ;
au moins un R¹ étant F, Cl, un groupe C₁-C₆-alkyle, éthényle ou éthynyle pouvant l'un ou l'autre être substitué, cyano, alcoxy, halogénoalkyle, ou C₃-C₆-cycloalkyle ;
ou les deux R¹, ensemble avec les atomes de carbone auxquels ils sont liés, forment un cycle phényle fusionné ;
R est H, un groupe alkyle éventuellement substitué par -((C₁-C₆-alkyle)OC(O)OC₁-C₆- alkyle, ou benzyle, le groupe benzyle pouvant être non substitué ou substitué par méthoxy ; et
le cycle A est un système cyclique hétéroaryle bicyclique entièrement aromatique ou partiellement réduit comprenant 3, 4, ou 5 atomes d'azote, substitué par 0, 1, 2, 3, ou 4 substituants choisis chacun indépendamment dans l'ensemble constitué par NH₂, cyano, NHC(=O)O-tBu, OH, carboxamido, C₁-C₆-alkyle, -S(O)₂(C₁-C₆-alkyle), -S(O)(C₁-C₆-alkyle), alkylnitrile, alcoxy, et halogénoalkyle ; étant entendu qu'un système cyclique hétéroaryle partiellement réduit peut également être substitué par un groupe oxo ;
ou sel pharmaceutiquement acceptable d'un tel composé, ou de formule (II) : dans laquelle 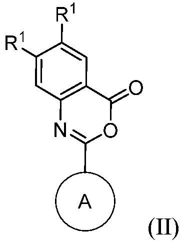
chaque R¹ est indépendamment H, F, Cl, un groupe éthényle or éthynyle pouvant l'un ou l'autre être substitué, cyano, alcoxy, halogénoalkyle, ou C₃-C₆-cycloalkyle ;
au moins un R¹ étant F, Cl, éthényle ou éthynyle pouvant l'un ou l'autre être substitué, cyano, alcoxy, halogénoalkyle, ou C₃-C₆-cycloalkyle ;
ou les deux R¹, ensemble avec les atomes de carbone auxquels ils sont liés, forment un cycle phényle fusionné ; et
le cycle A est un système cyclique hétéroaryle bicyclique entièrement aromatique ou partiellement réduit comprenant 3, 4, ou 5 atomes d'azote, substitué par 0, 1, 2, 3, ou 4 substituants choisis chacun indépendamment dans l'ensemble constitué par NH₂, cyano, NHC(=O)O-tBu, OH, carboxamido, C₁-C₆-alkyle, -S(O)₂(C₁-C₆-alkyle), -S(O)(C₁-C₆-alkyle), alkylnitrile, alcoxy, et halogénoalkyle ; étant entendu qu'un système cyclique hétéroaryle partiellement réduit peut également être substitué par un groupe oxo ;
ou sel pharmaceutiquement acceptable d'un tel composé.

2. Composé selon la revendication 1, le composé étant de formule (I), dans laquelle X est S, -N=C(R¹)-, ou -C(R¹)=C(R¹)- ;
chaque R¹ est indépendamment H, F, Cl, un groupe éthényle or éthynyle pouvant l'un ou l'autre être substitué, cyano, alcoxy ou halogénoalkyle ;
au moins un R¹ étant F, Cl, un groupe éthényle ou éthynyle pouvant l'un ou l'autre être substitué, cyano, alcoxy, ou halogénoalkyle ;
R est H, un groupe alkyle, ou benzyle, le groupe benzyle pouvant être non substitué ou substitué par méthoxy ; et
le cycle A est un système cyclique hétéroaryle bicyclique entièrement aromatique ou
partiellement réduit comprenant 3, 4, ou 5 atomes d'azote, substitué par 0, 1, 2, 3, ou 4 substituants choisis chacun indépendamment dans l'ensemble constitué par NH₂, cyano, NHC(=O)O-tBu, OH, carboxamido, alkylnitrile, alcoxy, et halogénoalkyle ; étant entendu qu'un système cyclique hétéroaryle partiellement réduit peut également être substitué par un groupe oxo.

3. Composé selon la revendication 1, le composé étant de formule (II).

4. Composé selon l'une quelconque des revendications 1 à 3, dans lequel le cycle A est substitué ou non substitué, et est un choisi dans le groupe constitué par : 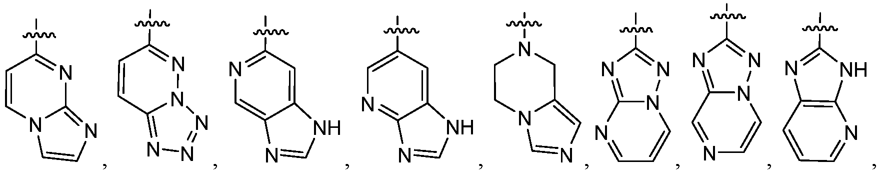 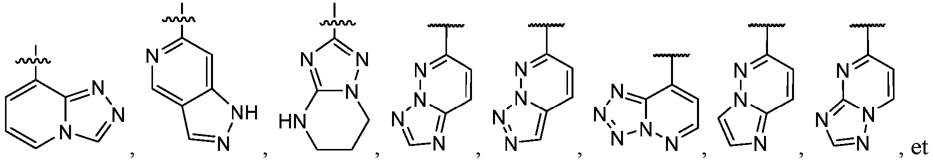 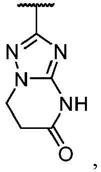 où un trait ondulé indique une position de liaison.

5. Composé, ou sel pharmaceutiquement acceptable d'un tel composé, choisi dans le tableau suivant :
| | | | |
|---|---|---|---|
| **1** | 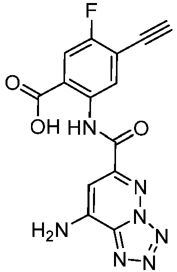 | **2** | 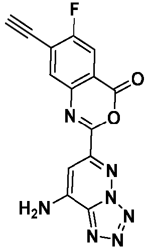 |
| **3** | 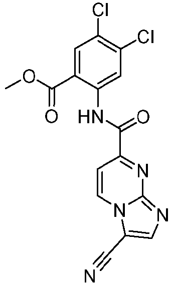 | **4** | 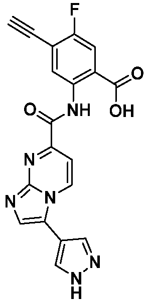 |
| **5** | 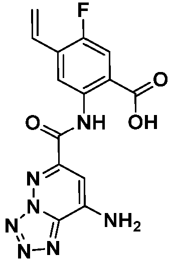 | **48** | 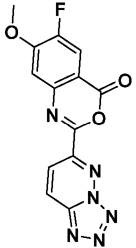 |
| **6** | 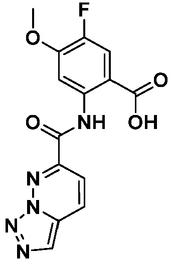 | **49** | 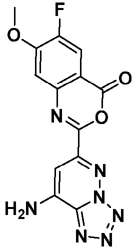 |
| **7** | 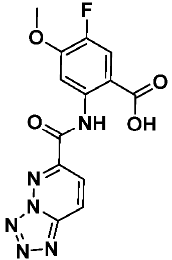 | **50** | 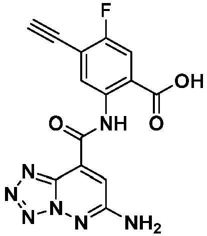 |
| **8** | 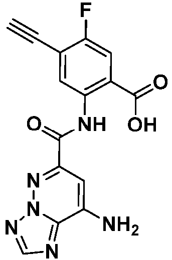 | **51** | 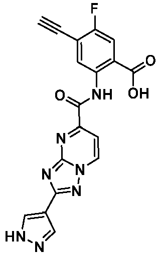 |
| **9** | 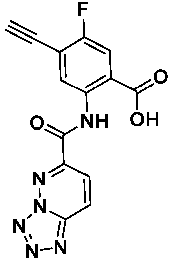 | **52** | 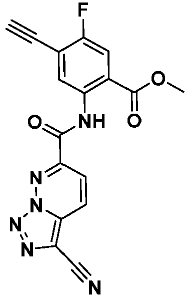 |
| **10** | 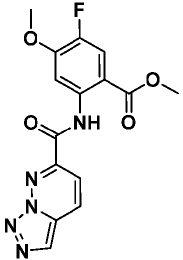 | **53** | 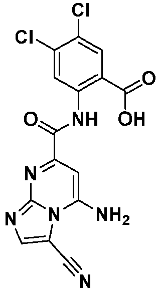 |
| **11** | 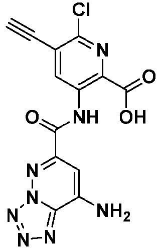 | **54** | 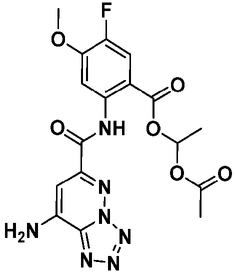 |
| **12** | 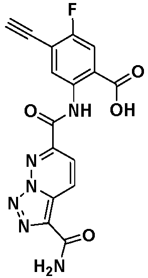 | **55** | 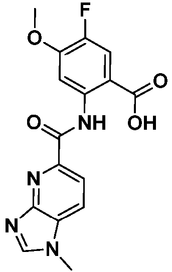 |
| **13** | 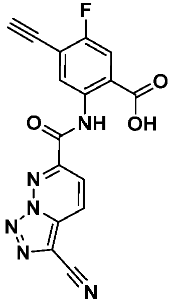 | **56** | 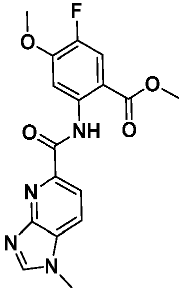 |
| **14** | 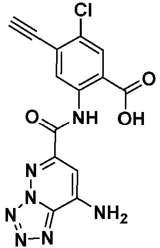 | **57** | 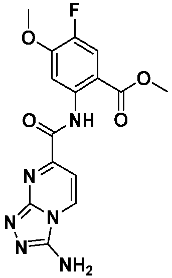 |
| **15** | 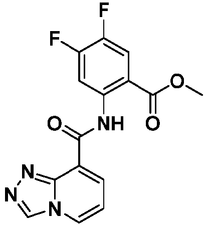 | ***58*** | 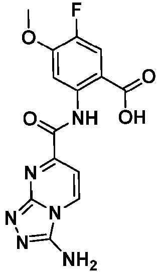 |
| **16** | 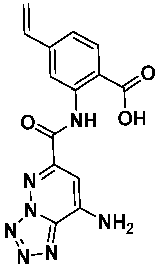 | **59** | 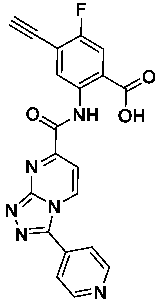 |
| **17** | 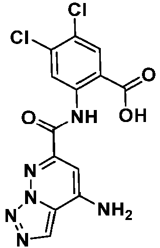 | **60** | 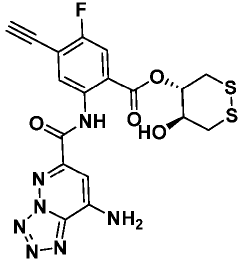 |
| **18** | 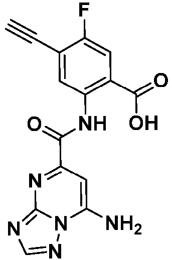 | **61** | 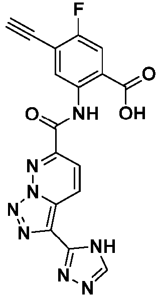 |
| **19** | 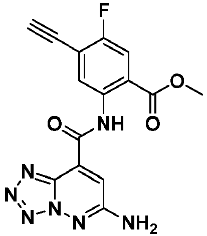 | **62** | 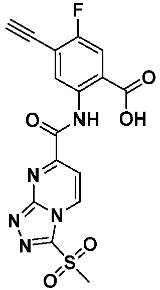 |
| **20** | 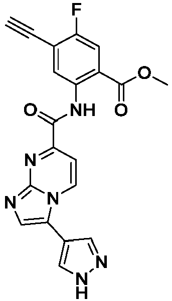 | **63** | 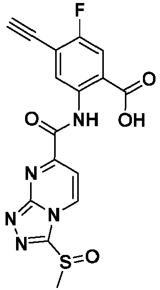 |
| **21** | 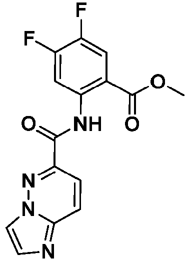 | **64** | 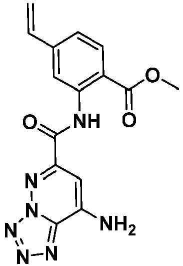 |
| **22** | 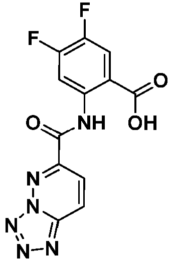 | **65** | 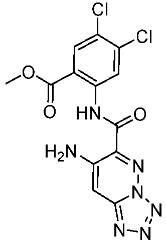 |
| **23** | 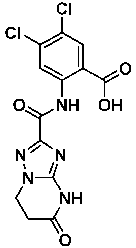 | **66** | 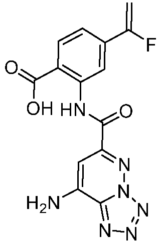 |
| **24** | 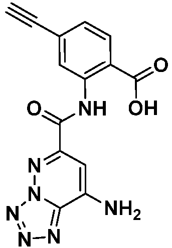 | **67** | 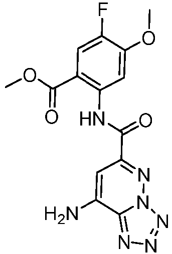 |
| **25** | 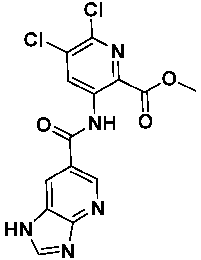 | **68** | 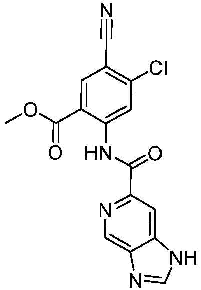 |
| **26** | 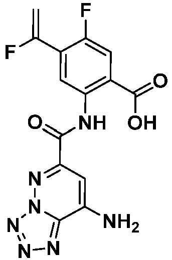 | **69** | 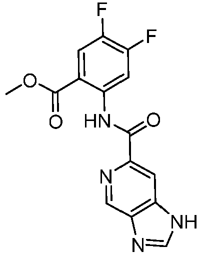 |
| **27** | 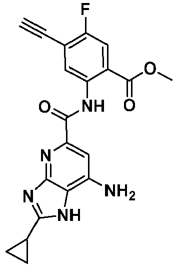 | **70** | 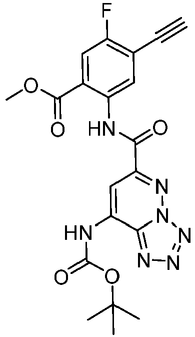 |
| **28** | 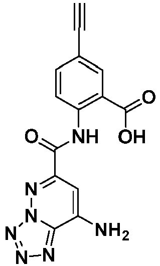 | **71** | 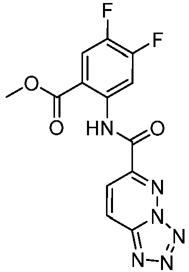 |
| **29** | 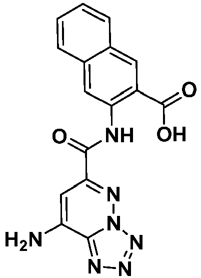 | **72** | 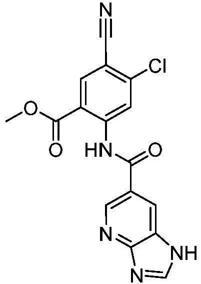 |
| **30** | 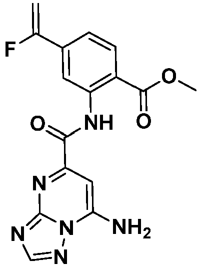 | **73** | 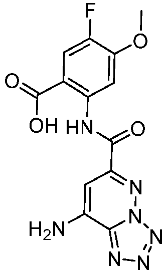 |
| **31** | 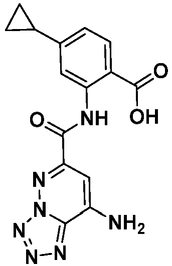 | **74** | 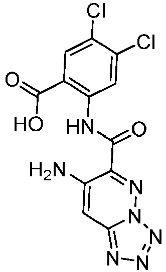 |
| **32** | 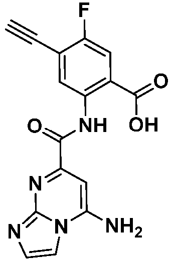 | **75** | 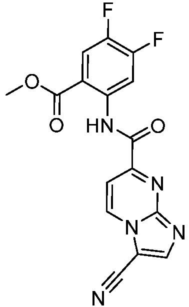 |
| **33** | 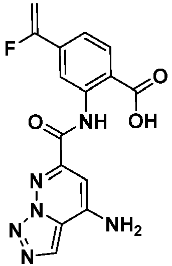 | **76** | 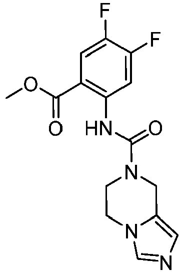 |
| **34** | 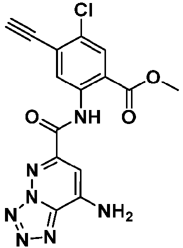 | **77** | 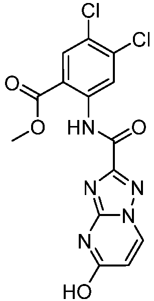 |
| **35** | 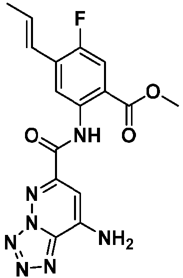 | **78** | 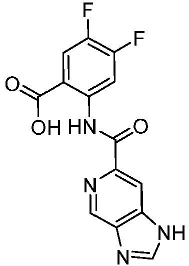 |
| **36** | 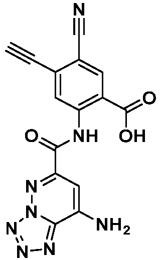 | **79** | 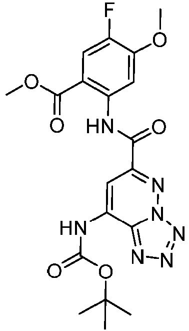 |
| **37** | 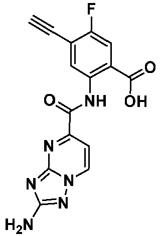 | **80** | 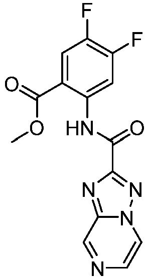 |
| **38** | 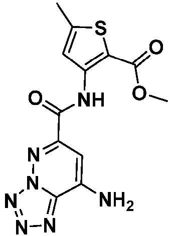 | **81** | 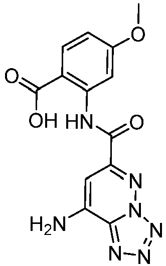 |
| **39** | 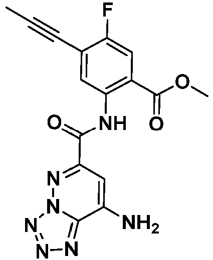 | **82** | 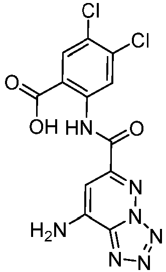 |
| **40** | 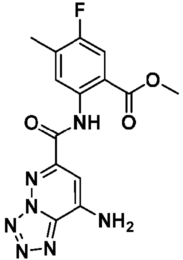 | **83** | 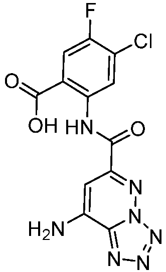 |
| **41** | 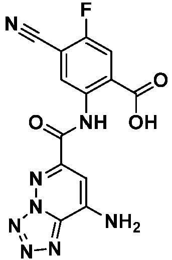 | **84** | 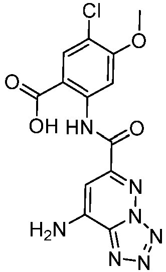 |
| **42** | 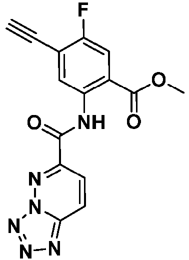 | ***85*** | 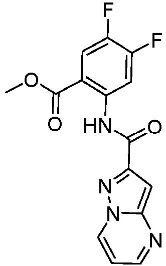 |
| **43** | 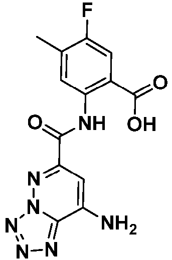 | **86** | 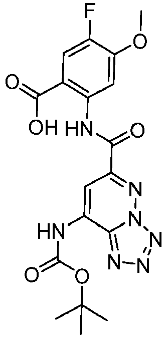 |
| **44** | 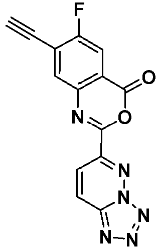 | **87** | 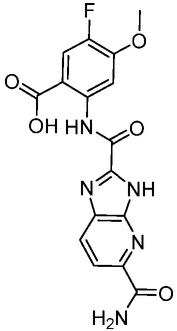 |
| **45** | 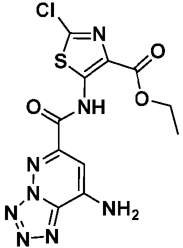 | **88** | 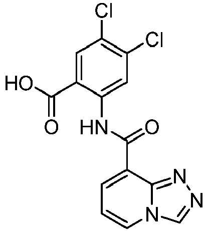 |
| **46** | 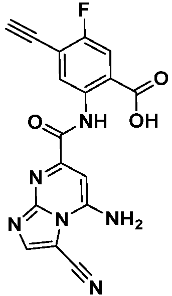 | **47** | 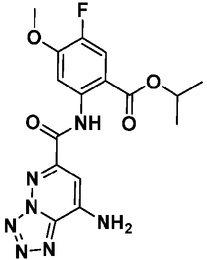 |
| **89** | 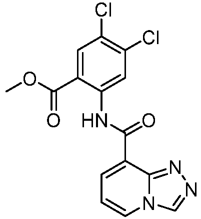 | **90** | 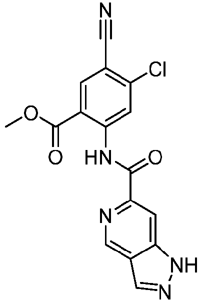 |
| **91** | 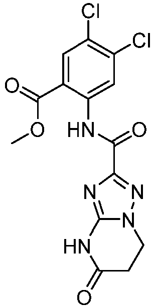 | | |

6. Composition pharmaceutique comprenant un composé ou sel pharmaceutiquement acceptable d'un tel composé selon l'une quelconque des revendications 1 à 5 et un véhicule pharmaceutiquement acceptable.

7. Composé ou sel pharmaceutiquement acceptable d'un tel composé selon l'une quelconque des revendications 1 à 5 pour utilisation dans la stimulation de l'expression de gènes d'interféron chez un patient humain.

8. Composé ou sel pharmaceutiquement acceptable d'un tel composé selon l'une quelconque des revendications 1 à 5 pour utilisation dans le traitement d'une tumeur chez un patient.

9. Composé ou sel pharmaceutiquement acceptable d'un tel composé pour utilisation selon la revendication 7 ou 8, le composé étant un conjugué anticorps-médicament ou dans une formulation liposomale.

10. Composé ou sel pharmaceutiquement acceptable d'un tel composé pour utilisation selon l'une quelconque des revendications 7 à 9, dans lequel l'utilisation comprend l'administration d'une dose efficace d'un médicament ciblant un point de contrôle immun.

11. Composé ou sel pharmaceutiquement acceptable d'un tel composé pour utilisation selon la revendication 10, dans lequel le médicament ciblant un point de contrôle immun comprend un anticorps anti-PD-L1, un anticorps anti-PD-1, un anticorps anti-CTLA-4, ou un anticorps anti-4-1BB.

12. Composé ou sel pharmaceutiquement acceptable d'un tel composé pour utilisation selon la revendication 8 ou 9, dans lequel le traitement comprend un rayonnement ionisant ou des médicaments anticancéreux.
